(19) Europäisches Patentamt
European Patent Office
Office européen des brevets

(11) EP 1 547 616 A1

(12) EUROPEAN PATENT APPLICATION
published in accordance with Art. 158(3) EPC

(43) Date of publication:
**29.06.2005 Bulletin 2005/26**

(21) Application number: **03792564.1**

(22) Date of filing: **22.08.2003**

(51) Int Cl.[7]: **A61K 45/00**, A61K 31/519, A61K 31/55, A61K 31/7088, A61K 39/395, A61K 48/00, A61P 17/04, A61P 43/00, C07D 401/14, C07D 403/06, C07D 417/14, C07D 471/14, C07D 519/00

(86) International application number:
**PCT/IB2003/003475**

(87) International publication number:
**WO 2004/017995 (04.03.2004 Gazette 2004/10)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **22.08.2002 JP 2002241522**

(71) Applicant: **KYOWA HAKKO KOGYO CO., LTD.**
**Chiyoda-ku, Tokyo 100-8185 (JP)**

(72) Inventors:
- **Saki, Mayumi, c/o Pharmaceutical Res. Inst. Sunto-gun, Shizuoka 411-8731 (JP)**
- **Nonaka, Hiromi, c/o Pharmaceutical Res. Inst. Sunto-gun, Shizuoka 411-873 (JP)**
- **Miyaji, Hiromasa, c/o Tokyo Research Lab. Machida-shi, Tokyo 194-8533 (JP)**
- **Ichikawa, Shunji, c/o Pharmaceutical Res. Inst. Sunto-gun, Shizuoka 411-8533 (JP)**
- **Takashima, Chiemi, c/o Pharmaceutical Res. Inst. Sunto-gun, Shizuoka 411-873 (JP)**
- **Matsumura, Tsutomu, c/o Sakai Research Lab. Sakai-shi, Osaka 590-8554 (JP)**
- **Arai, Hitoshi, c/o Pharmaceutical Res. Inst. Sunto-gun, Shizuoka 411-873 (JP)**
- **Sasaki, Katsutoshi, c/o Tokyo Research Lab. Machida-shi, Tokyo 194-8533 (JP)**
- **Kobatake, Choei, Head Office, Kyowa Hakko Chiyoda-ku,Tokyo 100-8185 (JP)**
- **Tsukumo, Yukihito, c/o Pharmaceutical Res. Inst. Sunto-gun, Shizuoka 411-8533 (JP)**
- **Iida, Kyoichiro, c/o Pharmaceutical Res. Inst. Sunto-gun, Shizuoka 411-8533 (JP)**
- **Kuboyama, Takeshi, c/o Pharmaceutical Res. Inst. Sunto-gun, Shizuoka 411-8533 (JP)**
- **Manabe, Haruhiko, c/o Pharmaceutical Res. Inst. Sunto-gun, Shizuoka 411-8533 (JP)**

(74) Representative: **Williams, Aylsa et al**
**D Young & Co**
**120 Holborn**
**London EC1N 2DY (GB)**

# (54) PREVENTIVE AND/OR THERAPEUTIC DRUGS FOR ITCH

(57) The present invention provides an agent for prevention and/or treatment of itching comprising a substance capable of suppressing the function involved in signal transduction of GPR4 as an active ingredient. It also provides a nitrogen-containing tricyclic compound represented by the formula (I) or a quaternary ammonium salt thereof, or a pharmaceutically acceptable salt thereof;

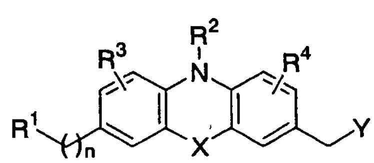

(I)

[wherein $R^1$ represents substituted or unsubstituted lower alkyl, etc.; $R^2$ represents hydrogen, substituted or unsubstituted lower alkyl, etc.; $R^3$ and $R^4$ are the same or different and each represents hydrogen, lower alkyl, etc.; n represents 0 or 1; X represents $-(CH_2)_2-$, etc.; and

EP 1 547 616 A1

Y represents the formula (II);

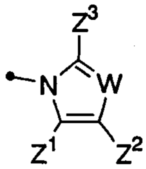

(II)

(wherein W represents CH or a nitrogen atom; $Z^1$ and $Z^2$ are the same or different and each represents hydrogen, substituted or unsubstituted lower alkyl, etc.; and $Z^3$ represents hydrogen, substituted or unsubstituted lower alkyl, etc.)]

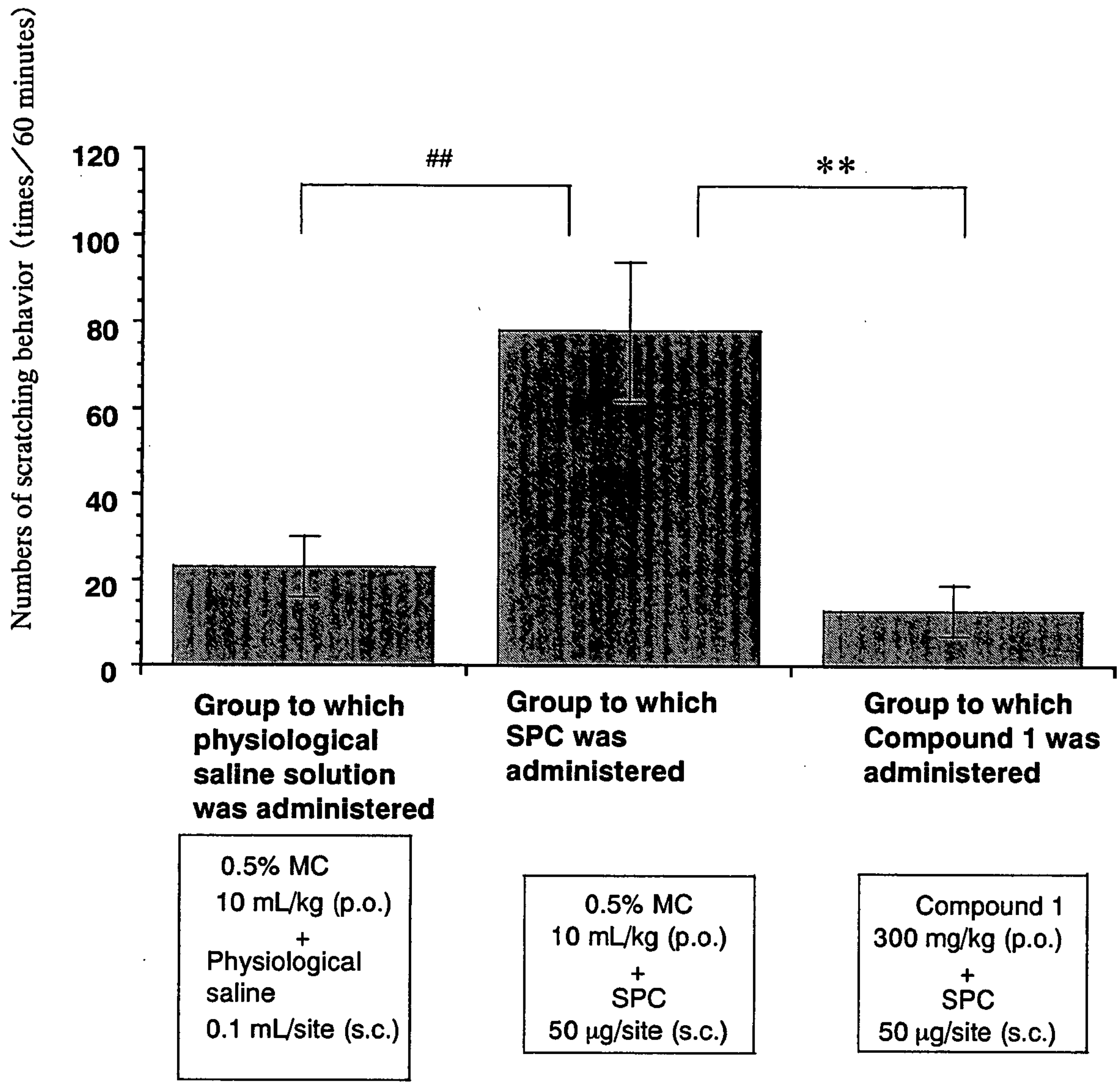


Fig.1

## Description

### Technical Field

The present invention relates to agents for prevention and/or treatment of itching, which comprises a substance capable of suppressing the function involved in signal transduction of GPR4 as an active ingredient. The present invention further relates to nitrogen-containing tricyclic compounds or quaternary ammonium salts thereof, or pharmaceutically acceptable salts thereof which are useful as an agent for prevention and/or treatment of itching. The present invention still further relates to a method for screening of a therapeutic agent for itching, wherein reduction in the number of scratching behavior induced by sphingosyl phosphorylcholine (SPC) is used as an index.

### Background Art

Itching is an important symptom in many dermatological diseases such as inflammatory dermatological diseases and induces scratching behavior whereby dermatological symptoms are worsened. In addition, some of systemic internal diseases such as chronic renal insufficiency and diabetes are accompanied by itching. It has been known that itching of urticaria which is one of the representative pruriginous dermatological diseases is mostly caused by histamine but generation mechanism for other itching has not been clarified yet. For the treatment thereof, anti-allergic drugs, antihistaminic drugs or steroidal agents for external application have been used but they are unable to be effective for all types of itching. Further, since long-term use of a steroidal agent for external application is accompanied by side effects, there has been a demand for better preventive or therapeutic agents for itching.

On the other hand, GPR4, which is a G-protein coupled-receptor protein (hereinafter, abbreviated as GPCR), is a receptor where sphingosyl phosphorylcholine (SPC) and lysophosphatidyl choline (LPC) act as intrinsic ligands [*Journal of Biological Chemistry,* volume 276, pages 41325-41335 (2001)]. With regard to SPC, it has been suggested that it participates in keratinization and inflammatory / allergic reactions of the skin noted in dermatitis such as atopic dermatitis [*Journal of Lipid Research,* volume 42, pages 1562-1570 (2001)] because it induces ICAM-1 expression and TNF-$\alpha$ production in human keratinocytes *[Journal of Investigative Dermatology,* volume 112, pages 91-96 (1999)] and it enhances a transglutaminase activity. In addition, in the horny layers of patients suffering from atopic dermatitis, an enzymatic activity for generation of SPC is enhanced as compared with healthy people and, therefore, accumulation of SPC is observed [*Journal of Investigative Dermatology*, volume 106, pages 1242-1249 (1996)]. It has been also reported that herbal medicine components which suppress a $Ca^{2+}$ increase induced by SPC are effective for atopic dermatitis (JP-A-9-124500). However, there has been no report showing a direct participation of SPC in itching. Kuraishi, et al. reported that scratching behavior is induced by subcutaneous administration of compound 48/80 or substance P to mouse [*European Journal of Pharmacology*, volume 275, pages 229-233 (1995)]. Since then, scratching behavior has been used as an animal model for itching but there has been no report that SPC induces scratching behavior.

LPC, which is another ligand for GPR4, accumulates in a diseased area of psoriasis [*British Journal of Pharmacology,* volume 134, pages 398-402 (1995)] and, when it is intracutaneously administered to a human being, edema, erythema and cellular infiltration are induced [*Acta. Dermato. Venereologica,* volume 80, pages 242-246 (2000)] whereby its participation in dermatitis is suggested. It has been reported that both SPC and LPC bind to each receptor of OGR-1 [*Nature Cell Biology,* volume 2, pages 261-267 (2000)] and G2A [*Science*, volume 293, pages 702-705 (2001)] besides GPR4 and induce signal transduction although action of each receptor has not been elucidated yet.

In WO 02/24222, it is literally disclosed to use a GPR4 antagonist for the treatment of atopic dermatitis.

It has been known that diseases causing itching are quite a lot and examples of such diseases are various skin diseases (atopic dermatitis, eczema/dermatitis, urticaria, pruritus, xeroderma, bite, scabies, fungal infection, skin itching, hypertrophic cicatrix, psoriasis, blister, drug eruption, etc.), liver/biliary tract diseases (primary biliary cirrhosis, cholestasis, cirrhosis, etc.), renal diseases (chronic renal insufficiency etc.), endocrinal/metabolic diseases (diabetes, thyroid dysfunction, etc.), blood diseases (polycythemia vera, iron deficiency anemia, etc.), malignant tumors (malignant lymphoma, gastral cancer, etc.), nerve diseases (multiple sclerosis, neurosis, etc.), AIDS and the like. There are some cases where itching is caused by pregnancy, drug, etc. [*Iyaku Journal* (published by Iyaku Journal Sha), volume 37, no. 11 (published on November 1, 2001), pages 73-77 and 79-82].

Among the GPCRs, there has been known a GPCR called a constitutively activated GPCR, which transduces signals even in the absence of a ligand when it is excessively expressed in cells. A signal which is transduced in the absence of a ligand is called a constitutive activity. Among the constitutively activated GPCRs, there are those which are present in nature and those which are mutated by introducing a mutation such as substitution, deletion, etc. of amino acids [*Molecular Pharmacology,* volume 57, page 890 (2000); WO 98/46995]. An antagonist which suppresses the constitutive activity of GPCR is called an inverse agonist.

In the literatures [*Bulletin de la Société Chimique,* page 185 (1981) and *European Journal of Medicinal Chemistry,* volume 12, page 219 (1977)], compounds wherein $R^1$ represents morpholino, $R^2$, $R^3$ and $R^4$ represent hydrogen,

a substituent corresponding to Y is morpholino, n is 1, and X represents $-(CH_2)_2-$ in the formula (I) which is described later are disclosed.

Disclosure of the Invention

An object of the present invention is:

1) to provide an agent for prevention and/or treatment of itching, which comprises, as an active ingredient, a substance capable of suppressing the function involved in signal transduction of GPR4,
2) to provide a nitrogen-containing tricyclic compound or a quaternary ammonium salt thereof, or a pharmaceutically acceptable salt thereof, which has an action of prevention and/or treatment of itching or has a suppressive action for the function involved in signal transduction of GPR4,
3) to provide an agent for prevention and/or treatment of itching comprising a nitrogen-containing tricyclic compound or a quaternary ammonium salt thereof, or a pharmaceutically acceptable salt thereof as an active ingredient, and
4) to provide a suppressor of the function involved in signal transduction of GPR4 comprising a nitrogen-containing tricyclic compound or a quaternary ammonium salt thereof, or a pharmaceutically acceptable salt thereof as an active ingredient.

Another object of the present invention is to provide a method for screening a therapeutic agent for itching, wherein reduction in the number of scratching behavior induced by SPC is used as an index.
The present invention relates to the following (1) to (22).

(1) An agent for prevention and/or treatment of itching, which comprises, as an active ingredient, a substance capable of suppressing the function involved in signal transduction of a protein having the amino acid sequence represented by SEQ ID NO: 11.
(2) An agent for prevention and/or treatment of itching, which comprises one of the following 1) to 4) as an active ingredient:

1) an oligonucleotide having a sequence complementary to that of oligonucleotide comprising continuous 5 to 60 nucleotides selected from the nucleotide sequence represented by SEQ ID NO: 12 or a derivative of said oligonucleotide,
2) an oligonucleotide having a sequence complementary to that of oligonucleotide comprising continuous 5 to 60 nucleotides selected from the nucleotide sequence represented by SEQ ID NO: 14 or a derivative of said oligonucleotide,
3) an oligonucleotide having a sequence complementary to that of oligonucleotide comprising continuous 5 to 60 nucleotides selected from the nucleotide sequence represented by SEQ ID NO: 18 or a derivative of said oligonucleotide, and
4) an oligonucleotide comprising 5 to 60 nucleotides which hybridizes under stringent conditions with DNA having the nucleotide sequence represented by one member selected from SEQ ID NOs: 12, 14 and 18 and which is capable of suppressing the function involved in signal transduction of a protein having the amino acid sequence represented by SEQ ID NO: 11 or a derivative of said oligonucleotide.

(3) An agent for prevention and/or treatment of itching, which comprises one of the following 1) to 4) as an active ingredient:

1) an antibody which recognizes a protein having the amino acid sequence represented by SEQ ID NO: 11,
2) an antibody which recognizes a protein having the amino acid sequence represented by SEQ ID NO: 13,
3) an antibody which recognizes a protein having the amino acid sequence represented by SEQ ID NO: 17, and
4) an antibody, which recognizes a protein having the amino acid sequence in which one or more amino acid (s) is/are deleted, substituted or added in the amino acid sequence represented by one member selected from SEQ ID NOs:11, 13 and 17 and which has the function involved in signal tranduction of a protein having the amino acid sequence represented by SEQ ID NO:11

(4) A nitrogen-containing tricyclic compound represented by the formula (I) or a quaternary ammonium salt thereof, or a pharmaceutically acceptable salt thereof;

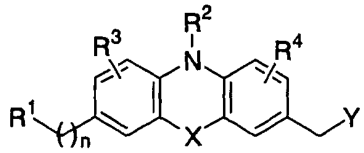

(I)

[wherein $R^1$ represents a substituted or unsubstituted heterocyclic group, $-NR^5R^6$ (wherein $R^5$ and $R^6$ are the same or different and each represents hydrogen, substituted or unsubstituted lower alkyl, substituted or unsubstituted cycloalkyl, substituted or unsubstituted lower alkenyl, substituted or unsubstituted lower alkynyl, substituted or unsubstituted aralkyl or substituted or unsubstituted heterocyclic alkyl, or $R^5$ and $R^6$ are combined together with the adjacent nitrogen atom to form a substituted or unsubstituted heterocyclic group), $-OR^7$ (wherein $R^7$ represents hydrogen, substituted or unsubstituted lower alkyl, substituted or unsubstituted lower alkanoyl, substituted or unsubstituted cycloalkyl, substituted or unsubstituted lower alkenyl, substituted or unsubstituted lower alkynyl, substituted or unsubstituted aryl, substituted or unsubstituted aralkyl or substituted or unsubstituted heterocyclic alkyl), $-SR^{7a}$ (wherein $R^{7a}$ has the same meaning as the above $R^7$), $-CONR^{5a}R^{6a}$ (wherein $R^{5a}$ and $R^{6a}$ have the same meanings as the above $R^5$ and $R^6$, respectively), $-CO_2R^{7b}$ (wherein $R^{7b}$ has the same meaning as the above $R^7$), $-N^+R^{5b}R^{6b}R^8$ (wherein $R^{5b}$ and $R^{6b}$ have the same meanings as the above $R^5$ and $R^6$, respectively, and $R^8$ represents lower alkyl, lower alkenyl or aralkyl), formyl, carboxy or cyano;

$R^2$ represents hydrogen, substituted or unsubstituted lower alkyl, substituted or unsubstituted cycloalkyl, substituted or unsubstituted lower alkenyl, substituted or unsubstituted lower alkynyl, substituted or unsubstituted aralkyl or substituted or unsubstituted heterocyclic alkyl;

$R^3$ and $R^4$ are the same or different and each represents hydrogen, lower alkyl or halogen;

n represents 0 or 1;

X represents $-(CH_2)_2-$ or -CH=CH-; and

Y represents the formula (II);

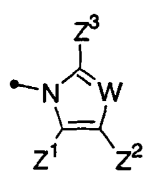

(II)

(wherein W represents CH or a nitrogen atom; $Z^1$ and $Z^2$ are the same or different and each represents hydrogen, substituted or unsubstituted lower alkyl, substituted or unsubstituted cycloalkyl, substituted or unsubstituted lower alkenyl, substituted or unsubstituted lower alkynyl, substituted or unsubstituted aryl, substituted or unsubstituted aralkyl or substituted or unsubstituted heterocyclic alkyl, or $Z^1$ and $Z^2$ are combined together with two carbon atoms being adjacent to each of them to form a substituted or unsubstituted aromatic ring or substituted or unsubstituted heterocycle; and

$Z^3$ represents hydrogen, substituted or unsubstituted lower alkyl, substituted or unsubstituted cycloalkyl, substituted or unsubstituted lower alkenyl, substituted or unsubstituted lower alkynyl, substituted or unsubstituted aryl, a substituted or unsubstituted heterocyclic group, substituted or unsubstituted aralkyl or substituted or unsubstituted heterocyclic alkyl)].

(5) The nitrogen-containing tricyclic compound or the quaternary ammonium salt thereof, or the pharmaceutically acceptable salt thereof according to (4), wherein $R^1$ is $-NR^5R^6$ and $R^5$ and $R^6$ are combined together with the adjacent nitrogen atom to form a substituted or unsubstituted heterocyclic group.

(6) The nitrogen-containing tricyclic compound or the quaternary ammonium salt thereof, or the pharmaceutically acceptable salt thereof according to (4) or (5), wherein $R^2$ is hydrogen.

(7) The nitrogen-containing tricyclic compound or the quaternary ammonium salt thereof, or the pharmaceutically acceptable salt thereof according to any one of (4) to (6), wherein $R^3$ and $R^4$ are hydrogen.

(8) The nitrogen-containing tricyclic compound or the quaternary ammonium salt thereof, or the pharmaceutically acceptable salt thereof according to any one of (4) to (7), wherein $Z^1$ and $Z^2$ are combined together with two carbon atoms being adjacent to each of them to form substituted or unsubstituted heterocycle.

(9) A pharmaceutical composition comprising the nitrogen-containing tricyclic compound or the quaternary ammonium salt thereof, or the pharmaceutically acceptable salt thereof according to any one of (4) to (8) as an acitive

ingredient.

(10) An agent for prevention and/or treatment of itching comprising the nitrogen-containing tricyclic compound or the quaternary ammonium salt thereof, or the pharmaceutically acceptable salt thereof according to any one of (4) to (8) as an acitive ingredient.

(11) A suppressor of the function involved in signal transduction of a protein having the amino acid sequence represented by SEQ ID NO: 11, comprising the nitrogen-containing tricyclic compound or the quaternary ammonium salt thereof, or the pharmaceutically acceptable salt thereof according to any of (4) to (8) as an acitive ingredient.

(12) A method for screening a therapeutic agent for itching, which comprises the following steps 1) to 4):

1) the step where sphingosyl phosphorylcholine (SPC) is subcutaneously and intracutaneously administered to a mammal except a human being so that scratching behavior in the mammal except a human being is induced,
2) the step where numbers of scratching behavior in the mammal except a human being induced by SPC in the presence or absence of the test compound are measured,
3) the step where numbers of scratching behavior in the mammal except a human being induced by SPC in the presence of the test compound and in the absence of the test compound are compared, and
4) the step where a substance which decreases the number of scratching behavior induced by SPC is selected from test compounds.

(13) A method for prevention and/or treatment of itching, which comprises administering an effective amount of the nitrogen-containing tricyclic compound or the quaternary ammonium salt thereof, or the pharmaceutically acceptable salt thereof described in any one of (4) to (8).

(14) Use of the nitrogen-containing tricyclic compound or the quaternary ammonium salt thereof, or the pharmaceutically acceptable salt thereof described in any one of (4) to (8) for the manufacture of an agent for prevention and/or treatment of itching.

(15) The agent for prevention and/or treatment of itching according to anyone of (1) to (3) and (10), wherein the itching is that which is accompanied by a disease selected from a skin disease, a liver/biliary tract disease, a renal disease, an endocrinal/metabolic disease, a blood disease, a malignant tumor, a nerve disease and AIDS.

(16) The agent for prevention and/or treatment of itching according to (15), wherein the itching is that accompanied by skin disease and said skin disease is that selected from atopic dermatitis, eczema/dermatitis, urticaria, pruritus, xeroderma, bite, scabies, fungal infection, skin itching, hypertrophic cicatrix, psoriasis, blister and drug eruption.

(17) A method for prevention and/or treatment of itching, which comprises administering a therapeutically effective amount of a substance capable of suppressing the function involved in signal transduction of a protein having the amino acid sequence mentioned in SEQ ID NO: 11.

(18) A method for prevention and/or treatment of itching, which comprises administering a therapeutically effective amount of an oligonucleotide or a derivative of said oligonucleotide which is any of one of 1) to 4) described in (2).

(19) A method for prevention and/or treatment of itching, which comprises administering a therapeutically effective amount of an antibody which is any one of 1) to 4) described in (3).

(20) Use of a substance capable of suppressing the function involved in signal transduction of a protein having the amino acid sequence represented by SEQ ID NO: 11 for the manufacture of an agent for prevention and/or treatment of itching.

(21) Use of an oligonucleotide or a derivative of said oligonucleotide which is any one of 1) to 4) described in (2) for the manufacture of an agent for prevention and/or treatment of itching.

(22) Use of an antibody which is any one of 1) to 4) described in (3) for the manufacture of an agent for prevention and/or treatment of itching.

The present inventors have found a new finding that a substance capable of suppressing the function involved in signal transduction of GPR4 which belongs to GPCRs is effective for prevention and/or treatment of itching and have achieved the present invention. The present inventors have searched substances capable of suppressing the constitutive activity of GPR4 which belongs to constitutively activated GPCRs and have found that a substance capable of suppressing the constitutive activity of GPR4 is effective for prevention and/or treatment of itching.

Substances capable of suppressing the function involved in signal transduction of GPR4 include a substance capable of inhibiting or suppressing the expression of GPR4 itself, a substance capable of inhibiting the binding of a ligand to GPR4, a substance capable of suppressing signal transduction caused by the binding of a ligand to GPR4 [such as changes (rise or fall) in intracellular cAMP concentration, changes (rise) in intracellular $Ca^{2+}$ concentration and phosphorylation of mitogen-activated protein (MAP) kinase], a substance capable of suppressing signal transduction caused by a constitutive activity of GPR4 (such as an inverse agonist of GPR4), etc. There is no particular limitation

for the structure for the above substances so far as they carry such functions, and substances having a known structure may be acceptable as well. Examples of GPR4 are a protein having the amino acid sequence represented by any one selected from SEQ ID NOs: 11, 13 and 17, a protein having the amino acid sequence in which one or more amino acid (s) is/are deleted, substituted or added in the amino acid sequence represented by any one selected from SEQ ID NOs: 11, 13 and 17 and which has the function involved in signal transduction of a protein having the amino acid sequence represented by SEQ ID NO: 11, etc.

The protein having an amino acid sequence wherein one or more amino acid(s) are/is deleted, substituted or added in the amino acid sequence represented by any one selected from SEQ ID NOs: 11, 13 and 17 and also having a function involved in the signal transduction of protein having the amino acid sequence represented by SEQ ID NO: 11 can be obtained, for example, by introducing a site-specific mutation into DNA encoding a protein having an amino acid sequence represented by any one selected from SEQ ID NO: 11, 13 and 17 by site-directed mutagenesis described in the literatures [Molecular Cloning, A Laboratory Manual, Second Edition, Cold Spring Harbor Laboratory Press (1989) (hereinafter, abbreviated as "Molecular Cloning, Second Edition"); Current Protocols in Molecular Biology, John Wiley & Sons (1987-1997) (hereinafter, abbreviated as "Current Protocols in Molecular Biology"); *Nucleic Acids Research*, 10, 6487 (1982); *Proc. Natl. Acad. Sci. USA,* 79, 6409 (1982); *Gene*, 34, 315 (1985); *Nucleic Acids Research,* 13, 4431 (1985); *Proc. Natl. Acad. Sci. USA,* 82, 488 (1985); etc.].

Although there is no particular limitation for the number(s) of amino acid(s) which is/are deleted, substituted or added, it/they is/are from 1 to several tens, preferably from 1 to 20, more preferably 1 to 10 and, still more preferably, 1 to 5.

The expression "one or more amino acid(s) are/is deletion, substitution or addition in the amino acid sequence represented by any one selected from SEQ ID NO: 11, 13 and 17" means that the amino acid sequence may contain deletion, substitution or addition of single or plural amino acid residue(s) at an arbitrary postion therein. Deletion, substitution or addition may be stimultaneously contained in one sequence and, the amino acid residue(s) to be deleted, substituted or added may be either natural or non-natural. Examples of natural amino acid residues are L-alanine, L-asparagine, L-aspartic acid, L-glutamine, L-glutamic acid, glycine, L-histidine, L-isoleucine, L-leucine, L-lysine, L-arginine, L-methionine, L-phenylalanine, L-proline, L-serine, L-threonine, L-tryptophan, L-tyrosine, L-valine and L-cysteine.

Preferred examples of amino acid residue(s) which is/are mutually able to be substituted are shown as hereunder. Amino acid residues belonging to the same group are able to be mutually substituted.

Group A: Leucine, isoleucine, norleucine, valine, norvaline, alanine, 2-aminobutanoic acid, methionine, O-methylserine, tert-butylglycine, tert-butylalanine and cyclohexylalanine;
Group B: Aspartic acid, glutamic acid, isoaspartic acid, isoglutamic acid, 2-aminoadipic acid and 2-aminosuberic acid;
Group C: Asparagine and glutamine;
Group D: Lysine, arginine, ornithine, 2,4-diaminobutanoic acid and 2,3-diaminopropionic acid;
Group E: Proline, 3-hydroxyproline and 4-hydroxyproline;
Group F: Serine, threonine and homoserine; and
Group G: Phenylalanine and tyrosine.

Also, in order that the protein having an amino acid sequence wherein one or more amino acid residue(s) is/ are deleted, substituted or added in the amino acid sequence represented by any one selected from SEQ ID NO: 11, 13 and 17 has a function involved in the signal transduction of the protein having the amino acid sequence represented by SEQ ID NO: 11, it is preferred that said amino acid sequence and the amino acid sequence represented by SEQ ID NO: 11 have a homology of at least 75%, usually not less than 80%, preferably not less than 90% or, more preferably, not less than 95%.

Homology of amino acid sequence and nucleotide sequence can be determined by using the algorithm BLAST by Karlin and Altschul [*Pro. Natl. Acad. Sci. USA,* 90, 5873 (1993)] or by FASTA [*Methods Enzymol. ,* 183, 63 (1990)]. On the basis of the algorithm BLAST, programs called BLASTN (database for nucleotide sequence vs. nucleotide sequence) and BLASTX (database for nucleotide sequence vs. amino acid sequence) have been developed [*J. Mol. Biol.,* 215, 403 (1990)]. When a nucleotide sequence is analyzed by BLASTN based upon BLAST, for example, parameters can be set to score = 100 and wordlength = 12. When an amino acid sequence is analyzed by BLASTX based upon BLAST, for example, parameters can be set to score = 50 and wordlength = 3. When BLAST and Gapped BLAST programs are used, a default parameter for each program can be used. [With regard to Gapped BLAST, refer to the literature (*Nuc. Acids Res.*, 25, 3389-3402 (1997)).] Specific means for those analytical methods are known (refer to http://www.ncbi.nlm.nih.gov).

Examples of a substance capable of inhibiting or suppressing the expression of GPR4 itself are an oligonucleotide (hereinafter, referred to as "antisense oligonucleotide") having a complementary sequence of oligonucleotide

comprising continuous 15 to 60 nucleotides selected from the nucleotide sequence represented by any one selected from SEQ ID NO: 12, 14 and 18; an oligonucleotide which hybridizes under stringent conditions with DNA having the nucleotide sequence represented by any one selected from SEQ ID NO: 12, 14 and 18 and suppresses the function involved in signal transduction of protein having the amino acid sequence represented by SEQ ID NO: 11; a derivative of the oligonucleotide as such (hereinafter, referred to as "oligonucleotide derivative"); etc.

The antisence oligonucleotide mentioned in the above is not particular limited so far as it is an antisense oligonucleotide having a sequence complementary to an oligonucleotide comprising continuous 15 to 60 nucleotides selected from the nucleotide sequences represented by any one selected from SEQ ID NO: 12, 14 and 18 although preferred one is an antisense oligonucleotide having 17 to 60 nucleotides, more preferably 20 to 60 nucleotides and, still more preferably, 30 to 60 nucleotides. Particularly preferred one is an antisense oligonucleotide having a complementary sequence of translation initiation region of the above-mentioned oligonucleotide. Said antisense oligonucleotide can be prepared by a conventional method such as by the using a DNA synthesizer on the basis of information concerning a nucleotide sequence for a nucleotide sequence represented by any one selected from SEQ ID NOs: 12, 14 and 18 or for a nucleotide sequence of fragment thereof.

Examples of the oligonucleotide derivative are an oligonucleotide derivative wherein a phosphoric acid diester bond in an oligonucleotide is converted into a phosphorothioate bond; an oligonucleotide derivative wherein a phosphoric acid diester bond in an oligonucleotide is converted to an N3'-P5' phosphoamidate bond; an oligonucleotide derivative wherein a bond of phosphoric acid diester with ribose in an oligonucleotide is converted to a peptide-nucleic acid bond; an oligonucleotide derivative wherein uracil in an oligonucleotide is substituted with C-5 propynyluracil; an oligonucleotide derivative wherein uracil in an oligonucleotide is substituted with C-5 thiazolyluracil; an oligonucleotide derivative wherein cytosine in an oligonucleotide is substituted with C-5 propynylcytosine; an oligonucleotide derivative wherein cytosine in an oligonucleotide is substituted with a phenoxazine-modified cytosine; an oligonucleotide derivative wherein ribose in an oligonucleotide is substituted with 2'-O-propylribose; and an oligonucleotide derivative wherein ribose in an oligonucleotide is substituted with 2'-methoxyethoxyribose [*Saibo Kogaku,* 16, 1463 (1997)].

The expression of GPR4 itself can be inhibited or suppressed by using the above-mentioned antisense oligonucleotide or oligonucleotide derivative in accordance with an antisense RNA/DNA technology [*Bioscience and Industry,* 50, 322 (1992); *Kagaku,* 46, 681 (1991); *Biotechnology,* 9, 358 (1992); *Trends in Biotechnology,* 10, 87 (1992); *Trends in Biotechnology,* 10, 152 (1992); *Saibo Kogaku,* 16, 1463 (1997)], a triple helix technology [*Trends in Biotechnology,* 10, 132 (1992)], a ribozyme technology [*Current Opinion in Chemical Biology,* 3, 274 (1999); *FEMS Microbiology Reviews,* 23, 257 (1999); *Frontiers in Bioscience,* 4, D497 (1999); *Chemistry & Biology,* 6, R33 (1999); *Nucleic Acids Research,* 26, 5237 (1998); *Trends in Biotechnology,* 16, 438 (1998)] or a decoy DNA method [*Nippon Rinsho - Japanese Journal* of *Clinical Medicine*, 56, 563 (1998); *Circulation Research*, 82, 1023 (1998); *Experimental Nephrology,* 5, 429 (1997); *Nippon Rinsho - Japanese Journal of Clinical Medicine,* 54, 2583 (1996)].

The oligonucleotide which hybridizes under stringent conditions to DNA having the nucleotide sequence represented by any one selected from SEQ ID NOs: 12, 14 and 18 is a DNA which is obtained by colony hybridization, plaque hybridization. Southern blot hybridization, etc. using a part of or whole DNA having the nucleotide sequence represented by any one selected from SEQ ID NOs: 12, 14 and 18 as a probe. To be more specific, the DNA includes a DNA which can be identified by carrying out hybridization at 65°C in the presence of 0.7 to 1.0 mol/l sodium chloride using a filter on which a DNA prepared from colonies or plaque is immobilized, and then washing the filter under the condition of 65°C using an SSC solution of 0.1- to 2-fold concentration (composition of an SSC solution of a 1-fold concentration comprises 150 mmol/l of sodium chloride and 15 mmol/l of sodium citrate). Hybridization can be carried out according to a method mentioned, for example, in Molecular Cloning, Second Edition; Current Protocols in Molecular Biology; DNA Cloning 1: Core Techniques, A Practical Approach, Second Edition, Oxford University (1995). The oligonucleotide which is hybridizable includes a DNA having at least 75% homology preferably not less than 80% homology, more preferably not less than 95% homology to DNA having a complementary sequence of DNA having the nucleotide sequence represented by any one selected from SEQ ID Nos: 12, 14 and 18 when calculated by, for example, the above-mentioned BLAST or FASTA. DNA, RNA and the like can be used as the oligonucleotide, and DNA can be used preferably.

A formulation prepared according to the following conventional method from the antisense oligonucleotide or the derivative of said antisense oligonucleotide described in the above, or the oligonucleotide or the derivative of said oligonucleotide, wherein the oligonucleotide hybridizes under stringent conditions to DNA having the nucleotide sequence represented by any of selected from SEQ ID NOs: 12, 14 and 18 either solely or after inserting into a vector for gene therapy such as a retrovirus vector, an adenovirus vector or an adenovirus associated virus vector or the like, also can be used as an agent for prevention and/or treatment of itching.

When the vector for gene therapy is used as said agent for prevention and/or treatment, it is able to be manufactured by compounding said vector for gene therapy with a carrier used for gene therapy agent [*Nature Genet.,* 8, 42 (1994)].

With regard to the above carrier, any carrier may be used so far as it is a carrier which is commonly used for

injection preparations and its examples are distilled water, a salt solution such as sodium chloride or a mixture of sodium chloride or inorganic salt, a solution of saccharide such as mannitol, lactose, dextran and glucose, a solution of amino acid such as glycine and arginine and a mixed solution of organic acid solution or a salt solution with glucose solution. It is also possible that, in accordance with the common method, an excipient such as osmotic pressure adjusting agent, pH adjusting agent, plant oil such as sesame oil or soybean oil, lecithin or surfactant such as nonionic surfactant is added for the carrier whereupon an injection solution is prepared as solution, suspension or dispersion. It is further possible that such an injection solution is prepared in a form of being dissolved immediately before use after an operation such as pulverization and freeze-drying.

Said agent for prevention and/or treatment may be used as it is when the agent is liquid or, when the agent is solid, it may be used after dissolving, immediately before use, in the above-mentioned carrier which is subjected to a sterilization treatment if necessary.

An example of the method for administration is a local administration so that it is able to be absorbed with the site of the patient to be treated. It is also possible to transport the DNA to the aimed site for the treatment by means of a non-viral gene transfection.

Examples of the non-viral gene transfection method are a calcium phosphate coprecipitation method [*Virology,* 52, 456-467 (1973); *Science,* 209, 1414-1422 (1980)], a microinjection method [*Proc. Natl. Acad. Sci. USA,* 77, 5399-5403 (1980); *Proc. Natl. Acad. Sci. USA,* 77, 7380-7384 (1980); *Cell,* 27, 223-231 (1981); *Nature,* 294, 92-94 (1981)]; a membrane fusion-intervening transfection method using liposome [*Proc. Natl. Acad. Sci. USA,* 84, 7413-7417 (1987); *Biochemistry,* 28, 9508-9514 (1989); *J. Biol. Chem.,* 264, 12126-12129 (1989); *Hum. Gene Ther. ,* 3, 267-275 (1992); *Science,* 249, 1285-1288 (1990); *Circulation,* 83, 2007-2011 (1992)], a direct DNA-incorporation or receptor-mediated DNA transfection method [*Science,* 247, 1465-1468 (1990); *J. Biol. Chem.,* 266, 14338-14342 (1991); *Proc. Natl. Acad. Sci. USA,* 87, 3655-3659 (1991); *J. Biol. Chem.* 264, 16985-16987 (1989); *BioTechniques,* 11, 474-485 (1991); *Proc. Natl. Acad. Sci. USA,* 87, 3410-3414 (1990); *Proc. Natl. Acad. Sci. USA,* 88, 4255-4259 (1991); *Proc. Natl. Acad. Sci . USA,* 87, 4033-4037 (1990); *Proc. Natl. Acad. Sci. USA,* 88, 8850-8854 (1991); *Hum. Gene Ther.* 3, 147-154 (1991)], etc.

The substance which inhibits a binding of a ligand to GPR4 includes an antibody which recognizes GPR4, a compound which has an antagonistic action to GPR4 or the like.

With regard to the above-mentioned antibody, any antibody may be used so far as it is an antibody which recognizes GPR4 although an antibody which specifically recognizes GPR4 is preferred. Said antibody may be either a polyclonal antibody or a monoclonal antibody. With regard to such an antibody, an example is a neutraizing antibody which recognizes GPR4. A chimera antibody of a human type, a humanized antibody, and the like also can be used as an antibody of the present invention.

The above-mentioned antibody can, for example, be prepared according to the following methods.

(1) Preparation of a polyclonal antibody

A polyclonal antibody can be prepared in such a manner that purified sample of GPR4 or a partial fragment polypeptide thereof or a peptide having a part of amino acid sequence of GPR4 is used as an antigen and administered to an animal.

With regard to the animal to which administration is conducted, it is possible to use rabbit, goat, rat, mouse, hamster, etc.

The amount of said antigen to be adiministered is preferred to be 50 to 100 μg per animal.

When a peptide is used, it is preferred to use a product wherein a peptide is subjected to a covalent bond to a carrier protein such as keyhole limpet hemocyanin or bovine thyroglobulin or the like. A peptide used as an antigen may be synthesized by a peptide synthesizer.

Administration of said antigen is conducted for three to ten times every one or two week(s) after the first administration. After each administration, blood is collected from venous plexus of fundus of the eye on the third to the seventh day and it is confirmed by means of an enzyme-linked immunosorbent assay [ELISA Method: published by Igaku Shoin (1976); Antibodies - A Laboratory Manual, Cold Spring Harbor Laboratory (1988)] or the like that said serum reacts with an antigen used for immunization.

Serum is obtained from non-human mammals wherein the serum thereof shows a sufficient antibody value to the antigen used for immunization and said serum is separated and purified whereupon a polyclonal antibody is able to be prepared.

With regard to a method for the separation and the purification, it is possible to conduct a treatment by centrifugal separation, salting-out using 40-50% saturated ammonium sulfate, precipitation with caprylic acid [Antibodies - A Laboratory Manual, Cold Spring Harbor Laboratory, (1988)], chromatography using DEAE-Sepharose column, anion exchange column, protein A or G column, gel filtration column or the like, etc. either solely or in combination.

(2) Preparation of monoclonal antibody

(a) Preparation of antibody-producing cells

To the pure sample of GPR4 used for immunization or the partial fragment polypeptide thereof or to the peptide having an amino acid sequence of a part of GPR4 is used a rat where its serum shows a sufficient antibody value as a supplying source of antibody-producing cells.

After the third to the seventh day from the final administration of an antigen substance to the rat showing the antibody value, its spleen is excised.

The spleen is finely cut in an MEM medium (manufactured by Nissui Pharmaceutical), loosened using a forceps and centrifuged at 1,200 rpm for 5 minutes and a supernatant liquid is discarded.

Spleen cells of the resulting precipitate fraction is treated with Tris-ammonium chloride buffer (pH 7.65) for 1 to 2 minute(s) to remove erythrocytes and washed with an MEM medium for three times and the resulting spleen cells are used as antibody-producing cells.

(b) Preparation of myeloma cells

Established cell line prepared from mouse or rat is used as myeloma cell. For example, it is possible to use 8-azaguanine resistant mouse (derived from BALB/c) myeloma cell line P3-X63Ag8-U1 (hereinafter, abbreviated as P3-U1) [*Curr. Topics Microbiol. Immunol.,* 81, 1 (1978); *Eur. J. Immunol.,* 6, 511 (1976)], SP2/0-Ag14 (SP-2) [*Nature*, 276, 269 (1978)], P3-X63-Ag8653 (653) [*J. Immunol.,* 123, 1548 (1979)], P3-X63-Ag8 (X63) [*Nature*, 256, 495 (1975)], etc. Those cell lines are passaged using an 8-azaguanine medium [8-azaguanine (15 μg/ml) is further added to a medium (hereinafter, referred to as "normal medium") wherein glutamine (1.5 mmol/l), 2-mercaptoethanol ($5 \times 10^{-5}$ mol/l), gentamicin (10 μg/ml) and fetal bovine serum (FCS) (manufactured by CSL; 10%) are added to an RPMI-1640 medium]. Thus incubation is conducted on a normal medium before 3 to 4 days of cell fusion and not less than $2 \times 10^7$ of said cells are used for the fusion.

(c) Preparation of hybridoma

The antibody-producing cells prepared in (a) and the myeloma cells prepared in (b) are well washed with an MEM medium or a PBS (1.83 g of disodium phosphate, 0. 21 g of monopotassium phosphate, 7.65 g of sodium chloride and 1 liter of distilled water; pH 7.2) and mixed so as to make the ratio of (antibody-producing cells) : (myeloma cells) = 5 to 10 : 1, the mixture is centrifuged at 1,200 rpm for 5 minutes and a supernatant liquid is discarded.

Cell group of the resulting precipitate fraction is well loosened, 0.2 to 1 ml of a solution wherein 2 g of polyethylene glycol (PEG-1000), 2 ml of MEM and 0.7 ml of dimethyl sulfoxide (DMSO) are mixed is added to said cell group per $10^8$ antibody-producing cells with stirring at 37°C and then 1 to 2 ml of an MEM medium is added for several times every 1 to 2 minute(s).

After addition, an MEM medium is added so as to prepare a solution in an amount of 50 ml. Said prepared solution is centrifuged at 900 rpm for 5 minutes and a supernatant liquid is discarded. Cells of the resulting precipitate fraction are gently loosened and gently suspended in 100 ml of an HAT medium [a medium where hypoxanthine ($10^{-4}$ mol/l), thymidine ($1.5 \times 10^{-5}$ mol/l) and aminopterin ($4 \times 10^{-7}$ mol/l) are added to a normal medium] by means of suction and spouting using a measuring pipette.

Said suspension is dispensed in a 96-well incubation plate in an amount of 100 μl per well and is incubated in a 5% $CO_2$ incubator at 37°C for 7 to 14 days.

After the incubation, a part of a supernatant liquid thereof is taken out and a hybridoma which specifically reacts with the partial fragment polypeptide of the polypeptide of the present invention is selected by an enzymatic immunoassay mentioned, for example, in Antibodies, A Laboratory Manual, Cold Spring Harbor Laboratory, Chapter 14 (1988).

The following methods may be listed as specific examples of the enzymatic immunoassay.

In immunization, GPR4 used as antigen or a pure sample of a partial fragment polypeptide thereof or a peptide having a partial amino acid sequence of GPR4 is coated on an appropriate plate, subjected to a reaction using supernatant liquid of incubated hybridoma or the pure antibody prepared in (d) which is mentioned later as the first antibody, then subjected to a reaction using anti-rat or anti-mouse immunoglobulin labeled with biotin, enzyme, chemoluminescent substance or radiation compound as the second antibody and subjected to a reaction in accordance with a labeled substance and that which specifically reacts with a polypeptide used as an antigen is selected as a hybridoma which produces a monoclonal antibody to be used in the present invention.

Cloning by a limiting dilution method using said hybridoma is repeated twice [in the first one, an HT medium (a medium wherein aminopterin is removed from an HAT medium) is used and, in the second one, a normal medium

is used] and that which shows a strong antibody value in a stable manner is selected as a hybridoma strain producing a monoclonal antibody to be used in the present invention.

(d) Preparation of monoclonal antibody

The hybridoma cells (5 to 20 $\times$ $10^6$ cells/animal) producing monoclonal antibody to be used in the present invention which are prepared in (c) are intraperitoneally injected to mouse or nude mouse of 8 to 10 weeks age treated with pristane [0.5 ml of pristane (2,6,10,14-tetramethylpentadecane) is intraperitoneally injected followed by breeding for two weeks]. Hybridoma becomes ascites tumor within 10 to 21 days.
Ascites is collected from said mouse becoming ascites tumor and centrifuged at 3,000 rpm for 5 minutes to remove solid.
From the resulting supernatant liquid, monoclonal antibody is able to be purified and prepared by the method similar to that used for polyclonal antibody.
Determination of subclass of the antibody is carried out using a mouse monoclonal antibody typing kit or a rat monoclonal antibody typing kit. Amount of the polypeptide is calculated by a Lowry method or from the absorbance at 280 nm.
An agent for prevention and/or treatment of itching comprising the above-mentioned antibody capable of recognizing GPR4 is able to be prepared, for example, as follows.
With regard to a medicament comprising said antibody as an active ingredient, although it is possible that said active ingredient is administered solely, it is usually preferred to provide as a pharmaceutical preparation by mixing of said active ingredient with one or more pharmaceutically acceptable carrier(s) followed by manufacturing by any method which is well known in the technical field of pharmaceutical preparation sciences. Preferably, an aseptic solution being dissolved in an aqueous carrier such as water or aqueous solution of sodium chloride, glycine, glucose, human albumin, etc. is used. It is also possible to add a pharmaceutically acceptable additive such as buffering or isotonizing agent for making the pharmaceutical preparation solution nearer the physiological condition such as sodium acetate, sodium chloride, sodium lactate, potassium chloride and sodium citrate. It is further possible to preserve by freeze-drying and to dissolve in an appropriate solvent in actual use.
With regard to the administering route, it is desirable to use that which is most effective for the therapy and its examples are oral administration and parenteral administration such as intravenous injection. Examples of the dosage form are tablets and injections or the like.
With regard to the preparation suitable for oral administration, tablets may be exemplified. They are able to be manufactured using additives including excipient such as lactose and mannitol, disintegrating agent such as starch, lubricant such as magnesium stearate, binder such as hydroxypropyl cellulose, surfactant such as fatty acid ester, plasticizer such as glycerol, etc.
With regard to the preparation suitable for parenteral administration, injections may be exemplified. They may be prepared, for example, using a carrier comprising a salt solution, a glucose solution or a mixture thereof. It is also possible to use the components exemplified as additives in the oral preparation even in the case of parenteral preparation.
Dose and administering frequency may vary depending upon aimed therapeutic effect, administering method, period of treatment, age, body weight, etc. and, usually, they are from 10 μg/kg to 8 mg/kg per day for adults.
A substance which is capable of suppressing the function involved in signal transduction resulted from a constitutive activity of GPR4 is also able to be prepared by investigating the substances which are able to suppress the signal transduction resulted by said constitutive activity.
An example of the compounds having an antagonistic action to GPR4 is a compound represented by the formula (I). Hereinafter, a compound represented by the formula (I) is referred to as compound (I). That is also applied to compounds having other formula numbers.
In definitions of the groups in compound (I), the following exemplification is listed.

(i) With regard to the lower alkyl and the lower alkyl moiety in the lower alkanoyl, linear or branched alkyl having 1 to 10 carbon(s) may be exemplified and specific examples thereof are methyl, ethyl, propyl, isopropyl, butyl, isobutyl, sec-butyl, tert-butyl, pentyl, isopentyl, neopentyl, hexyl, heptyl, octyl, isooctyl, nonyl and decyl.
(ii) With regard to the cycloalkyl, cycloalkyl having 3 to 8 carbons may be exemplified and specific examples thereof are cyclopropyl, cyclobutyl, cyclopentyl, cyclohexyl, cycloheptyl and cyclooctyl.
(iii) With regard to the lower alkenyl, linear, branched or cyclic alkenyl having 2 to 8 carbons may be exemplified and specific examples thereof are vinyl, allyl, 1-propenyl, butenyl, pentenyl, hexenyl, heptenyl, octenyl, cyclohexenyl and 2,6-octadienyl.
(iv) With regard to the lower alkynyl, linear or branched alkynyl having 2 to 8 carbons may be exemplified and specific examples thereof are ethynyl, propynyl, butynyl, pentynyl, hexynyl, heptynyl and octynyl.

(v) The halogen means each of fluorine, chlorine, bromine and iodine atoms.

(vi) With regard to the aryl and a group wherein one hydrogen atom is removed from the aromatic ring formed together with two carbon atoms being adjacent to each, monocyclic, bicyclic or tricyclic aryl having 6 to 14 carbons may be exemplified and specific examples thereof are phenyl, naphthyl, indenyl and anthranyl.

(vii) The alkylene moiety of the aralkyl and the heterocyclic alkyl has the same meaning as that where one hydrogen atom is removed from the definition for the above lower alkyl (i).

(viii) With regard to the aryl moiety of the aralkyl, a bicyclic fused ring group wherein the above aryl is fused to cycloalkyl may be exemplified in addition to the groups exemplified in the definition for the above aryl (vi) and specific examples thereof are indanyl, 1,2,3,4-tetrahydronaphthyl and 6,7,8,9-tetrahydro-5H-benzocycloheptyl.

(ix) With regard to the heterocyclic group, the heterocyclic moiety of the heterocyclic alkyl and a group wherein one hydrogen atom is removed from the heterocyclic ring formed together with two carbon atoms being adjacent to each, a five- or six-membered monocyclic heterocyclic group containing at least one atom selected from a nitrogen atom, an oxygen atom and a sulfur atom and a fused ring heterocyclic group which is bicyclic or tricyclic where three- to eight-membered rings are fused and which contains at least one atom selected from a nitrogen atom, an oxygen atom and a sulfur atom may be exemplified and specific examples thereof are pyridyl, pyrazinyl, pyrimidinyl, pyridazinyl, benzimidazolyl, 2-oxobenzimidazolyl, benzotriazolyl, benzofuryl, benzothienyl, purinyl, benzoxazolyl, benzothiazolyl, benzodioxolyl, indazolyl, indolyl, isoindolyl, quinolyl, isoquinolyl, phthalazinyl, naphthylidinyl, quinoxalinyl, pyrrolyl, pyrazolyl, quinazolinyl, cinnolinyl, triazolyl, tetrazolyl, imidazolyl, oxazolyl, isoxazolyl, thiazolyl, isothiazolyl, thienyl, furyl, pyrrolidinyl, 2,5-dioxopyrrolidinyl, thiazolidinyl, oxazolidinyl, piperidyl, piperidino, piperazinyl, homopiperazinyl, homopiperidyl, homopiperidino, morpholinyl, morpholino, thiomorpholinyl, thiomorpholino, pyranyl, tetrahydropyridyl, tetrahydropyranyl, tetrahydrofuranyl, tetrahydroquinolyl, tetrahydroisoquinolyl, octahydroquinolyl and indolinyl.

(x) With regard to the heterocyclic group formed together with the adjacent nitrogen atom, a five- to six-membered monocyclic heterocyclic group containing at least one nitrogen atom (said monocyclic heterocyclic group may contain other nitrogen atom, oxygen atom or sulfur atom) and a fused ring heterocyclic group which is bicyclic or tricyclic where three-to eight-membered rings are fused and which contains at least one nitrogen atom (said fused ring heterocyclic group may contain other nitrogen atom, oxygen atom or sulfur atom) may be exemplified and specific examples thereof are pyridyl, tetrahydropyridyl, indolinyl, isoindolinyl, pyrrolidinyl, thiazolinyl, oxazolidinyl, piperidino, homopiperidino, piperazinyl, homopiperazinyl, morpholino, thiomorpholino, perhydroazepinyl, perhydroazocinyl, tetrahydroquinolyl, tetrahydroisoquinolyl, octahydroquinolyl, benzimidazolyl, indazolyl, indolyl, isoindolyl, purinyl, dihydroindolyl, pyrrolyl, dihydropyrrolyl, pyrazolyl, triazolyl, tetrazolyl and imidazolyl.

(xi) With regard to the substituent in the substituted lower alkyl and the substituted lower alkanoyl, the number of the substituent(s), which may be the same or different, is 1 to 3 and examples thereof are the following. Thus, cycloalkyl, lower alkanoyl, lower alkoxy, aryloxy, substituted aryloxy [with regard to the substituent in said substituted aryloxy, the number of the substituent(s), which may be the same or different, is 1 to 3 and examples thereof are lower alkyl, lower alkoxy, lower alkoxycarbonyl, halogen, cyano, nitro, hydroxy, carboxy and amino; here, the lower alkyl has the same meaning as the above-mentioned lower alkyl (i), the halogen has the same meaning as the above-mentioned halogen (v) and the lower alkyl moiety in the lower alkoxy and the lower alkoxycarbonyl has the same meaning as the above-mentioned lower alkyl (i)], aralkyloxy, substituted aralkyloxy [with regard to the substituent in said substituted aralkyloxy, the number of the substituent(s), which may be the same or different, is 1 to 3 and examples thereof are lower alkyl, lower alkoxy, lower alkoxycarbonyl, halogen, cyano, nitro, hydroxy, carboxy and amino; here, the lower alkyl has the same meaning as the above-mentioned lower alkyl (i), the halogen has the same meaning as the above-mentioned halogen (v) and the lower alkyl moiety in the lower alkoxy and the lower alkoxycarbonyl has the same meaning as the above-mentioned lower alkyl (i)], lower alkanoyoxy, lower alkoxycarbonyl, halogen, cyano, nitro, hydroxy, carboxy, amino, lower alkylthio, substituted lower alkyl [with regard to the substituent in said substituted lower alkyl, the number of the substituent(s), which may be the same or different, is 1 to 3 and examples thereof are hydroxy and halogen], the substituted lower alkanoyl [with regard to the substituent in the substituted lower alkanoyl, the number of the substituent(s), which may be the same or different, is 1 to 3 and an example thereof is halogen], mono- or di-lower alkylamino, lower alkylsulfonyl, lower alkylsulfinyl, lower alkoxycarbonylamino, lower alkanoylamino, mono- or di-lower alkylaminocarbonyl, mono- or di-lower alkylaminocarbonyloxy, a heterocyclic group and the like are exemplified.

The aryl moiety in the aryloxy and the aralkyloxy, the cycloalkyl, the halogen, the heterocyclic group and the lower alkyl moiety in the lower alkanoyl, the lower alkoxy, the lower alkanoyloxy, the lower alkoxycarbonyl, the lower alkylthio, the lower alkylsulfonyl, the lower alkylsulfinyl, the lower alkoxycarbonylamino and the lower alkanoylamino shown here have the same meanings as the above-mentioned aryl (vi), cycloalkyl (ii), halogen (v), heterocyclic group (ix) and lower alkyl (i), respectively. The alkylene moiety in the aralkyloxy has the same meaning as that where one hydrogen atom is removed from the above-mentioned lower alkyl (i).

The lower alkyl moiety of the mono- or di-lower alkylamino, the mono- or di-lower alkylaminocarbonyl and the

mono- or di-lower alkylaminocarbonyloxy each has the same meaning as the above-mentioned lower alkyl (i). Two lower alkyl moieties in the di-lower alkylamino, the di-lower alkylaminocarbonyl and the di-lower alkylaminocarbonyloxy each may be the same or different.

(xii) With regard to the substituent in the substituted aryl, the substituted aralkyl, the substituted cycloalkyl, the substituted lower alkenyl, the substituted lower alkynyl, the substituted heterocyclic group, the substituted heterocyclic alkyl, the substituted heterocyclic group formed together with the adjacent nitrogen atom, the substituted aromatic ring formed together with two carbon atoms being adjacent to each and the substituted heterocycle formed together with two carbon atoms being adjacent to each, in addition to the above-mentioned groups exemplified in the definition for the substituent (xi) in the substituted lower alkyl are lower alkyl, aryl, substituted aryl, aralkyl, substituted aralkyl, a heterocyclic group, a substituted heterocyclic group, heterocyclic alkyl, substituted heterocyclic alkyl and the like are exemplified. Further, with regard to the substituent in the substituted aryl and the substituted heterocyclic group formed together with the adjacent nitrogen atom, it may be lower alkyl [said lower alkyl has the same meaning as the above-mentioned lower alkyl (i)] or substituted lower alkyl [said lower alkyl has the same meaning as the above-mentioned lower alkyl (i), and the number of the substituent(s), which may be the same or different, is 1 to 3 and examples thereof are halogen, hydroxy, carboxy and lower alkoxycarbonyl; here, the halogen has the same meaning as the above-mentioned halogen (v) and the lower alkyl moiety of the lower alkoxycarbonyl has the same meaning as the above-mentioned lower alkyl (i)].

The lower alkyl, the aryl, the heterocyclic group moiety of the heterocyclic group and the heterocyclic alkyl, the alkylene moiety of the aralkyl and the heterocylic alkyl, and the aryl moiety of the aralkyl shown here have the same meanings as the above-mentioned lower alkyl (i), aryl (vi), heterocylic group (ix), alkylene moiety (vii) of the aralkyl and aryl moiety (viii) of the aralkyl, respectively. With regard to the substituent in the substituted aryl, the substituted aralkyl, the substituted heterocyclic group and the substituted heterocyclic alkyl, the number of the substituent(s), which may be the same or different, is 1 to 3 and examples thereof are lower alkyl [said lower alkyl has the same meaning as the above-mentioned lower alkyl (i)], lower alkoxy [the lower alkyl moiety of said lower alkoxy has the same meaning as the above-mentioned lower alkyl (i)] and halogen [said halogen has the same meaning as the above-mentioned halogen (v)].

With regard to the quaternary ammonium salt of compound (I), there is no particular limitation so far as it is a quaternary ammonium salt wherein halogenated lower alkyl (said lower alkyl and said halogen have the same meanings as the above-mentioned ones, respectively), halogenated aralkyl (said halogen and said aralkyl have the same meanings as the above-mentioned ones, respectively), hydroxy lower alkyl (said lower alkyl has the same meaning as the above-mentioned one) or the like is added to a basic moiety of compound (I) and examples thereof are a quaternary ammonium salt prepared by the reaction of compound (I) having a dimethylamino group with methyl iodide, a quaternary ammonium salt prepared by the reaction of compound (I) having a piperidino group with methyl iodide, a quaternary ammonium compound prepared by the reaction of compound (I) having a pyrrolidino group with methyl iodide, a quaternary ammonium salt prepared by compound (I) having a morpholino group with benzyl bromide and a quaternary ammonium salt prepared by exchange of an iodide ion with a hydroxide ion in a quaternary ammonium salt prepared by the reaction of compound (I) having a pyrrolidino group with ethyl iodide.

With regard to the pharmaceutically acceptable salt of compound (I), that which has no toxicity and is soluble in water is preferred and examples thereof are acid addition salts such as inorganic salts including hydrochloride, hydrobromide, nitrate, sulfate, phosphate, etc. and organic salts including benzenesulfonate, benzoate, citrate, fumarate, gluconate, lactate, maleate, malate, oxalate, methanesulfonate, tartrate, etc., alkaline metal salts such as sodium salt and potassium salt etc., alkaline earth metal salt such as magnesium salt and calcium salt etc., metal salts such as aluminum salt and zinc salt, ammonium salt such as ammonium and tetramethylammonium etc., organic amine addition salts such as morpholine addition salt and piperidine addition salt etc. and amino acid addition salt such as glycine addition salt, phenylalanine addition salt, lysine addition salt, aspartic acid addition salt and glutamic acid addition salt etc.

Process for the production of compound (I) will be illustrated as hereunder.

In the process for the production as mentioned below, when the defined group changes under a reaction condition or when it is not appropriate for conducting the process, the production may be easily carried out by subjecting to a method commonly used in synthetic organic chemistry such as by means of protection and deprotection of functional group [e.g., Protective Groups in Organic Synthesis, the third edition, by T. W. Greene and Peter G. M. Wuts, John Wiley & Sons, Inc. (1999)]. If necessary, order of reaction steps such as introduction of substituents may be changed.

Compound (I-a) may be prepared by the production process as shown below.

Production Process 1

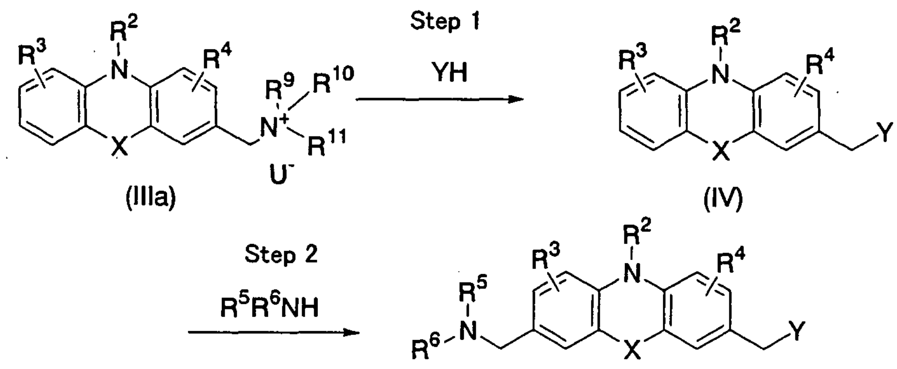


(IIIa) (IV) (I-a)

(wherein $R^2$, $R^3$, $R^4$, $R^5$, $R^6$, X and Y each have the same meaning as defined above, respectively; $R^9$ represents lower alkyl, allyl or benzyl; $R^{10}$ and $R^{11}$ are the same or different and each represents lower alkyl or cycloalkyl, or $R^{10}$ and $R^{11}$ may form a heterocyclic group together with the adjacent nitrogen atom; and U represents halogen, alkoxysulfonyloxy, aryloxysulfonyloxy, alkylsulfonyloxy or arylsulfonyloxy.)

In the above definitions, the lower alkyl, the cycloalkyl and the halogen each have the same meaning as that mentioned above, respectively. The alkyl moiety of the alkoxysulfonyloxy and the alkylsulfonyloxy and the aryl moiety of the aryloxysulfonyloxy and the arylsulfonyloxy each have the same as the above-mentioned lower alkyl and aryl, respectively. The heterocyclic group formed together with the adjacent nitrogen atom has the same meaning as the above-mentioned one.

<Step 1>

Compound (IIIa) is used as a starting material and is made to react with from one equivalent to a large excess of YH (wherein Y has the same meaning as defined above) by a process disclosed in the JP-A-7-61983 to give compound (IV). Incidentally, compound (IIIa) is able to be synthesized by the process disclosed in the JP-A-7-61983 or a process similar thereto.

<Step 2>

Compound (IV) is made to react with one equivalent to a large excess of $R^5R^6NH$ (wherein $R^5$ and $R^6$ each have the same meaning as defined above, respectively) or a hydrochloride thereof in an inert solvent in the presence of one equivalent to a large excess of aqueous formaldehyde solution to give compound (I-a). It is also possible to use a substance equivalent to formaldehyde such as trioxymethylene and paraformaldehyde instead of an aqueous formaldehyde solution.

Since the reaction usually well proceeds under an acidic condition, it is preferred to add an acid such as hydrochloric acid, acetic acid or trifluoroacetic acid to the reaction system if necessary. The reaction is usually carried out at the temperature between 0°C and the boiling point of the solvent used for the reaction, preferably, from room temperature to 80°C and finishes within from 5 minutes to 100 hours. With regard to the inert solvent, water, methanol, ethanol, acetic acid, trifluoroacetic acid, dichloroethane, chloroform, tetrahydrofuran, dimethylacetamide, dimethylformamide, acetone or the like may be used either solely or as as the mixture thereof. Preferably, a mixed solvent of chloroform and acetic acid is used.

Compound (I-c) is produced from compound (I-b) by a process as shown below.

## Production Process 2

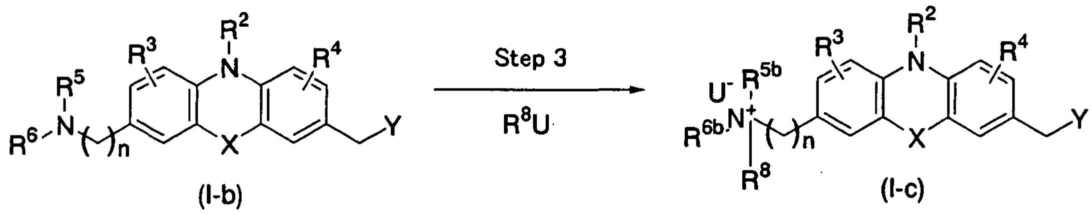


(I-b) (I-c)

(wherein $R^2$, $R^3$, $R^4$, $R^5$, $R^6$, $R^{5b}$, $R^{6b}$, $R^8$, U, n, X and Y each have the same meaning as defined above, respectively.)

<Step 3>

Compound (I-b) is made to react with from one equivalent to a large excess of $R^8U$ (wherein $R^8$ and U each have the same meaning as defined above, respectively) in an inert solvent for 1 to 48 hour(s) at the temperature of usually from -10°C to the boiling point of the solvent used for the reaction, preferably, at room temperature to give compound (I-c).

With regard to the inert solvent, it is possible to use, for example, water, methanol, ethanol, benzene, toluene, xylene, ethyl acetate, hexane, acetonitrile, dichloromethane, dichloroethane, chloroform, carbon tetrachloride, 1,4-dioxane, tetrahydrofuran, dimethylacetamide, dimethylformamide or acetone or the like either solely or as the mixture thereof. Preferably, ethyl acetate, dichloroethane, chloroform, etc. are used.

Compound (I-b) is able to be produced from compound (I-c) by the process as shown below.

## Producing Process 3

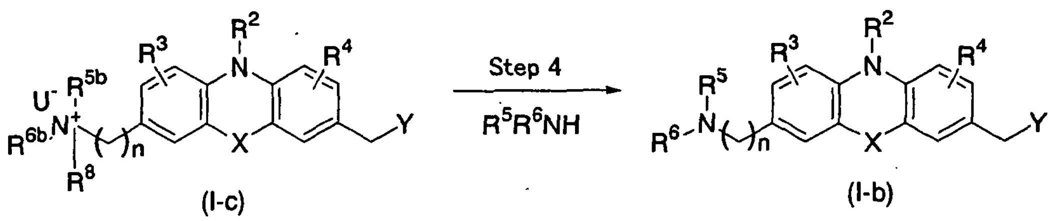


(I-c) (I-b)

(wherein $R^2$, $R^3$, $R^4$, $R^5$, $R^6$, $R^{5b}$, $R^{6b}$, U, n, X and Y each have the same meaning as defined above, respectively.)

<Step 4>

Compound (I-c) is made to react with from one equivalent to a large excess of $R^5R^6NH$ (wherein $R^5$ and $R^6$ each have the same meaning as defined above, respectively) in an inert solvent for 1 to 100 hour(s) at the temperature of usually from -10°C to the boiling point of the solvent used for the reaction, preferably, at the temperature between 20°C and 100°C to give compound (I-b).

With regard to the inert solvent, it is possible to use, for example, water, methanol, ethanol, benzene, toluene, xylene, ethyl acetate, hexane, acetonitrile, dichloromethane, dichloroethane, chloroform, carbon tetrachloride, 1,4-dioxane, tetrahydrofuran, dimethylacetamide, dimethylformamide or acetone or the like either solely or as the mixture thereof. Preferably, chloroform, dimethylformamide, etc. are used. Since the reaction usually well proceeds under a basic condition, it is desirable to add an appropriate base to a reaction system if necessary. With regard to the base, triethylamine, diisopropylethylamine, pyridine, N-methylmorpholine, potassium carbonate, sodium hydride, potassium hydride, calcium hydride, diisopropylethylamine, 1,8-diazabicyclo[5.4.0]undec-7-ene, etc. may be used and, among

them, triethylamine is preferred.

Compound (I-e) is able to be produced by the process mentioned below using compound (I-d) in compound (I-b).

Producing Process 4

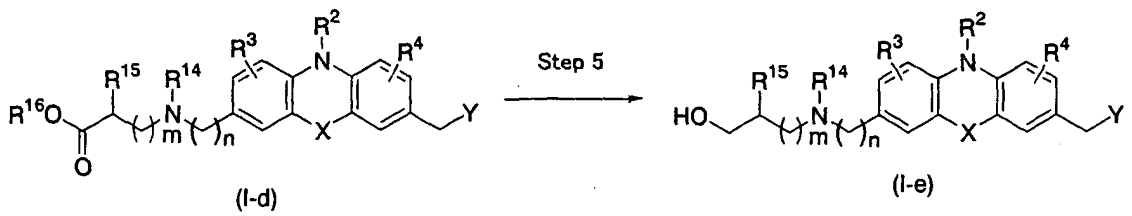


(wherein $R^2$, $R^3$, $R^4$, n, X and Y each have the same meaning as defined above, respectively; $R^{14}$ and $R^{15}$ are the same or different and each represents hydrogen, substituted or unsubstituted lower alkyl, substituted or unsubstituted cycloalkyl, substituted or unsubstituted lower alkenyl, substituted or unsubstituted lower alkynyl, substituted or unsubstituted aralkyl or substituted or unsubstituted heterocyclic alkyl, or $R^{14}$ and $R^{15}$ may form a substituted or unsubstituted heterocycle together with the adjacent $CH(CH_2)_mN$; $R^{16}$ represents hydrogen, substituted or unsubstituted lower alkyl, substituted or unsubstituted cycloalkyl, substituted or unsubstituted lower alkenyl, substituted or unsubstituted lower alkynyl, substituted or unsubstituted aralkyl or substituted or unsubstituted aryl; and m represents an integer of 0 to 3)

The lower alkyl, the cycloalkyl, the lower alkenyl, the lower alkynyl, the aralkyl, the heterocyclic alkyl and the aryl each have the same meaning as defined above, respectively and the substituents thereof also have the same meanings as defined above, respectively.

With regard to the substituted or unsubstituted heterocycle formed from $R^{14}$ and $R^{15}$ together with the adjacent $CH(CH_2)_mN$ thereto, tetrahydropyridine, pyrrolidine, piperidine, homopiperidine, piperazine, homopiperazine, morpholine, thiomorpholine, perhydroazepine, perhydroazocine, tetrahydroquinoline and tetrahydroisoquinoline or the like are exemplified and the substituent thereof has the same meaning as the substituent for the above-mentioned heterocyclic group formed together with the adjacent nitrogen atom.

<Step 5>

When compound (I-d) is treated for 10 minutes to 24 hours, preferably, 1 to 3 hour(s) in the presence of 2 to 4 equivalents of a reducing agent such as lithium aluminum hydride, diisopropyl lithium aluminum hydride, etc., preferably, diisopropyl lithium aluminum hydride usually at the temperature from -78°C to 40°C in an inert solvent, compound (I-e) is able to be prepared.

With regard to the inert solvent, dichloromethane, chloroform, carbon tetrachloride, dichloroethane, benzene, toluene, xylene, tetrahydrofuran, diethyl ether, etc. are used either solely or as the mixture thereof and, preferably, dichloromethane or toluene is used.

Compound (I-f) is able to be produced from compound (I-d) by the process as mentioned below.

## Producing Process 5

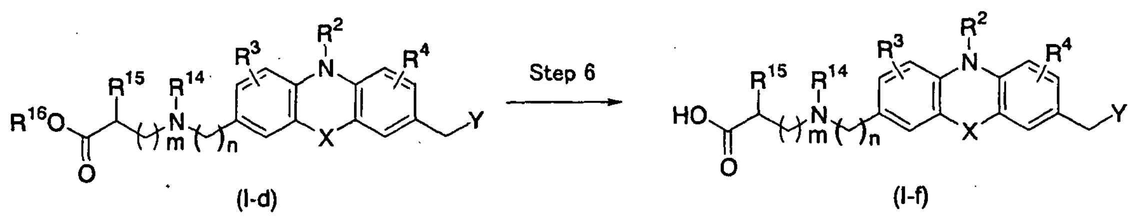


(wherein $R^2$, $R^3$, $R^4$, $R^{14}$, $R^{15}$, $R^{16}$, n, X, Y and m each have the same meaning as defined above, respectively.)

<Step 6>

When compound (I-d) is treated for 1 to 48 hours, preferably, 1 to 3 hour(s) in the presence of from one equivalent to a large excess of appropriate base at the temperature of usually from 0°C to the boiling point of the solvent used for the reaction, preferably, at the temperature between room temperature and 100°C in an inert solvent, compound (I-f) is able to be produced.

With regard to the appropriate base, sodium hydroxide, lithium hydroxide, potassium hydroxide, potassium carbonate, cesium carbonate and sodium methoxide are exemplified and sodium hydroxide is preferably exemplified. With regard to the inert solvent, water, tetrahydrofuran, diethyl ether, methanol, ethanol, propanol, dichloromethane, dichloroethane, benzene, toluene, xylene, etc. may be used either solely or as the mixture thereof and, preferably, tetrahydrofuran or methanol or a mixed solvent thereof with water is used.

Compound (I-h) is able to be produced by the following process from compound (I-g) in compound (I-c).

## Producing Process 6

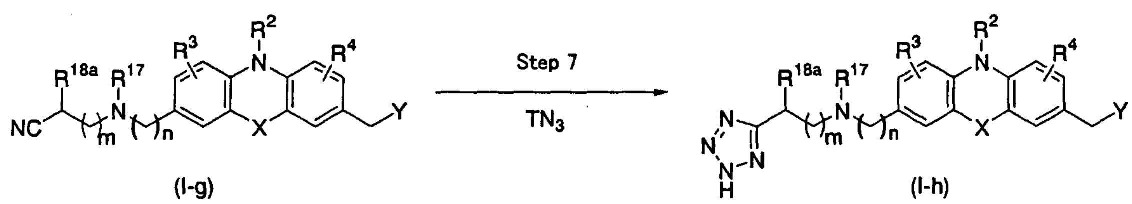


(wherein $R^2$, $R^3$, $R^4$, n, X, Y and m each have the same meaning as defined above, respectively; $R^{17}$ and $R^{18a}$ each have the same meaning as the above $R^{14}$ and the above $R^{15}$, respectively; and T represents alkaline metal, ammonium, trialkylsilyl or trialkyltin.)

The alkyl in the trialkylsilyl and trialkyltin in the above definition has the same meaning as the above-mentioned lower alkyl. Examples of the alkaline metal are sodium, potassium or the like.

<Step 7>

When compound (I-g) is made to react with from one equivalent to a large excess, preferably, 2 to 4 equivalents of $TN_3$ (wherein T has the same meaning as defined above) for 1 to 200 hours, preferably, for 3 to 48 hours usually in the presence of from catalytic amount to a large excess, preferably, 0.5 to 2 equivalent (s) of appropriate additive for accelerating the reaction at the temperature of from 0°C to the boiling point of the solvent used for the reaction, preferably, at the temperature between room temperature and 200°C in an inert solvent, compound (I-h) is able to be produced.

Examples of the appropriate additive are silicon tetrachloride, lithium chloride, aluminum chloride, ammonium

chloride, trialkyltin chloride, dialkyltin oxide, trialkyl aluminum, triethylamine hydrochloride, triethylamine hydrobromide, sodium hydroxide, potassium tert-butoxide, sodium hydroxide, and zinc bromide and preferred examples are ammonium chloride and dialkyltin oxide. With regard to the inert solvent, water, acetonitrile, dimethylformamide, dimethylacetamide, N-methyl-2-pyrrolidone, dimethyl sulfoxide, acetic acid, glacial acetic acid, tetrahydrofuran, benzene, toluene, xylene, etc. may be used either solely or as the mixture thereof. Preferably, dimethylformamide or toluene is used.

Compound (I-i) is able to be produced by the following process from compound (I-c).

Producing Process 7

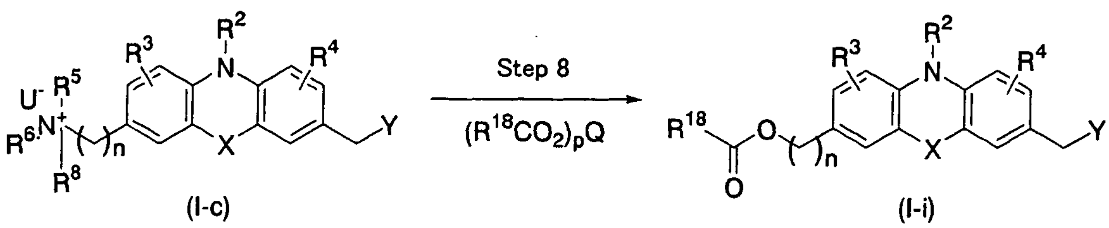


(wherein $R^2$, $R^3$, $R^4$, $R^5$, $R^6$, $R^8$, U, n, X and Y each have the same meaning as defined above, respectively; $R^{18}$ represents substituted or unsubstituted lower alkyl; Q represents alkaline metal or alkaline earth metal and, when Q represents alkaline metal, p represents 1, while, when Q represents alkaline earth metal, p represents 2)

In the above definition, the alkaline metal has the same meaning as the above-mentioned alkaline metal and examples of the alkaline earth metal are magnesium, calcium or the like.

<Step 8>

When compound (I-c) is made to react with from 1 equivalent to a large excess, preferably, 4 to 8 equivalents of $(R^{18}CO_2)_pQ$ (wherein $R^{18}$, Q and p each have the same meaning as defined above, respectively) for 1 to 100 hour (s), preferably, 3 to 72 hours at the temperature between 0°C and the boiling point of the solvent used for the reaction, preferably, the temperature between 70°C and 80°C in an inert solvent, compound (I-i) is able to be produced.

With regard to the inert solvent, dimethylacetamide, N-methyl-2-pyrrolidone, dimethyl sulfoxide, etc. may be used either solely or as the mixture thereof and, preferably, dimethyl sulfoxide is used.

Compound (I-j) is able to be produced from compound (I-c) by the process as shown below.

Production Process 8

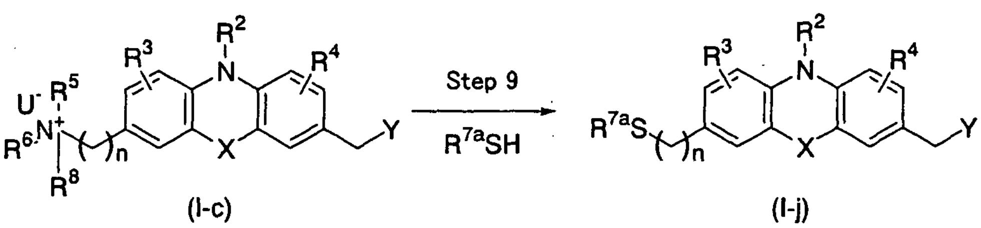


(wherein $R^2$, $R^3$, $R^4$, $R^5$, $R^6$, $R^{7a}$, $R^8$, U, n, X and Y each have the same meaning as defined above, respectively.)

<Step 9>

When compound (I-c) is made to react with from 1 equivalent to a large excess, preferably, 2 to 8 equivalents of $R^{7a}SH$ (wherein $R^{7a}$ has the same meaning as defined above) for 1 to 100 hour(s), preferably, 3 to 72 hours in the presence of 1 equivalent to a great excess, preferably, 1 to 3 equivalent (s) of an appropriate base at the temperature between 0°C to the boiling point of a solvent used for the reaction, preferably, between 30°C and 80°C in an inert solvent, compound (I-j) is able to be produced.

With regard to the appropriate base, triethylamine, diisopropylethylamine, pyridine, N-methylmorpholine, potassium carbonate, sodium hydride, potassium hydride, calcium hydride, diisopropylethylamine, 1,8-diazabicyclo[5.4.0]undec-7-ene, etc. may be used and, among them, 1,8-diazabicyclo[5.4.0]undec-7-ene is preferred. With regard to the inert solvent, dichloromethane, chloroform, carbon tetrachloride, dichloroethane, benzene, toluene, xylene, ethyl acetate, dimethylacetamide, N-methyl-2-pyrrolidone, dimethyl sulfoxide, etc. may be used either solely or as the mixture thereof and, preferably, chloroform may be used.

Compound (I-l) is able to be produced by the process as mentioned below from compound (I-k) in compound (I-j).

**Production Process 9**

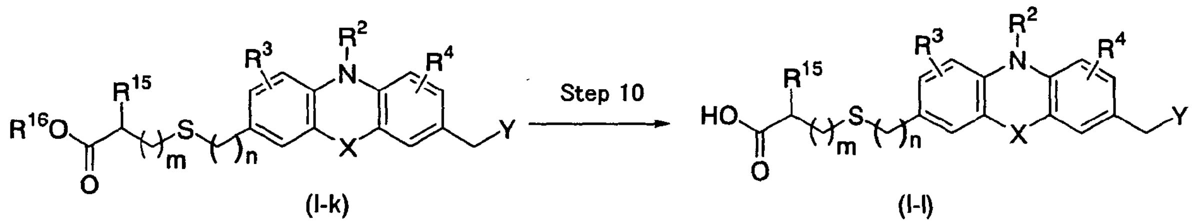


(I-k) (I-l)

(wherein $R^2$, $R^3$, $R^4$, $R^{15}$, $R^{16}$, m, n, X and Y each have the same meaning as defined above, respectively.)

<Step 10>

Compound (I-1) is able to be produced from compound (I-k) by conducting the reaction similar to the step 6 for Production Process 5.

Compound (I-m) is able to be produced from compound (I-i) by the process as mentioned below.

**Production Process 10**

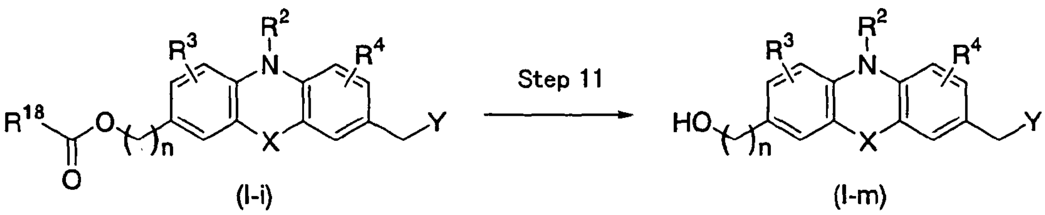


(I-i) (I-m)

(wherein $R^2$, $R^3$, $R^4$, $R^{18}$, n, X and Y each have the same meaning as defined above, respectively.)

<Step 11>

Compound (I-m) is able to be produced from compound (I-i) by conducting the reaction similar to the step 6

for Production Process 5.

Compound (I-n) is able to be produced from compound (I-m) by the process as mentioned below.

## Production Process 11

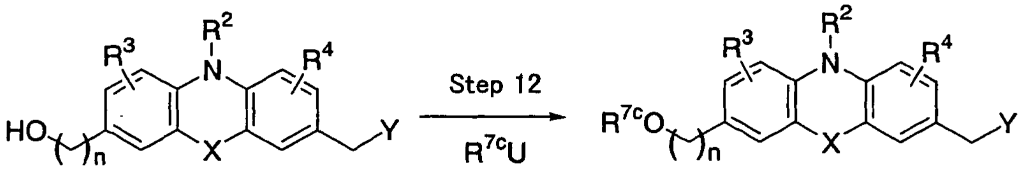


(I-m) (I-n)

(wherein $R^2$, $R^3$, $R^4$, U, n, X and Y each have the same meaning as defined above, respectively, and $R^{7c}$ represents a group wherein hydrogen is removed from the definition of the above $R^7$.)

<Step 12>

When compound (I-m) is made to react with from one equivalent to a large excess, preferably, from 1 to 3 equivalent(s) of $R^{7c}U$ (wherein $R^{7c}$ and U each have the same meaning as defined above, respectively) for 1 to 48 hour(s), preferably, 3 to 24 hour(s) at the temperature between 0°C and the boiling point of the solvent used for the reaction, preferably, at the temperature between room temperature and 80°C in the presence of from 1 equivalent to a large excess, preferably, 1 to 3 equivalent(s) of an appropriate base in an insert solvent, compound (I-n) is able to be prepared.

Examples of the appropriate base are potassium carbonate, sodium hydride, potassium hydride, calcium hydride and lower alkyllithium and preferred ones are sodium hydride, potassium hydride, etc. With regard to the inert solvent, dichloromethane, chloroform, carbon tetrachloride, dichloroethane, benzene, toluene, xylene, ethyl acetate, dimethylformamide, dimethylacetamide, N-methyl-2-pyrrolidone, tetrahydrofuran, diethyl ether, etc. may be used either either solely or as the mixture thereof. Preferably, chloroform is used.

Compound (I-o) is able to be produced by the process as mentioned below from compound (I-ma) which is compound (I-m) wherein n is 1.

## Producing Process 12

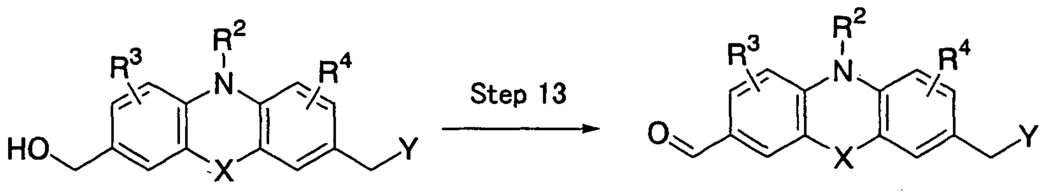


(I-ma) (I-o)

(wherein $R^2$, $R^3$, $R^4$, X and Y each have the same meaning as defined above, respectively.)

<Step 13>

When compound (I-ma) is treated for 1 to 48 hour(s), preferably, 3 to 24 hours in the presence of from 1

equivalent to a large excess, preferably, 3 to 6 equivalents of an appropriate oxidizing agent at the temperature between 0°C and the boiling point of the solvent used for the reaction, preferably, the temperature between room temperature and 60°C in an inert solvent, compound (I-o) is able to be prepared.

With regard to the appropriate oxidizing agent, manganese dioxide, chromic acid, pyridinium chlorochromate, pyridinium dichromate, potassium permanganate, sulfur trioxide-pyridine and oxone are exemplified and manganese dioxide is preferably exemplified. With regard to the inert solvent, dichloromethane, chloroform, carbon tetrachloride, dichloroethane, benzene, toluene, xylene, ethyl acetate, acetic acid, propionic acid, butyric acid, trifluoroacetic acid, water, pyridine, dimethylformamide, dimethylacetamide, N-methyl-2-pyrrolidone, 1,4-dioxane, tetrahydrofuran, diethyl ether, etc. may be used either solely or as the mixture thereof. Preferably, dimethylformamide, tetrahydrofuran, etc. may be used.

Compound (I-p) is able to be produced by the process as mentioned below from compound (I-o).

Producing Process 13

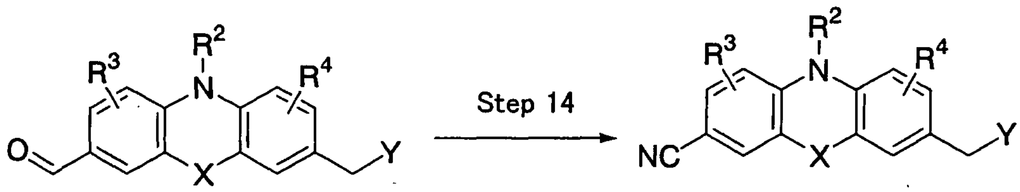


(I-o) (I-p)

(wherein $R^2$, $R^3$, $R^4$, X and Y each have the same meaning as defined above, respectively.)

<Step 14>

When compound (I-o) is made to react with from 1 equivalent to a large excess, preferably, 1 to 3 equivalent (s) of hydroxylamine or its hydrochloride, sulfate, p-toluenesulfonate, etc. thereof, O-phenylcarbamylhydroxylamine or its hydrochloride, sulfate, p-toluenesulfonate, etc. thereof or N-hydroxybenzamide, preferably, with hydroxylamine for 1 to 48 hour(s), preferably, 3 to 24 hours at the temperature between 0°C and the boiling point of the solvent used for the reaction, preferably, between room temperature and 90°C in an inert solvent, compound (I-p) is able to be prepared. If necessary, addition of 1 equivalent to a large excess, preferably, 1 to 3 equivalent(s) of an appropriate dehydrating agent, addition of 1 equivalent to a large excess, preferably, 2 to 6 equivalents of an appropriate base or irradiation with microwave may be carried out.

With regard to the appropriate dehydrating agent, acetic anhydride, phthalic anhydride, sodium hydrogen sulfate, oxone, sodium formate, dialkyltin oxide, alumina, silica gel, sodium acetate, formamide, diphosphorus pentaoxide, ferric chloride, formic acid, acetic acid, propionic acid, phosphorus oxychloride, p-toluenesulfonic acid, etc. may be exemplified and, preferably, acetic anhydride, phthalic anhydride, etc. may be exemplified. With regard to the appropriate base, triethylamine, pyridine, sodium hydride, potassium hydride, etc. may be exemplified and, preferably, triethylamine or pyridine may be exemplified.

With regard to the inert solvent, dichloromethane, chloroform, carbon tetrachloride, dichloroethane, benzene, toluene, xylene, nitrobenzene, acetonitrile, ethyl acetate, acetic acid, propionic acid, butyric acid, trifluoroacetic acid, pyridine, dimethylformamide, dimethylacetamide, N-methyl-2-pyrrolidone, 1,4-dioxane, tetrahydrofuran, diethyl ether, methanol, ethanol, propanol, etc. may be used either solely or as the mixture thereof. Preferably, acetonitrile, dimethylformamide, etc. may be used.

Compound (I-q) is able to be produced by the process as mentioned below from compound (I-p).

## Producing Process 14

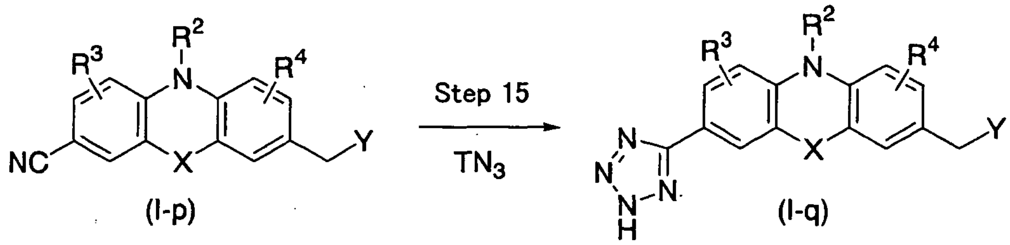


(I-p) (I-q)

(wherein $R^2$, $R^3$, $R^4$, T, X and Y each have the same meaning as define above, respectively.)

<Step 15>

Compound (I-q) is able to be produced using compound (I-p) by conducting the reaction similar to that in the step 7 of Producing Process 6.

Compound (I-r) is able to be produced by the process as mentioned below from compound (I-c)

## Producing Process 15

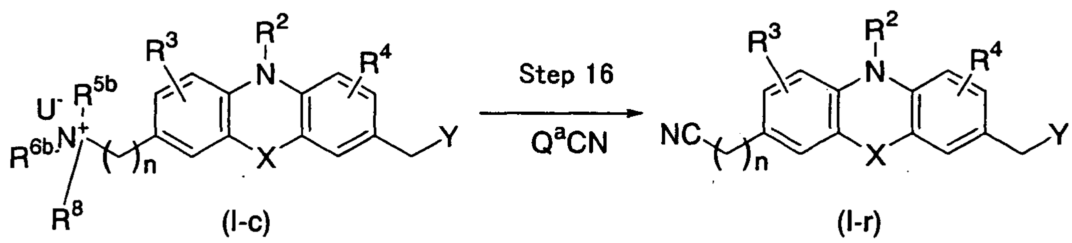


(I-c) (I-r)

(wherein $R^2$, $R^3$, $R^4$, $R^{5b}$, $R^{6b}$, $R^8$, U, n, X and Y each have the same meaning as defined above, respectively; and $Q^a$ represents the same alkaline metal as defined above.)

<Step 16>

When compound (I-c) is made to react with from 1 equivalent to a large excess, preferably, 2 to 4 equivalents of $Q^aCN$ (wherein $Q^a$ has the same meaning as defined above), preferably sodium cyanide for 1 to 48 hour(s), preferably, 3 to 24 hours at the temperature between room temperature and the boiling point of the solvent used for the reaction, preferably, between 40°C and 80°C in an inert solvent, compound (I-r) is able to be produced.

With regard to the inert solvent, dichloromethane, chloroform, carbon tetrachloride, dichloroethane, benzene, toluene, xylene, dimethylformamide, dimethylacetamide, N-methyl-2-pyrrolidone, 1,4-dioxane, tetrahydrofuran, etc. may be used either solely or as the mixture thereof. Preferably, dimethylformamide, etc. may be used.

Compound (I-s) is able to be produced by the process as shown below from compound (I-r).

Producing Process 16

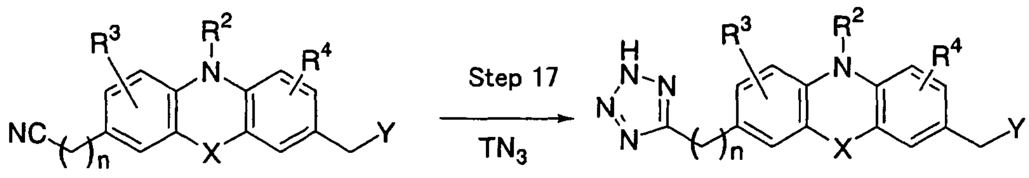


(I-r) (I-s)

(wherein $R^2$, $R^3$, $R^4$, T, n, X and Y each have the same meaning as defined above, respectively.)

<Step 17>

Compound (I-s) is able to be produced by conducting the reaction similar to the step 7 of Producing Process 6 using compound (I-r).

Compound (I-t) is able to be produced by the process as mentioned below from compound (I-r).

Producing Process 17

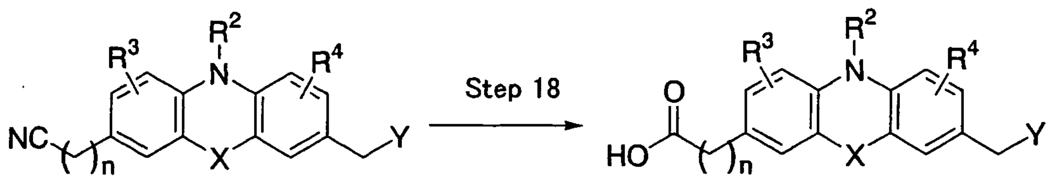


(I-r) (I-t)

(wherein $R^2$, $R^3$, $R^4$, n, X and Y each have the same meaning as defined above, respectively.)

<Step 18>

Compound (I-t) is able to be produced by conducting the reaction similar to the step 6 of Producing Process 5 using compound (I-r).

Compound (I-u) is able to be produced by the process as mentioned below from compound (IIIb).

Producing Process 18

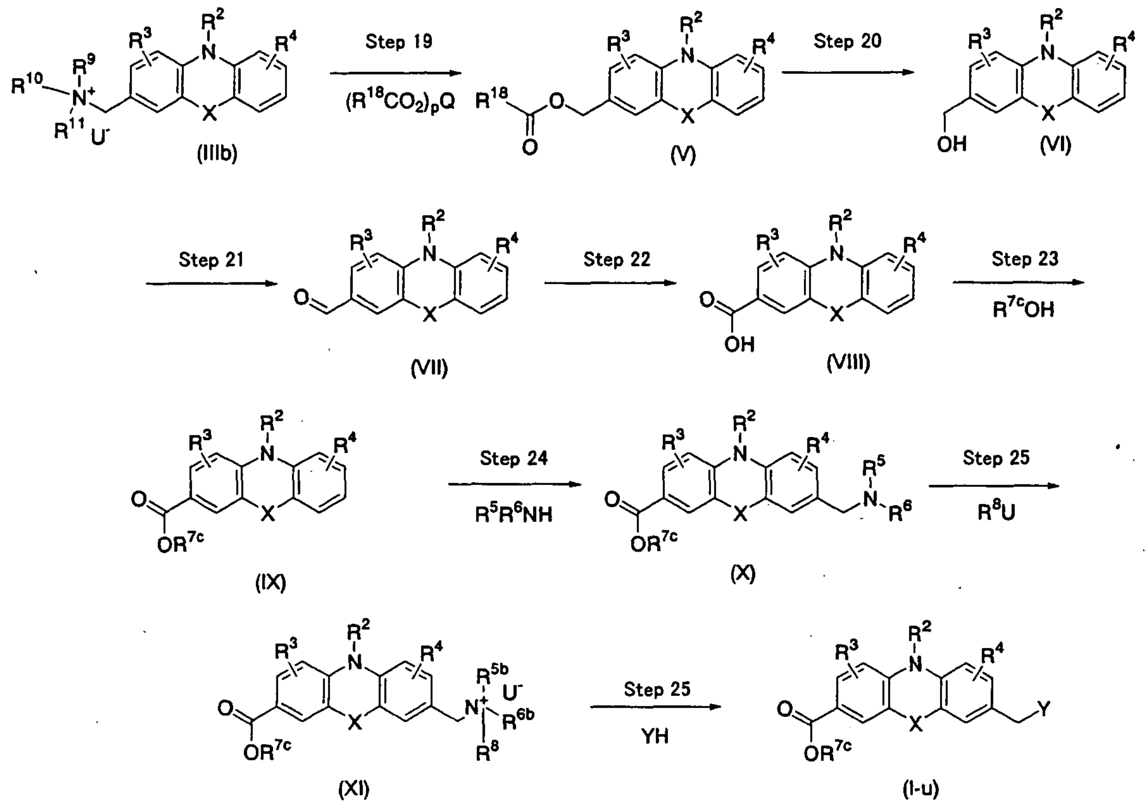


(wherein $R^2$, $R^3$, $R^4$, $R^5$, $R^6$, $R^{5b}$, $R^{6b}$, $R^{7c}$, $R^8$, $R^9$, $R^{10}$, $R^{11}$, $R^{18}$, Q, p, U, X and Y each have the same meaning as defined above, respectively.)

<Step 19>

Compound (V) is able to be produced by conducting the reaction similar to the step 8 of Producing Process 7 using compound (IIIb).

<Step 20>

Compound (VI) is able to be produced by conducting the reaction similar to the step 6 of Producing Process 5 using compound (V).

<Step 21>

Compound (VII) is able to be produced by conducting the reaction similar to the step 13 of Producing Process 12 using compound (VI).

<Step 22>

When compound (VII) is treated for 10 minutes to 24 hours, preferably, 1 to 4 hour(s) in the presence of 2 to 4 equivalent(s) of an oxidizing agent such as silver nitrate, silver (I) oxide, silver (II) oxide, chromic acid, pyridinium chlorochromate, pyridinium dichlorochromate, potassium permanganate, sodium periodate, sodium perchlorate, hydrogen peroxide and sodium hypochlorite, preferably, silver nitrate or sodium perchlorate usually at the temperature between 0°C and 80°C in an inert solvent, compound (VIII) is able to be produced. If necessary, 0.1 to 4 equivalent(s) of an organic substance such as acetic acid or an inorganic substance such as sulfuric acid, sodium dihydrogen phosphate, sulfamic acid and ruthenium oxide may be added as an additive.

With regard to the inert solvent, diethyl ether, tetrahydrofuran, 1,4-dioxane, dimethylformamide, dimethylacetamide, dimethyl sulfoxide, benzene, toluene, xylene, dichloromethane, chloroform, 1,2-dichloroethane, acetonitrile,

ethyl acetate, methyl acetate, methyl ethyl ketone, hydrochloric acid, acetic acid, acetic anhydride, sulfuric acid, water, etc. may be exemplified and, preferably, acetonitrile, water, etc. are exemplified. Each of them may be used solely or as the mixture thereof.

<Step 23>

When compound (VIII) is made to react with 1 to 20 equivalent (s) of halogenating agent for 10 minutes to 24 hours usually at the temperature between 0°C and 80°C, preferably, at room temperature in an inert solvent and, after that, it is made to react with from 1 equivalent to a large excess of $R^{7c}OH$ (wherein $R^{7c}$ has the same meaning as defined above), compound (IX) is able to be produced.

With regard to the halogenating agent, thionyl chloride, oxalyl chloride and phosphorus oxychloride are exemplified and, preferably, thionyl chloride is exemplified. With regard to the inert solvent, dichloromethane, chloroform, tetrahydrofuran, dimethylformamide, dimethylacetamide, 1,4-dioxane, acetonitrile, benzene, toluene and xylene are exemplified and each of them may be used either solely or as the mixture thereof. With regard to the inert solvent, dichloromethane is preferably exemplified.

<Step 24>

Compound (X) is able to be produced by conducting the reaction similar to the step 2 of Producing Process 1 using compound (IX).

<Step 25>

Compound (XI) is able to be produced by conducting the reaction similar to the step 3 of Producing Process 2 using compound (X).

<Step 26>

Compound (I-u) is able to be produced by conducting the reaction similar to the step 1 of Producing Process 1 using compound (XI).

Compound (I-v) is able to be produced by the process as shown below from compound (I-u).

Producing Process 19

(step 27)

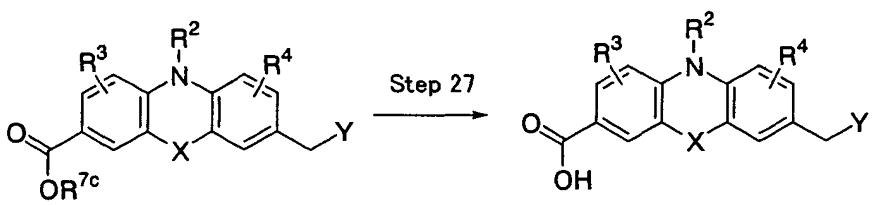


(I-u) (I-v)

(wherein $R^2$, $R^3$, $R^4$, $R^{7c}$, X and Y each have the same meaning as defined above, respectively.)

<Step 27>

Compound (I-v) is able to be produced by conducting the reaction similar to the step 6 of Producing Process 5 using compound (I-u).

Compound (I-w) is able to be produced by the process as shown below from compound (I-v).

Producing Process 20

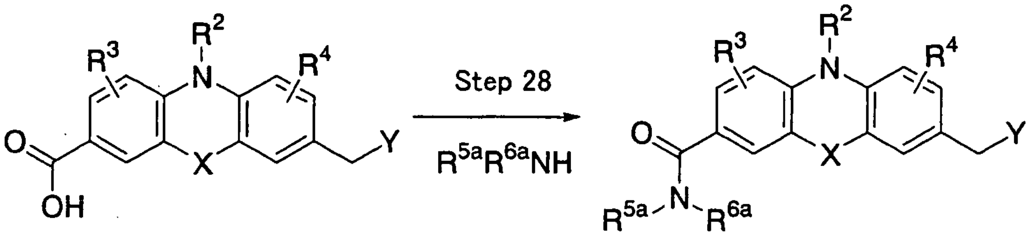


(I-v) (I-w)

(wherein $R^2$, $R^3$, $R^4$, $R^{5a}$, $R^{6a}$, X and Y each have the same meaning as defined above, respectively.)

<Step 28>

When compound (I-v) is made to react with 1 to 20 equivalent (s) of a halogenating agent for 10 minutes to 24 hours usually at the temperature between 0°C and 80°C, preferably, at room temperature in an inert solvent and, after that, made to react with 1 equivalent to a large excess of $R^{5a}R^{6a}NH$ (wherein $R^{5a}$ and $R^{6a}$ each have the same meaning as defined above, respectively), compound (I-w) is able to be produced. If necessary, 1 equivalent to a large excess of an appropriate base may be added thereto.

With regard to the halogenating agent, thionyl chloride, oxalyl chloride, phosphorus oxychloride, etc. may be exemplified and, preferably, thionyl chloride is exemplified. With regard to the appropriate base, pyridine, triethylamine, diisopropylethylamine, N-methylmorpholine, etc. may be exemplified and, preferably, triethylamine may be exemplified. With regard to the inert solvent, dichloromethane, chloroform, tetrahydrofuran, dimethylformamide, dimethylacetamide, 1,4-dioxane, acetonitrile, benzene, toluene, xylene, etc. may be exemplified and each of them may be used solely or as the mixture thereof. With regard to the inert solvent, dichloromethane is preferably exemplified.

In the production of compound (I-w), it is also possible to apply the method which has been commonly used in peptide chemistry. Thus, when compound (I-v) is made to react with 1 to 10 equivalent(s) of $R^{5a}R^{6a}NH$ (wherein $R^{5a}$ and $R^{6a}$ each have the same meaning as defined above, respectively) together with 0.5 to 10 equivalent(s) of an appropriate condensing agent usually at the temperature between 0°C and 50°C for 10 minutes to 70 hours in an inert solvent, compound (I-w) is able to be produced.

With regard to the inert solvent, diethyl ether, tetrahydrofuran, 1,4-dioxane, dimethylformamide, dimethylacetamide, dimethyl sulfoxide, benzene, toluene, xylene, acetonitrile, ethyl acetate, pyridine, dichloromethane, chloroform and carbon tetrachloride may be exemplified and, preferably, tetrahydrofuran and dimethylformamide may be exemplified.

With regard to the appropriate condensing agent, 1,3-dicyclohexylocarbodiimide, 1-ethyl-3-(3-dimethylaminopropyl)carbodiimide hydrochloride, 1-ethyl-3-(3-dimethylaminopropyl)carbodiimide-bonded polystyrene resin (EDC resin), etc. may be exemplified. It is also possible to add an additive such as N-hydroxysuccinimide, 3,4-dihydro-3-hydroxy-4-oxo-1,2,3-benzotriazine, 1-hydroxybenzotriazole, preferably, 1-hydroxybenzotriazole thereto.

EDC resin is able to be produced by a process mentioned in *Tetrahedron Letters,* volume 34, no. 48, page 7685 (1993).

Compound (I-y) is able to be produced by the process shown below from compound (I-x) in compound (I).

Producing Process 21

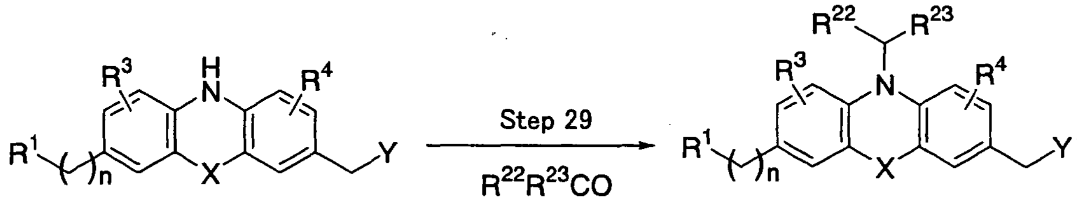


(I-x) (I-y)

(wherein $R^1$, $R^3$, $R^4$, n, X and Y each have the same meaning as defined above, respectively; and $R^{22}$ and $R^{23}$ are the same or different and each represents hydrogen, substituted or unsubstituted lower alkyl, substituted or unsubstituted cycloalkyl, substituted or unsubstituted lower alkenyl, substituted or unsubstituted lower alkynyl, substituted or unsubstituted aralkyl or substituted or unsubstituted heterocyclic alkyl.)

In the above definitions, the lower alkyl, the cycloalkyl, the lower alkenyl, the lower alkynyl, the aralkyl and the heterocyclic alkyl each have the same meaning as defined above, respectively and the substituent thereof also has the same meaning as defined above, respectively.

<Step 29>

When compound (I-x) is made to react with 1 equivalent to a large excess, preferably, 1 to 10 equivalent (s) of $R^{22}R^{23}CO$ (wherein $R^{22}$ and $R^{23}$ each have the same meaning as defined above, respectively) usually at the temperature between -78°C and 100°C, preferably, the temperature between 0°C and 50°C for 10 minutes to 48 hours in the presence of 1 equivalent to large excess, preferably, 1 to 3 equivalent(s) of an appropriate reducing agent in an inert solvent, compound (I-y) is able to be produced.

With regard to the appropriate reducing agent, sodium borohydride, sodium triacetoxyborohydride, sodium cyanoborohydride, etc. may be exemplified and, preferably, sodium cyanoborohydride may be exemplified. If necessary, catalytic amount to solvent amount, preferably, 0.5 equivalent to solvent amount of appropriate acid may be added thereto. With regard to the appropriate acid, formic acid, acetic acid, trifluoroacetic acid, propionic acid, hydrochloric acid, etc. may be exemplified and, preferably, acetic acid may be exemplified.

With regard to the inert solvent, dichloromethane, chloroform, carbon tetrachloride, dichloroethane, benzene, toluene, xylene, diethyl ether, 1,4-dioxane, dimethylformamide, dimethylacetamide, acetonitrile, hexane, formic acid, acetic acid, trifluoroacetic acid, propionic acid, hydrochloric acid, etc. may be exemplified and each of them may be used solely or as the mixture thereof. Preferably, tetrahydrofuran, acetic acid, etc. may be exemplified.

Transformation of each functional group in compound (I) and the starting compounds and transformation of a functional group contained in the substituent may also be carried out a known method [such as a method mentioned in Comprehensive Organic Transformation, second edition, by R. C. Larock, John Wiley & Sons, Inc. (1999)], etc.

When the above-mentioned method, etc. are carried out in an appropriately combined manner, it is possible to prepare compound (I) having a desired functional group at a desired position.

Isolation and purification of an intermediate and the product in the above producing process may be carried out by an appropriate combination of the methods which are used in common organic synthesis such as filtration, extraction, washing, drying, concentration, crystallization, various kinds of chromatographies, etc. It is also possible to conduct a purifying method commonly used in general parallel synthetic methods such as purifying methods using scavenger resin and ion-exchange resin. It is also possible that the intermediate is not particularly purified but just subjected to the next reaction.

In some of compound (I), there may be isomers such as regioisomer, geometric isomer, optical isomer or tautomer and all possible isomers including the above and mixtures of any ratio of said isomers are within the scope of the present invention.

When a salt of compound (I) is to be prepared, the product may be just purified in case a salt of compound (I) is obtained while, in case compound (I) is obtained in a free form, compound (I) may be dissolved or suspended in an appropriate solvent and isolated and purified after addition of acid or base.

Some of compound (I) or a pharmaceutically acceptable salt thereof may be present in a form of an adduct with water or with various solvents and such adducts are also within the scope of the present invention.

In a screening method for the therapeutic agent for itching according to the present invention, a compound having an activity for treating itching is able to be obtained as follows.

1) SPC is subcutaneously and intracutaneously administered to the mammal except a human being and a scratching behavior in the mammal except a human being is induced,
2) numbers of scratching behavior in the mammal except a human being induced by SPC in the presence or absence of the test compound are measured,
3) numbers of scratching behavior in the mammal except a human being induced by SPC in the presence of the test compound and in the absence of the test compound are compared, and
4) a substance which decreases the numbers of scratching behavior induced by SPC is selected from the test compounds.

With regard to the above-mentioned mammal except a human being, mouse or the like may be exemplified. Scratching behavior means a behavior where the animal scrapes its own body by a hind paw. With regard to a more specific example of a screening method for the agent for treating itching, the method mentioned in Test Example 2 or the like which is mentioned later may be exemplified.
Although specific examples of compound (I) prepared by the present invention will be shown in the following Table 1 to Table 12, the scope of the present invention is not limited thereto. Specific examples of other compound which suppresses the function involved in signal transduction of GPR4 will be shown in Table 13.

Table 1

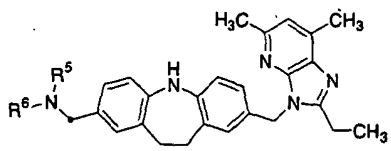

| Compound No. | $\bullet-NR^5R^6$ |
|---|---|
| 1 | 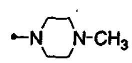 |
| 2 | 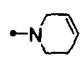 |
| 3 | 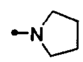 |
| 4 | 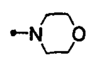 |
| 5 | 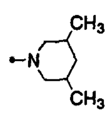 |
| 6 | 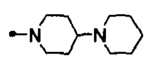 |
| 7 | 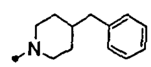 |
| 8 | 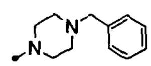 |
| 9 | 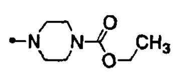 |
| 10 | 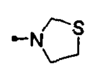 |
| 11 | 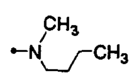 |
| 12 | 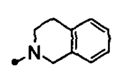 |
| 13 | 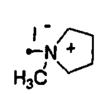 |
| 14 | 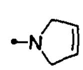 |

Table 1 (continued)

| Compound No. | •—$NR^5R^6$ |
|---|---|
| 15 | 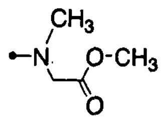 |
| 16 | 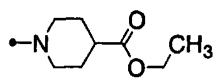 |
| 17 | 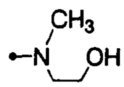 |
| 18 | 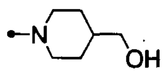 |
| 19 | 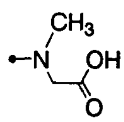 |
| 20 | 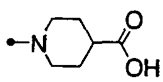 |
| 21 | 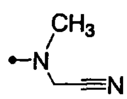 |
| 22 | 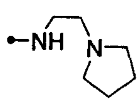 |
| 23 | 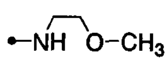 |
| 24 | 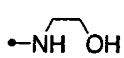 |
| 25 | •—$NH_2$ |
| 26 | 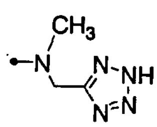 |
| 27 | 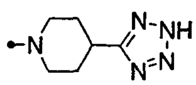 |

Table 2

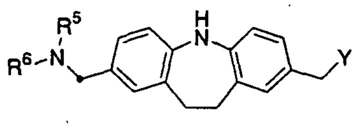

| Compound No. | •—$NR^5R^6$ | •—Y | Mass Spectrometric Data |
|---|---|---|---|
| 28 | 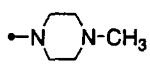 | 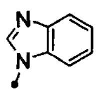 | MS m/z 438 $(M+H)^+$ |
| 29 | 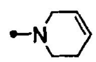 | 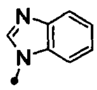 | MS m/z 421 $(M+H)^+$ |
| 30 | 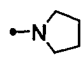 | 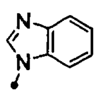 | MS m/z 409 $(M+H)^+$ |
| 31 | 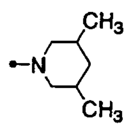 | 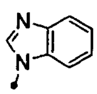 | MS m/z 451 $(M+H)^+$ |
| 32 | 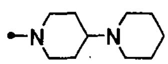 | 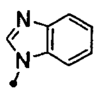 | MS m/z 506 $(M+H)^+$ |
| 33 | 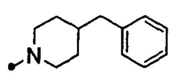 | 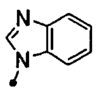 | MS m/z 513 $(M+H)^+$ |
| 34 | 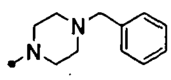 | 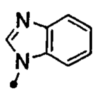 | MS m/z 514 $(M+H)^+$ |
| 35 | 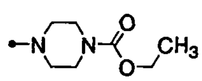 | 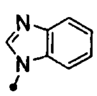 | MS m/z 496 $(M+H)^+$ |
| 36 | 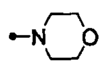 | 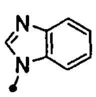 | MS m/z 425 $(M+H)^+$ |
| 37 | 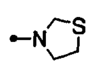 | 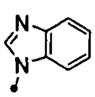 | MS m/z 427 $(M+H)^+$ |
| 38 | 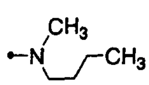 | 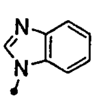 | MS m/z 425 $(M+H)^+$ |
| 39 | 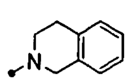 | 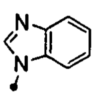 | MS m/z 471 $(M+H)^+$ |

Table 3

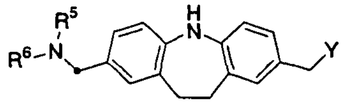

| Compound No. | •—$NR^5R^6$ | •—Y | Mass Spectrometric Data |
|---|---|---|---|
| 40 | 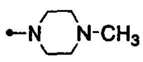 | 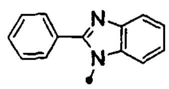 | MS m/z 514 $(M+H)^+$ |
| 41 | 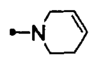 | 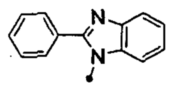 | MS m/z 497 $(M+H)^+$ |
| 42 | 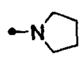 | 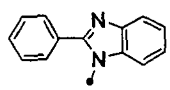 | MS m/z 485 $(M+H)^+$ |
| 43 | 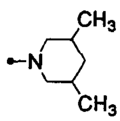 | 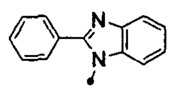 | MS m/z 527 $(M+H)^+$ |
| 44 | 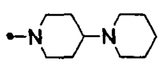 | 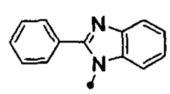 | MS m/z 582 $(M+H)^+$ |
| 45 | 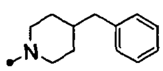 | 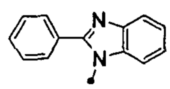 | MS m/z 589 $(M+H)^+$ |
| 46 | 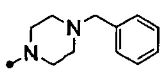 | 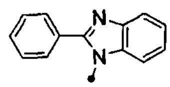 | MS m/z 590 $(M+H)^+$ |
| 47 | 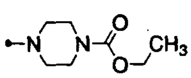 | 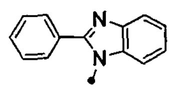 | MS m/z 572 $(M+H)^+$ |
| 48 | 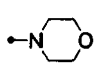 | 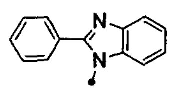 | MS m/z 501 $(M+H)^+$ |
| 49 | 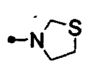 | 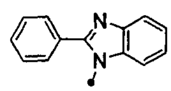 | MS m/z 503 $(M+H)^+$ |
| 50 | 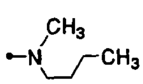 | 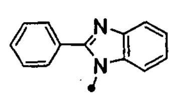 | MS m/z 501 $(M+H)^+$ |
| 51 | 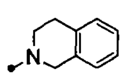 | 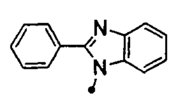 | MS m/z 547 $(M+H)^+$ |

Table 4

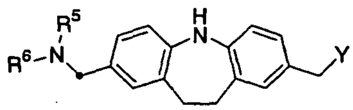

| Compound No. | •—NR⁵R⁶ | •—Y | Mass Spectrometric Data |
|---|---|---|---|
| 52 | 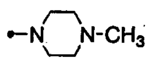 | 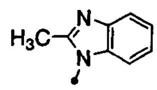 | MS m/z 452 $(M+H)^+$ |
| 53 | 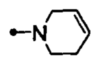 | 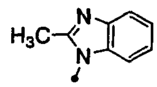 | MS m/z 435 $(M+H)^+$ |
| 54 | 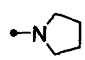 | 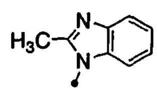 | MS m/z 423 $(M+H)^+$ |
| 55 | 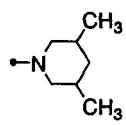 | 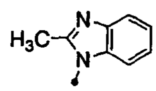 | MS m/z 465 $(M+H)^+$ |
| 56 | 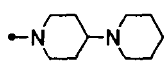 | 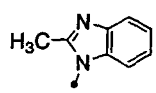 | MS m/z 520 $(M+H)^+$ |
| 57 | 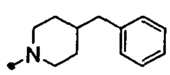 | 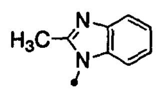 | MS m/z 527 $(M+H)^+$ |
| 58 | 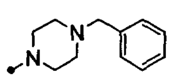 | 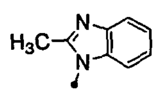 | MS m/z 528 $(M+H)^+$ |
| 59 | 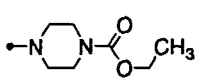 | 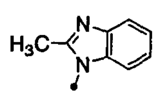 | MS m/z 510 $(M+H)^+$ |
| 60 | 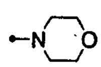 | 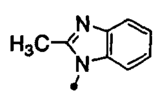 | MS m/z 439 $(M+H)^+$ |
| 61 | 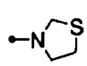 | 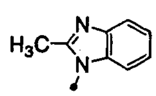 | MS m/z 441 $(M+H)^+$ |
| 62 | 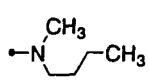 | 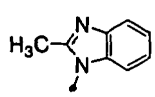 | MS m/z 439 $(M+H)^+$ |
| 63 | 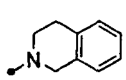 | 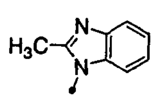 | MS m/z 485 $(M+H)^+$ |

Table 5

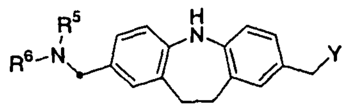

| Compound No. | •—$NR^5R^6$ | •—Y | Mass Spectrometric Data |
|---|---|---|---|
| 64 | 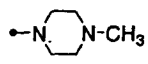 | 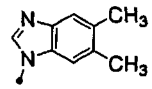 | MS m/z 466 $(M+H)^+$ |
| 65 | 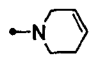 | 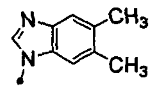 | MS m/z 449 $(M+H)^+$ |
| 66 | 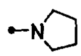 | 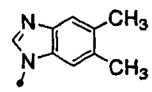 | MS m/z 437 $(M+H)^+$ |
| 67 | 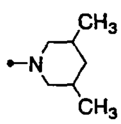 | 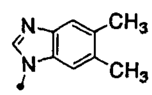 | MS m/z 479 $(M+H)^+$ |
| 68 | 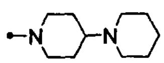 | 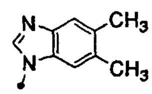 | MS m/z 534 $(M+H)^+$ |
| 69 | 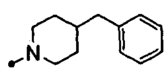 | 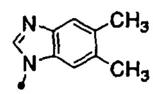 | MS m/z 541 $(M+H)^+$ |
| 70 | 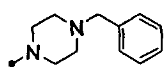 | 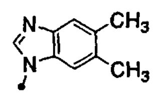 | MS m/z 542 $(M+H)^+$ |
| 71 | 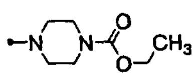 | 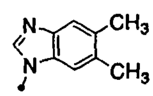 | MS m/z 524 $(M+H)^+$ |
| 72 | 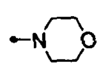 | 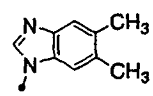 | MS m/z 453 $(M+H)^+$ |
| 73 | 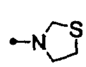 | 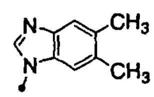 | MS m/z 455 $(M+H)^+$ |
| 74 | 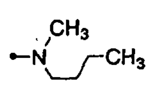 | 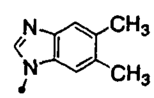 | MS m/z 453 $(M+H)^+$ |
| 75 | 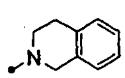 | 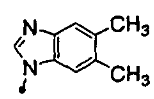 | MS m/z 499 $(M+H)^+$ |

Table 6

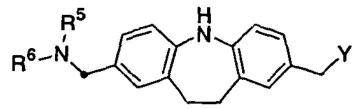

| Compound No. | •—$NR^5R^6$ | •—Y | Mass Spectrometric Data |
|---|---|---|---|
| 76 | 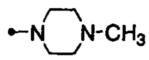 | 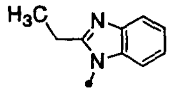 | MS m/z 466 $(M+H)^+$ |
| 77 | 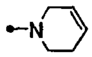 | 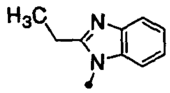 | MS m/z 449 $(M+H)^+$ |
| 78 | 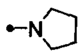 | 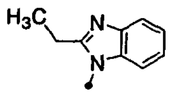 | MS m/z 437 $(M+H)^+$ |
| 79 | 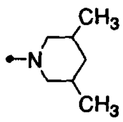 | 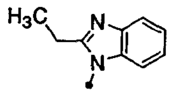 | MS m/z 479 $(M+H)^+$ |
| 80 | 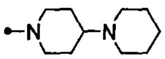 | 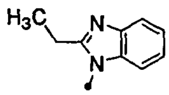 | MS m/z 534 $(M+H)^+$ |
| 81 | 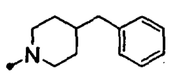 | 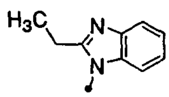 | MS m/z 541 $(M+H)^+$ |
| 82 | 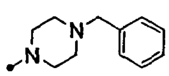 | 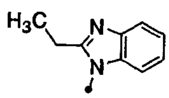 | MS m/z 542 $(M+H)^+$ |
| 83 | 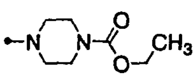 | 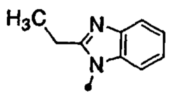 | MS m/z 524 $(M+H)^+$ |
| 84 | 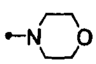 | 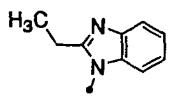 | MS m/z 453 $(M+H)^+$ |
| 85 | 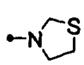 | 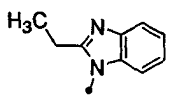 | MS m/z 455 $(M+H)^+$ |
| 86 | 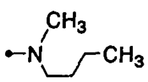 | 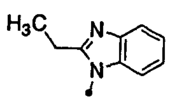 | MS m/z 453 $(M+H)^+$ |
| 87 | 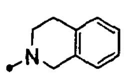 | 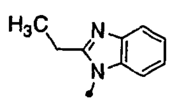 | MS m/z 499 $(M+H)^+$ |

Table 7

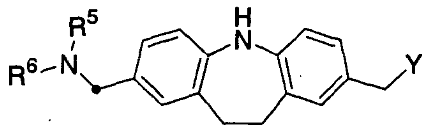

| Compound No. | •—$NR^5R^6$ | •—Y |
|---|---|---|
| 88 | 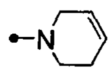 | 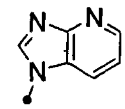 |
| 89 | 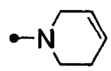 | 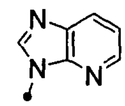 |
| 90 | 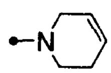 | 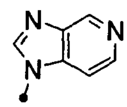 |

Table 8

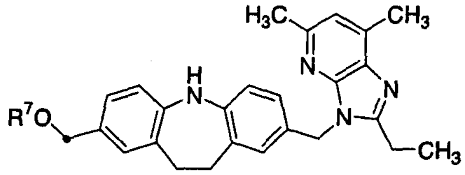

| Compound No. | •—OR$^7$ |
|---|---|
| 92 | 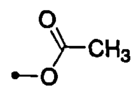 |
| 93 | •—OH |
| 94 | •—O—$CH_3$ |
| 95 | 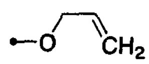 |
| 96 | 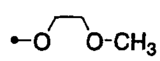 |
| 97 | 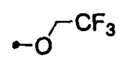 |
| 98 | 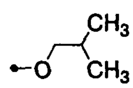 |
| 99 | 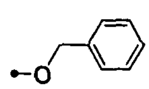 |
| 100 | 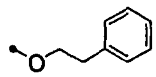 |
| 101 | 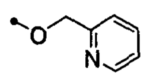 |
| 102 | 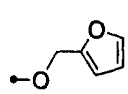 |

Table 9

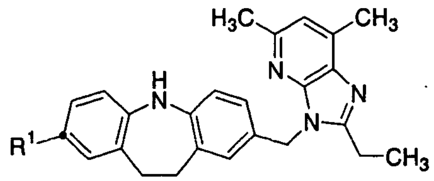

| Compound No. | •—R[1] |
|---|---|
| 103 | 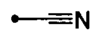 |
| 104 | 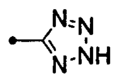 |
| 105 | 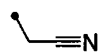 |
| 106 | 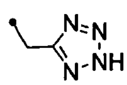 |
| 107 | 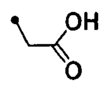 |
| 108 | 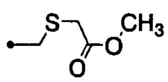 |
| 109 | 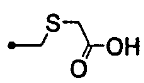 |
| 110 | 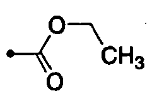 |
| 111 | 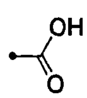 |

Table 10

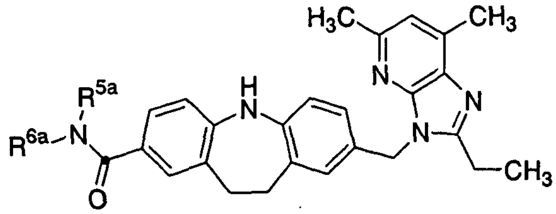

| Compound No. | •—$NR^{5a}R^{6a}$ |
|---|---|
| 112 | 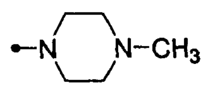 |
| 113 | 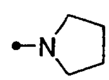 |
| 114 | 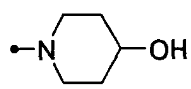 |
| 115 | 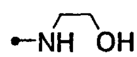 |
| 116 | 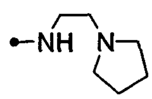 |
| 117 | 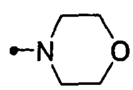 |
| 118 | 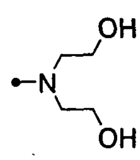 |
| 119 | •—$NH_2$ |

Table 11

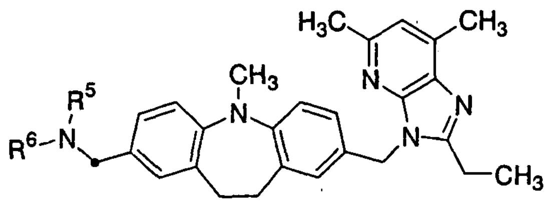

| Compound No. | •—$NR^5R^6$ |
|---|---|
| 120 | 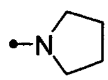 |
| 121 | 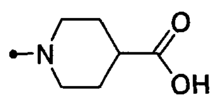 |
| 122 | 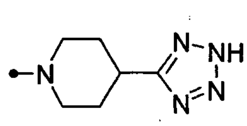 |
| 123 | 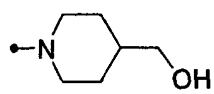 |

Table 12

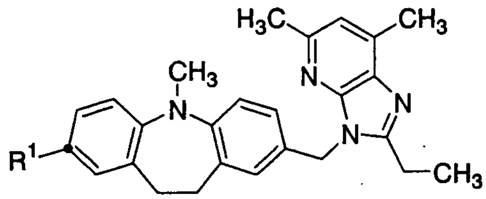

| Compound No. | •—$R^1$ |
|---|---|
| 124 | 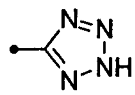 |
| 125 | 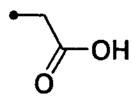 |

Table 13

| Compound No. | |
|---|---|
| 91 | 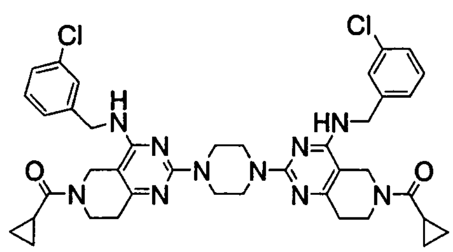 |

Table 14

| Compound No. | Analytical Data |
|---|---|
| 5 | MS m/z 508 $(M+H)^+$ |
| 6 | MS m/z 563 $(M+H)^+$ |
| 7 | MS m/z 570 $(M+H)^+$ |
| 8 | MS m/z 571 $(M+H)^+$ |
| 9 | MS m/z 553 $(M+H)^+$ |
| 10 | MS m/z 484 $(M+H)^+$ |
| 11 | MS m/z 482 $(M+H)^+$ |
| 12 | MS m/z 528 $(M+H)^+$ |

Brief Description of the Drawings

Fig. 1 shows a suppressive action of compound 1 (by oral administration) to itching induced by SPC. In Fig. 1, each of the symbols (##, **) has the following meaning, respectively.

## : the significant difference when p = 0.0083 (ratio of a positive control group to a negative control group; Aspin-Welch test).

** : the significant difference when p = 0.0031 (ratio of a group to which compound 1 was administered per os to a positive control group; Aspin-Welch test).

Fig. 2 shows a suppressive action of compound 3 (by oral administration) to itching induced by SPC. In Fig. 2, each of the symbols (##, **) has the following meaning, respectively.

## : the significant difference when p = 0.0029 (ratio of a positive control group to a negative control group; Aspin-Welch test).

** : the significant difference when p = 0.0031 (ratio of a group to which compound 3 was administered per os to a positive control group; Aspin-Welch test).

Pharmacological action of the compounds is illustrated by way of Test Examples.

Test Example 1: Antagonistic action on GPR4

Cells for GPR4 assay prepared in Referential Example 5 (said assay cells express GPR4 by stimulation with 17β-estradiol) were seeded on white plates at the rate of $10^5$ cells/well, and a solution where 17β-estradiol (manufactured by Sigma) was diluted with a medium so as to make 10 nmol/L in the reaction solution and test compounds were added to the plate and the mixture was incubated at 37°c for 6 hours in a 5% $CO_2$ incubator. After that, a Steady Glo Luciferase Assay System (manufactured by Promega) solution was added to stop the reaction and the amount of luminescence during one second was measured using a Top Count (Packard, Meriden, CT, U. S. A.).

Activity of the each of test compounds (antagonistic action) was expressed by an inhibition rate calculated on the basis of counts per second in the presence or absence of 17β-estradiol as shown by the following formula. $IC_{50}$ value was calculated from the inhibition rate by a linear approximate analysis of the Logit-Log conversion method.

In the formula, A, B and C each have the following meaning, respectively.

A: counts per second when 17β-estradiol and the test compound were added

B: counts per second when neither 17β-estradiol nor the test compound was added
C: counts per second when only 17β-estradiol was added

$$\text{Inhibition Rate (\%)} = [1 - \{(A - B)/(C - B)\}] \times 100$$

The result is shown in Table 15.

Table 15

| Compound Nos. | $IC_{50}$ (nmol/L) |
|---|---|
| 1 | 4.0 |
| 2 | 3.2 |
| 3 | 2.3 |
| 4 | 5.8 |
| 5 | 14 |

From the above results, compound (I) has been shown to have an antagonistic action to GPR4.
Test Example 2: Suppressive effect on Itching Induced by SPC
SPC (50 μg/site) was subcutaneously administered (an SPC-administered group) to the rostal back of ddY male mice (3 to 4 weeks age), the mice were placed in a cage made of acrylic for the observation (7.5 × 7.5 × 15 cm) and behavior of the mice was taken by a video camera for 60 minutes in the absence of observing persons. The video camera was subjected to a playback and numbers of scratching behavior by hind limb were counted by visual observation. In a negative control group (a group to which a physiological saline was administered), a physiological saline (0.1 mL/site) was administered instead of SPC. In the compound-administered group, each compound 1 and compound 3 (300 mg/kg) was suspended in a 0.5 w/v% aqueous solution of methyl cellulose (MC) and orally administered at one hour before administration of SPC. In each of the SPC-administered group and the physiological saline-administered group, a 0.5 w/v% aqueous solution of MC (10 mL/kg) was orally administered at one hour before administration of SPC and physiological saline, respectively. The test was conducted for ten mice per group. Suppressive rate by the compounds for scratching behavior induced by SPC was calculated by the following formula.
In the formula, A and B have the following meanings, respectively.

A: numbers of scratching behavior of the SPC-administered group
B: numbers of scratching behavior of the compound-administered group

$$\text{Suppressive Rate (\%)} = [(A - B)/A] \times 100$$

The result for compound 1 is shown in Fig. 1 and the result for compound 3 is shown in Fig. 2.
As shown in Fig. 1, numbers (78 times) of scratching behavior of the SPC-administered group significantly increased as compared with those (23 times) of scratching behavior of the negative control group (p = 0.0083). Numbers of scratching behavior of the compound 1-administered group were 13 and, in the compound 1-administered group, numbers of scratching behavior of the positive control group was suppressed by 83% (p = 0.0031).
As shown in Fig. 2, numbers (69 times) of scratching behavior of the SPC-administered group significantly increased as compared with those (18 times) of scratching behavior of the negative control group (p = 0.0029). Numbers of scratching behavior of the compound 3-administered group were 18 and, in the compound 1-administered group, numbers of scratching behavior of the positive control group was suppressed by 74% (p = 0.0031).
From the above result, it has been suggested that a substance which suppresses the function involved in signal transduction of protein having an amino acid sequence mentioned in SEQ ID NO: 11 is useful as a therapeutic agent for itching.
Test Example 3: Effect on Itching Induced by Compound 48/80 (manufactured by Sigma)
Compound 48/80 (10 μg/site) was subcutaneously administered (a Compound 48/80-administered group) to the rostal back of ddY strain mice (4 weeks age), the mice were placed in a cage made of acrylic for the observation (7.5 × 7. 5 × 15 cm) and behavior of the mice was taken by a video camera for 60 minutes in the absence of observing persons. The video camera was subjected to a playback and numbers of scratching behavior by hind limb were counted by visual observation. In a negative control group (a group to which a physiological saline was administered), a physiological saline (0.1 mL/site) was administered instead of Compound 48/80. In the compound-administered group, each compound 1 and compound 3 (300 mg/kg) was suspended in a 0.5 w/v% aqueous solution of methyl cellulose (MC) and orally administered at one hour before administration of Compound 48/80. In each of the Compound 48/80-administered group and the physiological saline-administered group, a 0.5 w/v% aqueous solution of MC was orally administered at one hour before administration of Compound 48/80 and physiological saline, respectively. The test was conducted for ten mice per group. Suppressive rate by the compound for scratching behavior induced by Compound 48/80 was calculated by the following formula.

In the formula, C and D have the following meanings.

C: numbers of scratching behavior of the Compound 48/80-administered group
D: numbers of scratching behavior of the compound-administered group

$$\text{Suppressive Rate (\%)} = [(C - D)/C] \times 100$$

The result for compound 1 is shown in Table 16 and the result for compound 3 is shown in Table 17.

Table 16

| Group | Numbers of Scratching Behavior (numbers/60 min) | Suppressive rate(%) |
|---|---|---|
| Physiological saline was Administered | 7 | |
| Compound48/80 was Administered | 35 | 54 |
| Compound 1 was Administered | 16 | |

Table 17

| Group | Numbers of Scratching behavior (numbers/60 min) | Suppressive Rate(%) |
|---|---|---|
| Physiological Saline was Administered | 13 | |
| Compound48/80 was Administered | 54 | 52 |
| Compound 3 was Administered | 26 | |

As shown in Table 16, numbers of scratching behavior of the Compound 48/80-administered group were 35 times and increased as compared with those (7 times) of scratching behavior of the negative control group. The numbers of scratching behavior of the compound 1-administered group were 16 and, in the compound 1-administered group, the numbers of scratching behavior in the Compound 48/80-administered group was suppressed by 54%.

As shown in Table 17, the numbers of scratching behavior of the Compound 48/80-administered group were 54 times and increased as compared with those (13 times) of scratching behavior of the negative control group. The numbers of scratching behavior of the compound 3-administered group were 26 and, in the compound 3-administered

group, the numbers of scratching behavior in the Compound 48/80-administered group was suppressed by 52%.

From the above results, it has been suggested that a substance which suppresses the function involved in signal transduction of protein having an amino acid sequence mentioned in SEQ ID NO: 11 is useful as a therapeutic agent for itching.

Test Example 4: Effect on Itching in Chronic Dermatitis Model to which Hapten was Repeatedly Applied

Preparation of chronic dermatitis model to which hapten was repeatedly applied was conducted according to the method by Kitagaki, et al. [*Journal of Investigative Dermatology,* volume 105, pages 749-755 (1995)] with some modifications.

With regard to hapten, oxazolone (manufactured by Sigma-Aldrich) was used and dissolved in acetone (manufactured by Kanto Kagaku) to prepare a 0.5 w/v% solution of oxazolone in acetone (antigen solution). The antigen solution (10 μL) was applied to the rostal back of BALB/c male mice (6 weeks age) with the hair was shaved to sensitize the mice. Starting from 7 days thereafter (day 0), the antigen solution (10 μL) was repeatedly challenged to apply at the same site until day 16 every two or three days to prepare chronic dermatitis models.

Compound 1 was suspended in a 0.5 w/v% aqueous solution of MC in a concentration of 10 and 30 mg/mL, respectively, and orally administered at a dose of 10 mL/kg at 1 hour before application of the antigen solution on the day 16. As a control group, a group to which only 0.5 w/v% aqueous solution of MC was similarly administered per os was prepared.

Analysis of the itching reaction in mice on the day 16 was conducted by the method of Kuraishi, et al. [*European Journal of Pharmacology,* volume 275, pages 229-233 (1995)] with some modifications.

In order to adjust the mice to new environment, they were allowed to place for one hour in a cage (7.5 × 8 × 15 cm) made of acrylic for observation. Immediately after application of the antigen solution to the rostal back of the mice (Day 16), the mice were returned to the cage and their behavior was taken by a 8-mm video camera recorder in the absence of observing person. By a playback of the video, the scratching behavior for one hour after application of the antigen solution was observed. The numbers of scraching behavior by hind limb to the applied site and around there were counted. Mice showed very quick and continuous scratching behavior for several times per second and a series of behavior as such was regarded as one scratching behavior.

The result is shown in Table 18.

Table 18

| | Control Group | Compound 1-Administered Group (mg/kg) | |
|---|---|---|---|
| | | 100 | 300 |
| Numbers of Scratching Behavior for 1 Hour after Application of Hapten | 264 ± 29 | 138 ± 33 ** | 2 ± 1 *** |

The numbers of scratching behavior of the compound 1-administered group was 138 ± 33 (mean ± standard error) and 2 ± 1 at doses of 100 mg/kg and 300 mg/kg, respectively. As such, they significantly decreased as compared with the numbers of scratching behavior (264 ± 29) of the control group (**: $p < 0.01$, ***: $p < 0.001$; Dunnett test).

From the above result, it has been suggested that a substance which suppresses the function involved in signal transduction of protein having an amino acid sequence mentioned in SEQ ID NO: 11 is useful as an agent for treating of itching.

Test Example 5: Analysis of expression of GPR4 mRNA in skin and dorsal root ganglion (DRG) of mice

Analysis of expression of GPR4 mRNA in skin and dorsal root ganglion (DRG) of mice was conducted by a Reverse Transcriptase polymerase chain reaction (RT-PCR) method. Incidentally, the following gene experiment operations were conducted by the method mentioned in Molecular Cloning.

(1) Setting up of primer: With regard to primers, a primer corresponding to a base sequence of 641st to 660th (sense chain; SEQ ID NO: 19) and 943rd to 961st (antisense chain; SEQ ID NO: 20) of mouse GPR4 (SEQ ID NO: 14) was set up and synthesized (Invitrogen).

(2) Preparation of template cDNA: skin of the back and DRG were excised from BALB/c mice. Extraction of total RNA from each tissue was conducted by a guanidium thiocyanate-phenol-chloroform (AGPC) method. cDNA was prepared using the resulting total RNA (5 μg) by a SUPERSCRIPT Preamplification System (Invitrogen) according to the manual. In order to check the contamination of genome in the preparation of cDNA, a sample to which no Reverse Transcriptase was added was prepared as a negative control [RTase(-)].

(3) Confirmation of expression by RT-PCR: The PCR was conducted using a thermal cycler PTC-200 (MJ RESEARCH). The PCR was conducted in such a manner that 20 μL of reaction solution containing 1 μL of the above-mentioned cDNA, 200 μmol/L of each dNTP (dATP, dGTP, dCTP and dTTP), 10 μmol/L of primer (SEQ ID NO: 19

and SEQ ID NO: 20), 2.5 units of Taq Gold polymerase (Perkin Elmer) and 1 × Taq Gold (Mg plus) buffer was used, heated at 95°C for 10 minutes, subjected to 28 cycles of treatment each comprising at 94°C for 1 minute and at 63°C for 30 seconds and further heated at 72°C for 5 minutes. After completion of the reaction, 10 μL was taken out from the resulting PCR solution and subjected to electrophoresis using 2% agarose gel [prepared by dissolving Agarose Nusieve (FMC Bioproduct) in a Tris-acetate buffer (40 mmol/L Tris-acetate and 1 mmol/L ethylenediamine tetraacetic acid)]. The gel was stained for 30 minutes with a 10,000-fold diluted solution of Vistra Green nucleic acid gel stain RPN 5787 (Amersham and Molecular Dynamics) and amplification of a predicted DNA fragment (0.32 kb) was confirmed by Fluor Imager (Molecular Dynamics).

As a result, no band was detected after electrophoresis in the absence of Reverse Transcriptase [RTase(-)] while, in the presence of Reverse Transcriptase [RTase(+)], band was detected after electrophoresis whereupon it was confirmed that GPR4 mRNA was expressed in skin and DRG.

From the above result, it has been also suggested that compound (I) which is also a GPR4 antagonist is useful as an agent for the treating of itching.

Since GPR4 is expressed in the skin, it is suggested that a substance which suppresses the function involved in signal transduction of protein having an amino acid sequence mentioned in SEQ ID NO: 11 is useful as an agent for treating keratinizing skin diseases, psoriasis group (vulgar psoriasis, pustular psoriasis, psoriatic erythroderma and arthrogenous psoriasis), ichthyosis group (vulgar ichthyosis, bullous congenital ichthyosiform erythroderma and non-bullous congenital ichthyosiform erythroderma), nail keratosis, pityriasis rubra pilaris/erythematous keratodermia, Darier's diseases, palm postulosis, oral leukoderma, oral papilloma/oral lichen, amyloid lichen, pemphigus group, pemphigoid, keloid, etc.

On the other hand, GPR4 is expressed in DRG and, therefore, it is suggested that a substance which suppresses the function involved in signal transduction of protein having an amino acid sequence mentioned in SEQ ID NO: 11 is also useful as an agent for treating pain after operation or of cancer, headache, toothache, menorrhalgia, otalgia, pharyngalgia, trauma ache, sympatomatic neuralgia, etc.

In addition, since GPR4 is expressed in angioendothelium (refer to http://ajpheart.physiology.org/cgi/reprint/00359.2003vl.pd f), it is suggested that a substance which suppresses the function involved in signal transduction of protein having an amino acid sequence mentioned in SEQ ID NO: 11 is useful as an agent for treating hypotension, edema, arteriosclerosis, etc.

Further, since WO 02/90925 discloses that GPR4 antagonist is able to be used for treating cancer, it is likely that compound (I) is useful as an agent for treating cancer as well.

The medicament of the present invention is characterized in containing a substance selected from a group consisting of the nitrogen-containing tricyclic compound represented by the formula (I) or the quaternary ammonium salt thereof, or the pharmaceutically acceptable salt thereof and the hydrate and the solvate thereof as an active ingredient. With regard to the medicament of the present invention, the above-mentioned substance which is an active ingredient may be administered as it is but, usually, it is desirable to administer in a form of a pharmaceutical composition containing the above-mentioned substance which is an active ingredient and one or more additives for the preparation. Such a pharmaceutical composition is able to be prepared by a method which is known or common in the field of pharmaceutical preparation science. The medicament according to the present invention in a form of a pharmaceutical composition may contain one or more other pharmaceutically active ingredient(s). Incidentally, the pharmaceutical of the present invention is able to be applied to mammals including a human being.

With regard to the administration route of the medicament of the present invention, there is no particular limitation and the most effective administration route for the treatment and/or the prevention may be appropriately selected from oral administration and parenteral administration such as intravenous injection. An example of the pharmaceutical preparation suitable for oral administration is tablets or the like and an example of the pharmaceutical preparation suitable for parenteral administration is injection preparation or the like.

In the manufacture of a solid preparation such as tablet, it is possible to use excipient such as lactose and mannitol; disintegrating agent such as starch; lubricant such as magnesium stearate; binder such as hydroxypropyl cellulose; surfactant such as fatty acid ester; and plasticizer such as glycerol.

Among the preparation suitable for parenteral administration, preparation for administration into blood vessel such as injection preparation may be preferably prepared using an aqueous medium which is isotonic to human blood. For example, an injection preparation is able to be prepared according to a conventional method as solution, suspension or dispersion together with an appropriate excipient using an aqueous medium selected from salt solution, glucose solution, a mixture of salt and glucose solution, etc. For the manufacture of pharmaceutical preparation for parenteral use, it is possible to use one or more additive(s) for pharmaceutical preparations selected from diluent, flavor, antiseptic, excipient, disintegrating agent, lubricant, binder, surfactant, plasticizer and the like.

There is no particular limitation for the dose and administering frequency of the pharmaceutical of the present invention but it is possible to appropriately select depending upon various conditions such as type of the above substance which is an active ingredient, administration route, object of treatment and/or prevention, age and body weight of patient, nature of symptom and degree of severeness. For example, it is preferred to administer 0.1 to 100 mg/kg per day for an adult by dividing into three to four times a day. However, dose and administering frequency as such may vary depending upon the above-mentioned various conditions, etc.

## Best Mode for Carrying Out the Invention

The present invention is now more specifically illustrated by way of the following Examples, Referential Examples, Preparation Examples, the scope of the present invention is not limited by Examples or the like.
Physicochemical data for each of the compounds in the following Examples and Referential Examples were measured by the following instruments.
$^1$H-NMR: JOEL JNM-EX270 (270 MHz) or JEOL JNM-GX270 (270 MHz)
MS: Micromass LCT or Micromass Quatro (measured by an APCI method)

### Example 1: Synthesis of compound 1 {2-(2-Ethyl-5,7-dimethyl-3H-imidazo[4,5-b]pyridin-3-ylmethyl)-8-(4-methylpiperazin-1-ylmethyl)-10,11-dihydro-5H-dibenz[b,f]azepine}

2-(2-Ethyl-5,7-dimethyl-3H-imidazo[4,5-b]pyridin-3-ylmethyl)-10,11-dihydro-5H-dibenz[b,f]azepine (30.0 g, 78.4 mmol) mentioned in the JP-A-7-61983 was dissolved in a mixed solvent of chloroform (300 mL) and acetic acid (300 mL), then 1-methylpiperazine (23.6 g, 236 mmol) and formaldehyde (37% aqueous solution; 7.64 g, 94.1 mmol) were added thereto and the mixture was heated at 60°C and stirred for 18 hours. After confirming the progress of the reaction by a thin-layer chromatography, a saturated aqueous solution of sodium bicarbonate was added under cooling with ice and extracted with ethyl acetate. The organic layer was successively washed with a saturated aqueous solution of sodium bicarbonate, water and a saturated aqueous solution of salt, dried over anhydrous sodium sulfate and concentrated *in vacuo.* The crystals separated therefrom were triturated with ethyl acetate to give compound 1 (27.4 g, 55.4 mmol, yield: 71%).
APCI-MS: m/z 495 ([M + H]$^+$)
$^1$H NMR ($CDCl_3$) δ(ppm): 1.30 (t, J = 7.6 Hz, 3H), 2.27 (s, 3H), 2.45 (m, 8H), 2.60 (s, 3H), 2.63 (s, 3H), 2.79 (q, J = 7.6 Hz, 2H), 2.98 (m, 4H), 3.38 (s, 2H), 5.34 (s, 2H), 6.00 (s, 1H), 6.57-6.66 (m, 2H), 6.79-7.00 (m, 5H).
The corresponding fumarate was prepared according to the following process.
Thus, the above compound 1 (15 g) was dissolved in methanol (110 mL) and fumaric acid (7.0 g, 2.0 equivalents) was added. A suspension wherefrom crystals were separated was once concentrated to dryness, acetonitrile (100 mL) was added and a suspension was stirred for not shorter than 1 hour. After that, the crystals were filtered and dried in *vacuo* to give a difumarate of compound 1 (20.1 g, yield: 91%).

### Example 2: Synthesis of compound 2 {2-(2-Ethyl-5,7-dimethyl-3H-imidazo[4,5-b]pyridin-3-ylmethyl)-8-(1,2,5,6-tetrahydropyridin-1-ylmethyl)-10,11-dihydro-5H-dibenz[b,f]azepine}

The operation similar to that in Example 1 was conducted using 1,2,3,6-tetrahydropyridine instead of 1-methylpiperazine and, starting from 2-(2-ethyl-5,7-dimethyl-3H-imidazo[4,5-b]pyridin-3-ylmethyl)-10,11-dihydro-5H-dibenz[b,f]azepine mentioned in the JP-A-7-61983, compound 2 was obtained in the yield of 20%.
APCI-MS: m/z 478 ([M + H]$^+$)
$^1$H NMR ($CDCl_3$) δ(ppm): 1.30 (t, J = 7.5 Hz, 3H), 2.04 (m, 2H), 2.53 (t, J = 5.7 Hz, 2H), 2.60 (s, 3H), 2.62 (s, 3H), 2.79 (q, J = 7.5 Hz, 2H), 2.86-3.02 (m, 6H), 3.45 (s, 2H), 5.33 (s, 2H), 5.64 (m, 1H), 5.74 (m, 1H), 6.02 (s, 1H), 6.57-6.70 (m, 2H), 6.78-6.82 (m, 2H), 6.88 (s, 1H), 6.95-7.00 (m, 2H).

### Example 3: Synthesis of compound 3 {2-(2-Ethyl-5,7-dimethyl-3H-imidazo[4,5-b]pyridin-3-ylmethyl)-8-(pyrrolidin-1-ylmethyl)-10,11-dihydro-5H-dibenz[b,f]azepine}

The operation similar to that in Example 1 was conducted using pyrrolidine instead of 1-methylpiperazine and, starting from 2-(2-ethyl-5,7-dimethyl-3H-imidazo[4,5-b]pyridin-3-ylmethyl)-10,11-dihydro-5H-dibenz[b,f]azepine mentioned in the JP-A-7-61983, compound 3 was obtained in the yield of 20%.
APCI-MS: m/z 466 ([M + H]$^+$)
$^1$H NMR ($CDCl_3$) δ(ppm): 1.30 (t, J = 7.5 Hz, 3H), 1.78 (m, 4H), 2.50 (m, 4H), 2.60 (s, 3H), 2.63 (s, 3H), 2.79 (q, J = 7.5 Hz, 2H), 2.98 (m, 4H), 3.50 (s, 2H), 5.34 (s, 2H), 6.02 (s, 1H), 6.58-6.66 (m, 2H), 6.79-6.81 (m, 2H), 6.88 (s, 1H), 6.98-7.02 (m, 2H).

Example 4: Synthesis of compound 4 {2-(2-Ethyl-5,7-dimethyl-3H-imidazo[4,5-b]pyridin-3-ylmethyl)-8-morpholinomethyl-10,11-dihydro-5H-dibenz[b,f] azepine}

The operation similar to that in Example 1 was conducted using morpholine instead of 1-methylpiperazine and, starting from 2-(2-ethyl-5,7-dimethyl-3H-imidazo[4,5-b]pyridin-3-ylmethyl)-10,11-dihydro-5H-dibenz[b,f]azepine mentioned in the JP-A-7-61983, compound 4 was obtained in the yield of 46%.
APCI-MS: m/z 482 ($[M + H]^+$)
$^1H$ NMR ($CDCl_3$) δ(ppm): 1.30 (t, J = 7.5 Hz, 3H), 2.43 (m, 4H), 2.60 (m, 3H), 2.63 (m, 3H), 2.79 (q, J = 7.5 Hz, 2H), 2.98 (m, 4H), 3.38 (s, 2H), 3.69 (m, 4H), 5.34 (s, 2H), 6.07 (s, 1H), 6.58-6.67 (m, 2H), 6.78-6.81 (m, 2H), 6.88 (s, 1H), 6.96-7.01 (m, 2H).

Example 5: Synthesis of compound 5 to compound 12

2-(2-Ethyl-5,7-dimethyl-3H-imidazo[4,5-b]pyridin-3-ylmethyl)-10,11-dihydro-5H-dibenz[b,f]azepine (19 mg, 0.050 mmol) mentioned in the JP-A-7-61983 was dissolved in a mixed solvent of chloroform (0.30 mL) and acetic acid (0.30 mL), then a chloroform solution of the corresponding $R^5R^6NH$ (1.0 mol/L, 0.15 mL) and formaldehyde (37% aqueous solution, 0.005 mL) were added thereto and the mixture was heated at 60°C and stirred for 20 hours. After confirming the progress of the reaction by a thin-layer chromatography, the solvent was evaporated and the residue was dissolved in chloroform and washed with water for two times. The organic layer was dried over anhydrous sodium sulfate and concentrated. To the residue were added chloroform (0.50 mL) and N-methylisatoic anhydride polystyrene (manufactured by Nova Biochem, 0.15 mL) were added followed by stirring at room temperature for one night. The resin in the reaction mixture was filtered off and the residue was purified by means of ion-exchange chromatography (Bondecil SCX, manufactured by Varian, eluted with a 2 mol/L methanolic solution of ammonia) to give objective compound 5 to compound 12.
Structures and analytical data (APCI-MS) of the compounds are shown in Table 1 and Table 14, respectively.

Example 6: Synthesis of compound 13 {1-[8-(2-Ethyl-5,7-dimethyl-3H-imidazol[4,5-b]pyridin-3-ylmethyl)-10,11-dihydro-5H-dibenz[b,f]azepine-2-ylmethyl]-1-methylpyrrolidinium iodide}

Compound 3 (11.4 g, 24. 5 mmol) prepared in Example 3 was dissolved in dichloromethane (200 mL), methyl iodide (1.98 mL, 31.8 mmol) was added thereto and the mixture was stirred at room temperature for 10 hours. After the reaction solution was concentrated *in vacuo*, ethyl acetate was added thereto. The resulting suspension was heated at 60°C, stirred for 0.5 hour and then stirred at room temperature for 1 hour. The solid separated out was filtered to give compound 13 (13.7 g, 22.5 mmol, yield: 92%).
APCI-MS: m/z 480 ($[M - I]^+$)
$^1H$ NMR ($CDCl_3$) δ(ppm): 1.31 (t, J = 7.6 Hz, 3H), 2.13 (br s, 2H), 2.25 (br s, 2H), 2.58 (s, 3H), 2.62 (s, 2H), 2.79 (q, J = 7.6 Hz, 2H), 2.85 (m, 4H), 3.06 (s, 3H), 3.52 (br s, 2H), 3.83 (br s, 2H), 4.74 (s, 2H), 5.32 (s, 2H), 6.76 (m, 2H), 6.88 (s, 1H), 6.95-7.18 (m, 4H), 7.43 (s, 1H).

Example 7: Synthesis of compound 14 {2-(2,5-Dihydropyrrol-1-ylmethyl)-8-(2-ethyl-5,7-dimethyl-3H-imidazo[4,5-b] pyridin-3-ylmethyl)-10,11-dihydro-5H-dibenzo[b,f]azepine}

The operation similar to that in Example 1 was conducted using 2,5-dihydropyrrole instead of 1-methylpiperazine and, starting from 2-(2-ethyl-5,7-dimethyl-3H-imidazo[4,5-b]pyridin-3-ylmethyl)-10,11-dihydro-5H-dibenz[b,f] azepine mentioned in the JP-A-7-61983, compound 14 was obtained in the yield of 82%.
APCI-MS: m/z 464 ($[M + H]^+$)
$^1H$ NMR ($CDCl_3$) δ(ppm): 1.30 (t, J = 7. 5 Hz, 3H), 2.59 (s, 3H), 2.63 (s, 3H), 2.79 (q, J = 7. 5 Hz, 2H), 2.9-3.1 (m, 4H), 3.45 (s, 4H), 3.70 (s, 2H), 5.34 (s, 2H), 5.87 (s, 2H), 6.07 (s, 1H), 6.59 (d, J = 8.7 Hz , 2H), 6.63 (d, J = 8.7 Hz , 2H), 6.75-6.85 (m, 2H), 6.88 (s, 1H), 7.00-7.05 (m, 2H).

Example 8: Synthesis of compound 15 <Methyl{N-8-(2-ethyl-5,7-dimethyl-3H-imidazo[4,5-b]pyridin -3-ylmethyl)-10,11-dihydro-5H-dibenz[b,f]azepine-2-ylmethyl}-N-methylamino}acetate>

The operation similar to that in Example 1 was conducted using sarcosine methyl ester hydrochloride instead of 1-methylpiperazine and, starting from 2-(2-ethyl-5,7-dimethyl-3H-imidazo[4,5-b]pyridin-3-ylmethyl)-10,11-dihydro-5H-dibenz[b,f]azepine mentioned in the JP-A-7-61983, compound 15 was obtained in the yield of 31%.
APCI-MS: m/z 498 ($[M + H]^+$)
$^1H$ NMR ($CDCl_3$) δ(ppm): 1.30 (t, J = 7.6 Hz, 3H), 2.36 (s, 3H), 2.60 (s, 3H), 2.63 (s, 3H), 2.79 (q, J = 7.6 Hz,

2H), 2.98 (m, 4H), 3.23 (s, 2H), 3.53 (s, 2H), 3.70 (s, 3H), 5.34 (s, 2H), 5.98 (s, 1H), 6.59-6.67 (m, 2H), 6.82 (m, 2H), 6.88 (s, 1H), 6.97-7.02 (m, 2H).

Example 9: Synthesis of compound 16 {Ethyl 1-[8-2-ethyl-5,7-dimethyl-3H-imidazo[4,5-b]pyridin-3-ylmethyl)-10,11-dihydro-5H-dibenz[b,f]azepine-2-ylmethyl] piperidine-4-carboxylate}

The operation similar to that in Example 1 was conducted using ethyl isonipecotate instead of 1-methylpiperazine and, starting from 2-(2-ethyl-5,7-dimethyl-3H-imidazo[4,5-b]pyridin-3-ylmethyl)-10,11-dihydro-5H-dibenz[b,f]azepine mentioned in the JP-A-7-61983, compound 16 was obtained in the yield of 60%.
APCI-MS: m/z 552 ($[M + H]^+$)
$^1H$ NMR ($CDCl_3$) δ(ppm): 1.23 (t, J = 7.0 Hz, 3H), 1.30 (t, J = 7.6 Hz, 3H), 1.68-1.90 (m, 6H), 1.97 (td, J = 11.3, 2.7 Hz, 2H), 2.26 (m, 1H), 2.60 (s, 3H), 2.62 (s, 3H), 2.79 (q, J = 7.6 Hz, 2H), 2.83 (m, 2H), 2.98 (m, 4H), 3.36 (s, 2H), 4.11 (q, J = 7.0 Hz, 2H), 5.33 (s, 2H), 6.03 (s, 1H), 6.57-6.66 (m, 2H), 6.78-6.82 (m, 2H), 6.88 (s, 1H), 6.94-6.99 (m, 2H).

Example 10: Synthesis of compound 17 <2-{N-[8-(2-Ethyl-5,7-dimethyl-3H-imidazo[4,5-b]pyridin-3-ylmethyl)-10,11-dihydro-5H-dibenz[b,f]azepine-2-ylmethyl]-N-methylamino}ethanol>

Lithium aluminum hydride (15.7 mg, 0.38 mmol) was suspended in tetrahydrofuran (0.3 mL) with stirring under cooling with ice. Compound 15 (126 mg, 0.253 mmol) prepared in Example 8 dissolved in tetrahydrofuran (0.9 mL) was added thereto followed by stirring at room temperature for 1. 5 hours. After confirming the progress of the reaction by means of thin-layer chromatography, water (0.016 mL), a 2 mol/L aqueous solution of sodium hydroxide (0.016 mL) and water (0.048 mL) were dropped thereinto successively with stirring. The precipitate was filtered off, the filtrate was concentrated and the resulting residue was purified by an NH-silica gel chromatography (eluting solvent: ethyl acetate) to give compound 17 (47.6 mg, 0.101 mmol, yield: 40%).
APCI-MS: m/z 470 ($[M + H]^+$)
$^1H$ NMR ($CDCl_3$) δ(ppm): 1.30 (t, J = 7.5 Hz, 3H), 1.7 (br s, 1H), 2.21 (s, 3H), 2.57 (t, J = 5.5 Hz, 2H), 2.60 (s, 3H), 2.63 (s, 3H), 2.80 (q, J = 7.5 Hz, 2H), 2.98 (m, 4H), 3.44 (s, 2H), 3.61 (t, J = 5.5 Hz, 2H), 5.34 (s, 2H), 5.99 (s, 1H), 6.59-6.67 (m, 2H), 6.81 (m, 2H), 6.88 (s, 1H), 6.91-6.98 (m, 2H).

Example 11: Synthesis of compound 18 <{1-[8-(2-Ethyl-5,7-dimethyl-3H-imidazo[4,5-b]pyridin-3-ylmethyl)-10,11-dihydro-5H-dibenz[b,f]azepin-2-ylmethyl] piperidin-4-yl}methanol>

Compound 16 was used instead of compound 15 and, in the manner similar to that in Example 10, compound 18 was prepared in a yield of 50%.
APCI-MS: m/z 510 ($[M + H]^+$)
$^1H$ NMR ($CDCl_3$)δ(ppm): 1.30 (t, J = 7.6 Hz, 3H), 1.24-1.74 (m, 6H), 1.91 (m, 2H), 2.60 (s, 3H), 2.63 (s, 3H), 2.79 (q, J = 7.6 Hz, 2H), 2.86-3.02 (m, 6H), 3.37 (s, 2H), 3.48 (d, J = 6.3 Hz, 2H), 5.34 (s, 2H), 5.98 (s, 1H), 6.58-6.67 (m, 2H), 6.82 (m, 2H), 6.89 (s, 1H), 6.94-7.00 (m, 2H).

Example 12: Synthesis of compound 19 <{N-[8-(2-Ethyl-5,7-dimethyl-3H-imidazo[4,5-b]pyridin-3-ylmethyl)-10,11-dihydro-5H-dibenz[b,f]azepin-2-ylmethyl]-N -methylamino}acetic acid>

Compound 15 (151 mg, 0.303 mmol) prepared in Example 8 was dissolved in methanol (3.0 mL), a 1 mol/L methanol solution of sodium hydroxide (1.5 mL) was added thereto and the mixture was heated at 60°C and stirred for 9 hours. After confirming the progress of the reaction by a thin-layer chromatography, it was cooled to room temperature and pH was adjusted to 6.0 by addition of 4 mol/L hydrochloric acid. The crystals separated out therefrom were filtered and dried *in vacuo*. The crystals were suspended in ethyl ether, stirred for 1 hour under heating with refluxing and then stirred for 1 hour at room temperature. The crystals were filtered and dried *in vacuo* to give compound 19 (119 mg, 0.246 mmol, yield: 81%).
APCI-MS: m/z 483 ($[M + H]^+$)
$^1H$ NMR (DMSO-$d_6$) δ(ppm): 1.23 (t, J = 7.4 Hz, 3H), 2.34 (s, 3H), 2.48-2.52 (s x 2, 6H, overlapped with DMSO), 2.78 (q, J = 7.4 Hz, 2H), 2.89 (m, 4H), 3.11 (s, 2H), 3.66 (s, 2H), 5.29 (s, 2H), 6.75-7.02 (m, 7H), 8.36 (s, 1H).

Example 13: Synthesis of compound 20 {1-[8-(2-Ethyl-5,7-dimethyl-3H-imidazo[4,5-b]pyridin-3-ylmethyl)-10,11-dihydro-5H-dibenz[b,f]azepin-2-ylmethyl] piperidine-4-carboxylic acid}

The operation similar to that in Example 12 was conducted using compound 16 instead of compound 15 to

give compound 20 in a yield of 70%.

APCI-MS: m/z 524 ($[M + H]^+$)

$^1H$ NMR (DMSO-$d_6$) δ(ppm): 1.23 (t, J = 7.4 Hz, 3H), 1.52 (m, 2H), 1.75 (m, 2H), 1.97 (m, 2H), 2.18 (m, 1H), 2.48-2.54 (s x 2, 6H, overlapped with DMSO), 2.71-2.92(m, 8H), 3.32 (s, 2H), 5.29 (s, 2H), 6.75-6.94 (m, 7H), 8.23 (s, 1H).

Example 14: Synthesis of compound 21 <{N-[8-(2-Ethyl-5,7-dimethyl-3H-imidazo[4,5-b]pyridin-3-yl methyl)-10,11-dihydro-5H-dibenz[b,f]azepin-2-ylmethyl]-N-methylamino}acetonitrile>

Compound 13 (700 mg, 1.15 mmol) prepared in Example 6 was dissolved in chloroform (1.2 mL), then methylaminoacetonitrile (368 mg, 3.46 mmol) and triethylamine (0.561 mL, 4.03 mmol) were added thereto and the mixture was stirred for one night under refluxing. The reaction solution was cooled to room temperature, a saturated aqueous solution of sodium bicarbonate was added and the mixture was extracted with chloroform for three times. The organic layers were combined, washed with a saturated aqueous saline solution, dried over anhydrous magnesium sulfate and concentrated and the residue was purified by a silica gel chromatography (eluting solvent: methanol/chloroform = 1/99). Ethanol was added to the concentrated residue of the fraction containing the objective substance and the resulting suspension was stirred at 60°C for 0.5 hour and then stirred at room temperature for 1 hour. The crystals separated out therefrom were filtered and dried *in vacuo* to give compound 21 (415 mg, 0.893 mmol, yield: 78%).

APCI-MS: m/z 465 ($[M + H]^+$)

$^1H$ NMR ($CDCl_3$) δ(ppm): 1.30 (t, J = 7.5 Hz, 3H), 2.42 (s, 3H), 2.60 (s, 3H), 2.63 (s, 3H), 2.79 (q, J = 7.5 Hz, 2H), 2.98 (m, 4H), 3.43 (s, 2H), 3.48 (s, 2H), 5.34 (s, 2H), 6.10 (s, 1H), 6.58-6.69 (m, 2H), 6.78-6.83 (m, 2H), 6.88 (s, 1H), 6.95-7.02 (m, 2H).

Example 15: Synthesis of compound 22 {N-[8-(2-Ethyl-5,7-dimethyl-3H-imidazo[4,5-b]pyridin-3-ylmethyl)-10,11-dihydro-5H-dibenz[b,f]azepin-2-ylmethyl]-N -[2-(pyrrolidin-1-yl)ethyl]amine difumarate}

Step 1

Compound 93 (1.25 g, 3.03 mmol) prepared in Example 24 which is mentioned later was dissolved in a mixed solvent of chloroform (54 mL) and acetone (6 mL), then manganese dioxide (2.7 g, 31 mmol) was added thereto and the mixture was stirred for one night at room temperature. After confirming the progress of the reaction by a thin-layer chromatography, the solid was filtered off using a Celite and the filtrate was concentrated. Ethyl acetate was added to the residue, the resulting suspension was stirred for 0.5 hour under reflux, then cooled to room temperature and stirred for 0.5 hour. The crystals separated out therefrom were filtered and dried *in vacuo* to give 8-(2-ethyl-5,7-dimethyl-3H-imidazo[4,5-b]pyridin-3-ylmethyl)-10,11-dihydro-5H-dibenz[b,f]azepin-2-carboaldehyde (1.02 g, 2.48 mmol, yield: 82%).

APCI-MS: m/z 411 ($[M + H]^+$)

$^1H$ NMR ($CDCl_3$) δ(ppm): 1.31 (t, J = 7.5 Hz, 3H), 2.60 (s, 3H), 2.64 (s, 3H), 2.80 (q, J = 7.5 Hz, 2H), 2.99 (m, 2H), 3.06 (m, 2H), 5.37 (s, 2H), 6.60-6.91 (m, 6H), 7.52-7.61 (m, 2H), 9.77 (s, 1H).

Step 2

8-(2-Ethyl-5,7-dimethyl-3H-imidazo[4,5-b]pyridin-3-ylmethyl)-10,11-dihydro-5H-dibenz[b,f]azepin-2-carboaldehyde (0.300 g, 0.73 mmol) prepared in step 1 was suspended in a mixed solvent of tetrahydrofuran (10 mL) and chloroform (6 mL), then 2-(pyrrolidin-1-yl)ethylamine (139 μL, 1.10 mmol) was added thereto and the mixture was heated to reflux for 10 minutes. After that, the reaction solution was cooled to room temperature, sodium triacetoxyborohydride (464 mg, 2.19 mmol) was added thereto and the mixture was stirred for 12 hours at room temperature. To the reaction solution were added ethyl acetate and a 1 mol/L aqueous solution of sodium hydroxide and the organic layer was dried over anhydrous magnesium sulfate. After that, the solution was concentrated *in vacuo* and the residue was purified by a silica gel chromatography (eluting solvent: chloroform/2 mol/L methanolic solution of ammonia = 20/1) to give N-[8-(2-ethyl-5,7-dimethyl-3H-imidazo[4,5-b]pyridin-3-ylmethyl)-10,11-dihydro-5H-dibenz[b,f]azepin-2-ylmethyl]-N -[2-(pyrrolidin-1-yl)ethyl]amine (0.301 g, 0.592 mmol, yield: 81%). This was made into a fumarate by the method similar to Example 1 to give compound 22.

APCI-MS: m/z 509 ($[M + H]^+$)

$^1H$ NMR (DMSO-$d_6$) δ(ppm): 1.23 (t, J = 7.4 Hz, 3H), 1.65-1.85 (m, 4H), 2.50 (s, 3H), 2.51 (s, 3H), 2.6-2.7 (m, 4H), 2.7-3.0 (m, 8H), 3.86 (s, 2H), 5.29 (s, 2H), 6.55 (s, 4H), 6.75-6.95 (m, 6H), 7.0-7.15 (m, 2H), 8.43 (s, 1H).

Example 16: Synthesis of compound 23 {N-[8-(2-Ethyl-5,7-dimethyl-3H-imidazo[4,5-b]pyridin-3-ylmethyl)-10,11-dihydro-5H-dibenz[b,f]azepin-2-ylmethyl]-N -(2-methoxyethyl) amine monofumarate}

The operation similar to that in step 2 of Example 15 was conducted using 2-methoxyethylamine instead of 2-(pyrrolidin-1-yl)ethylamine to give N-[8-(2-ethyl-5,7-dimethyl-3H-imidazo[4,5-b]pyridin-3-ylmethyl)-10,11-dihydro-5H-dibenz[b,f]azepin-2-ylmethyl]-N -(2-methoxyethyl) amine in a yield of 78%. This was converted to a fumarate by the manner similar to that in Example 1 to give compound 23.

APCI-MS: m/z 470 ($[M + H]^+$)

$^1H$ NMR (DMSO-$d_6$) δ(ppm): 1.23 (t, J = 7.4 Hz, 3H), 2.50 (s, 3H), 2.51 (s, 3H), 2.80 (q, J = 7.4 Hz, 2H), 2.8-3.0 (m, 6H), 3.24 (s, 3H), 3.49 (t, J = 6.5 Hz, 2H), 3.80 (s, 2H), 5.29 (s, 2H), 6.48 (s, 2H), 6.84 (d, J = 8.1 Hz, 1H), 6.85-7.0 (m, 4H), 7.0-7.1 (m, 2H), 8.43 (s, 1H).

Example 17: Synthesis of compound 24 <2-{[8-(2-Ethyl-5,7-dimethyl-3H-imidazo[4,5-b]pyridin-3-ylmethyl)-10,11-dihydro-5H-dibenz[b,f]azepin-2-ylmethyl]} aminoethanol 0.5 fumarate}

The operation similar to that in step 2 of Example 15 was conducted using 2-ethanolamine instead of 2-(pyrrolidin-1-yl)ethylamine to give 2-{[8-(2-ethyl-5,7-dimethyl-3H-imidazo[4,5-b]pyridin-3-ylmethyl)-10,11-dihydro-5H-dibenz[b,f]azepin-2-ylmethyl] amino}ethanol in a yield of 39%.

This was converted to a fumarate by the manner similar to that in Example 1 to give compound 24.

APCI-MS: m/z 456 ($[M + H]^+$)

$^1H$ NMR (DMSO-$d_6$) δ(ppm): 1.23 (t, J = 7.4 Hz, 3H), 2.50 (s, 3H), 2.51 (s, 3H), 2.70-2.75 (m, 2H), 2.77 (q, J = 7.4 Hz, 2H), 2.85-2.9 (m, 4H), 3.55 (t, J = 5.5 Hz, 2H), 3.78 (s, 2H), 5.29 (s, 2H), 6.44 (s, 1H), 6.79 (dd, J = 1.5 Hz, 8.3 Hz, 1H), 6.85-6.95 (m, 4H), 7.0-7.1 (m, 2H), 8.39 (s, 1H).

Example 18: Synthesis of compound 25 <{[8-(2-Ethyl-5,7-dimethyl-3H-imidazo[4,5-b]pyridin-3-ylmethyl)-10,11-dihydro-5H-dibenz[b,f]azepin-2-yl]methyl} amine monofumarate>

Compound 13 (0.300 g, 0.516 mmol) prepared in Example 6 was dissolved in a 7 mol/L methanolic solution of ammonia (5 mL), sealed and heated at 80°C for 48 hours. After that, the reaction solution was concentrated *in vacuo*. The residue was purified by a silica gel chromatography (eluting solvent: chloroform/2mol/L methanolic ammonia solution = 20/1) to give {[8-(2-ethyl-5,7-dimethyl-3H-imidazo[4,5-b]pyridin-3-ylmethyl)-10,11-dihydro-5H-dibenz[b,f]azepin-2-yl]methyl} amine (0.135 g, 0.219 mol, yield: 64%).

This was converted to a fumarate by the manner similar to that in Example 1 to give compound 25.

APCI-MS: m/z 412 ($[M + H]^+$)

$^1H$ NMR (DMSO-$d_6$) δ(ppm): 1.23 (t, J = 7.4 Hz, 3H), 2.50 (s, 3H), 2.51 (s, 3H), 2.77 (q, J = 7.4 Hz, 2H), 2.85-2.9 (m, 4H), 3.81 (s, 2H), 5.29 (s, 2H), 6.42 (s, 2H), 6.8-7.0 (m, 5H), 7.0-7.15 (m, 2H), 8.46 (s, 1H).

Example 19: Synthesis of compound 26 {N-[8-(2-Ethyl-5,7-dimethyl-3H-imidazo[4,5-b]pyridin-3-ylmethyl)-10,11-dihydro-5H-dibenz[b,f]azepin-2-ylmethyl] -N-methyl-N-(2H-tetrazol-5-ylmethyl)amine}

Compound 13 (667 mg, 1.10 mmol) prepared in Example 6 was dissolved in chloroform (11 mL), then N-methyl-N-(2-trityl-2H-tetrazol-5-ylmethyl)amine (390 mg, 1.10 mmol) prepared in Referential Example 1a and triethylamine (0.31 mL, 2.3 mmol) were added thereto and the mixture was stirred at 60°C for one night. The reaction solution was cooled to room temperature, a saturated aqueous solution of sodium bicarbonate was added and the mixture was extracted with chloroform for three times. The organic layers were combined, washed with a saturated saline solution, dried over anhydrous magnesium sulfate and concentrated. The residue was passed through silica gel (eluting solvent: methanol/chloroform = 2/98) to remove starting point components and concentrated. To the residue were added acetone (1.9 mL), water (1.9 mL) and acetic acid (1.9 mL) and the mixture was stirred at 60°C for 1.5 hours. The reaction solution was cooled down to 0°C, separated substances were filtered off, the filtrate was concentrated and the resulting residue was recrystallized from ethanol to give compound 26 (66.7 mg, 0.131 mmol, yield: 12%).

APCI-MS: m/z 508 ($[M + H]^+$)

$^1H$ NMR ($CDCl_3$) δ(ppm): 1.32 (t, J = 5.0 Hz, 3H), 2.58 (s, 3H), 2.63 (s, 3H), 2.75-2.79 (m, 7H), 2.81 (q, J = 5.0 Hz, 2H), 4.08 (s, 2H), 4.28 (s, 2H), 5.34 (s, 2H), 6.37 (s, 1H), 6.46 (d, J = 8.1 Hz, 1H), 6.58 (d, J = 8.1 Hz, 1H), 6.72-6.80 (m, 2H), 6.84-6.94 (m, 3H).

Example 20: Synthesis of compound 27 {2-(2-Ethyl-5,7-dimethyl-3H-imidazo[4,5-b]pyridin-3-ylmethyl)-8-[4-(2H-tetrazol-5-yl)piperidin-1-ylmethyl]-10,11-dihydro-5H-dibenz[b,f]azepine}

The operation similar to that in Example 14 was conducted using piperidine-4-carbonitrile instead of methylaminoacetonitrile to give 1-[8-(2-ethyl-5,7-dimethyl-3H-imidazo[4,5-b]pyridin-3-ylmethyl)-10,11-dihydro-5H-dibenz[b,f]azepin-2-ylmethyl]-piperidine-4-carbonitrile.

The resulting 1-[8-(2-ethyl-5,7-dimethyl-3H-imidazo[4,5-b]pyridin-3 -ylmethyl)-10,11-dihydro-5H-dibenz[b,f]azepin-2-ylmethyl]-piperidine-4-carbonitrile (0.252 g, 0.500 mmol) was dissolved in toluene (4 mL), then trimethylsilyl azide (0.13 mL, 1.00 mmol) and dibutyltin oxide (12.4 mg, 0.05 mmol) were added and the mixture was heated with stirring at 110°C for 22 hours. The reaction solution was concentrated *in vacuo* and ethanol was added to the residue. The resulting suspension was heated to reflux for 0.5 hour and the solid was filtered to give compound 27 (0.110 g, 0.200 mmol, yield: 40%).

APCI-MS: m/z 548 ($[M + H]^+$)

$^1H$ NMR (DMSO-$d_6$) δ(ppm): 1.22 (t, J = 7.4 Hz, 3H), 1.65-1.85 (m, 2H), 1.9-2.05 (m, 2H), 2.2-2.35 (m, 2H), 2.48 (s, 3H), 2.58 (s, 3H), 2.77 (q, J = 7.4 Hz, 2H), 2.85-3.05 (m, 7H), 3.52 (s, 2H), 5.29 (s, 2H), 6.85-7.05 (m, 8H), 8.36 (s, 1H).

Example 21: Synthesis of compounds 28 to 90

Step 1

1-(10,11-Dihydro-5H-dibenz[b,f]azepin-2-ylmethyl)-1-methylpiperidinium iodide (0.015 g, 0.050 mmol) was dissolved in dimethyl formamide (0.50 mL), then a chloroform solution of the corresponding YH (wherein Y has the same meaning as defined above) (1.0 mmol/L, 0.060 mL) and lithium hydroxide monohydrate (0.070 g) were added and the mixture was stirred at room temperature for 20 hours. After confirming the progress of the reaction by a thin-layer chromatography, the solvent is evaporated, the residue was dissolved in dichloromethane and the resulting solution was washed with water for three times. The organic layer was dried over anhydrous sodium sulfate and concentrated, chloroform (0.60 mL) and N-methylisatoic anhydride polystyrene (manufactured by Nova Biochem, 0.15 mL) were added thereto and the mixture was stirred at room temperature for one night. The resin in the reaction mixture was filtered off, the filtrate was concentrated and the residue was purified by an ion-exchange chromatography (Bondecil SCX, manufactured by Barian, eluted with 2 mol/L methanolic solution of ammonia) to give various intermediates corresponding to compound (IV) in the Producing Process 1.

Step 2

The operation similar to that in Example 5 was conducted to give desired compounds 28 to 90 from various intermediates corresponding to compound (IV) in the Producing Process 1 obtained in step 1 and the corresponding $R^5R^6NH$ (wherein $R^5$ and $R^6$ each have the same meaning as defined above, respectively). Incidentally, compounds 41, 42, 48 and 89 were isolated as oxalates.

Structures and analytical data (APCI-MS) of compounds 28 to 87 are shown in Tables 2 to 6. Analytical data ($^1H$-NMR) of compounds 29, 30, 36, 41, 42, 48, 53, 54, 60, 65, 66, 72, 77, 78 and 84 are shown below.

Compound 29:

{2-(Benzimidazol-1-ylmethyl)-8-(1,2,5,6-tetrahydropyridin -1-ylmethyl)-10,11-dihydro-5H-dibenz[b,f]azepine}

$^1H$ NMR (DMSO-$d_6$) δ(ppm): 2.0-2.1 (m, 2H), 2.44 (t, J = 5.6 Hz, 2H), 2.75-2.85 (m, 2H), 2.9-3.0 (m, 4H), 3.32 (s, 2H), 5.31 (s, 2H), 5.5-5.8 (m, 2H), 6.8-7.1 (m, 6H), 7.1-7.3 (m, 2H), 7.56 (d, J = 7.1 Hz, 1H), 7.62 (d, J = 7.4 Hz, 1H), 8.28 (s, 1H), 8.35 (s, 1H).

Compound 30:

{2-(Benzimidazol-1-ylmethyl)-8-(pyrrolidin-1-ylmethyl)-10, 11-dihydro-5H-dibenz[b,f]azepine}

$^1H$ NMR (DMSO-$d_6$) δ(ppm): 1.5-1.7 (m, 4H), 2.3-2.5 (m, 4H), 2.8-3.0 (m, 4H), 3.39 (s, 2H), 5.31 (s, 2H), 6.8-6.95 (m, 4H), 6.95-7.0 (m, 2H), 7.1-7.3 (m, 2H), 7.55 (d, J = 8.9 Hz, 1H), 7.63 (d, J = 8.4 Hz, 1H), 8.26 (s, 1H), 8.34 (s, 1H).

Compound 36:

{2-(Benzimidazo-1-ylmethyl)-8-morpholinomethyl-10,11-dihydro-5H-dibenz[b,f]azepine}

$^1H$ NMR (DMSO-$d_6$)δ(ppm): 2.2-2.4 (m, 4H), 2.8-3.0 (m, 4H), 3.27 (s, 2H), 3.5-3.6 (m, 4H), 5.30 (s, 2H), 6.7-7.1 (m, 6H), 7.1-7.25 (m, 2H), 7.54 (d, J = 7.6 Hz, 1H), 7.62(d, J = 7.6 Hz, 1H), 8.28 (s, 1H), 8.34 (s, 1H).

Compound 41:

{2-(2-Phenylbenzimidazol-1-ylmethyl)-8-(1,2,5,6-tetrahydropyridin-1-ylmethyl)-10,11-dihydro-5H-dibenz [b,f]azepine monooxalate}

$^1H$ NMR (DMSO-$d_6$) δ(ppm): 2.2-2.5 (m, 2H), 2.7-3.0 (m, 4H), 3.0-3.2 (m, 2H), 3.4-3.6 (m, 2H), 4.05 (s, 2H), 5.45 (s, 2H), 5.69 (m, 1H), 5.85 (m, 1H), 6.6-6.8 (m, 2H), 6.88 (d, J = 8.3 Hz, 1H), 6.97 (d, J = 7.9 Hz, 1H), 7.05-7.2 (m, 2H), 7.2-7.5 (m, 2H), 7.5-7.7 (m, 4H), 7.7-7.85 (m, 3H), 8.54 (s, 1H).

Compound 42:

{2-(2-Phenylbenzimidazol-1-ylmethyl)-8-(pyrrolidin-1-ylmethyl)-10,11-dihydro-5H-dibenz[b,f]azepine monooxalate}

$^1H$ NMR (DMSO-$d_6$) δ(ppm): 1.8-2.0 (m, 4H), 2.8-3.0 (m, 4H), 3.0-3.2 (m, 4H), 4.12 (s, 2H), 5.45 (s, 2H), 6.6-6.7 (m, 2H), 6.88 (d, J = 8.1 Hz, 1H), 6.96 (d, J = 7.8 Hz, 1H), 7.1-7.2 (m, 2H), 7.2-7.3 (m, 2H), 7.4-7.6 (m, 4H), 7.6-7.8 (m, 3H), 8.53 (s, 1H).

Compound 48:

{2-Morpholinomethyl-8-(2-phenylbenzimidazol-1-ylmethyl) -10,11-dihydro-5H-dibenz[b,f]azepine monooxalate}

$^1H$ NMR (DMSO-$d_6$) δ(ppm): 2.7-3.0 (m, 8H), 3.6-3.8 (m, 4H), 3.83 (s, 2H), 5.42 (s, 2H), 6.65-6.7 (m, 2H), 6.85 (d, J = 8.2 Hz, 1H), 6.92 (d, J = 8.1 Hz, 1H), 7.0-7.1 (m, 2H), 7.2-7.3 (m, 2H), 7.4-7.6 (m, 4H), 7.65-7.8 (m, 3H), 8.44 (s, 1H).

Compound 53:

{2-(2-Methylbenzimidazol-1-ylmethyl)-8-(1,2,5,6-tetrahydropyridin-1-ylmethyl)-10,11-dihydro-5H-dibenz [b,f]azepine}

$^1H$ NMR (DMSO-$d_6$) δ(ppm): 2.0-2.1 (m, 2H), 2.45 (t, J = 5.6 Hz, 2H), 2.54 (s, 3H), 2.75-2.85 (m, 2H), 2.85-3.0 (m, 4H), 3.35 (s, 2H), 5.28 (s, 2H), 5.55-5.75 (m, 2H), 6.8-7.0 (m, 6H), 7.1-7.2 (m, 2H), 7.4-7.6 (m, 2H), 8.28 (s, 1H).

Compound 54:

{2-(2-Methylbenzimidazol-1-ylmethyl)-8-(pyrrolidin-1-ylmethyl)-10,11-dihydro-5H-dibenz[b,f]azepine}

$^1H$ NMR (DMSO-$d_6$) δ(ppm): 1.5-1.8 (m, 4H), 2.3-2.5 (m, 4H), 2.54 (s, 3H), 2.8-3.0 (m, 4H), 3.39 (s, 2H), 5.28 (s, 2H), 6.7-6.9 (m, 6H), 7.1-7.2 (m, 2H), 7.3-7.5 (m, 2H), 8.25 (s, 1H).

Compound 60:

{2-(2-Methylbenzimidazo-1-ylmethyl)-8-morpholinomethyl-10, 11-dihydro-5H-dibenz[b,f]azepine}

$^1H$ NMR (DMSO-$d_6$) δ(ppm): 2.2-2.4 (m, 4H), 2.49 (s, 3H), 2.8-3.0 (m, 4H), 3.28 (s, 2H), 3.5-3.6 (m, 4H), 5.28 (s, 2H), 6.8-7.0 (m, 6H), 7.1-7.2 (m, 2H), 7.5-7.6 (m, 2H), 8.28 (s, 1H).

Compound 65:

{2-(5,6-Dimethylbenzimidazol-1-ylmethyl)-8-(1,2,5,6-tetrahydropyridin-1-ylmethyl)-10,11-dihydro-5H-dibenz [b,f] azepine}

$^1$H NMR (DMSO-$d_6$) δ(ppm): 2.0-2.1 (m, 2H), 2.2-2. 4 (m, 6H), 2. 45 (t, J = 5.2 Hz, 2H), 2.75-2.85 (m, 2H), 2.85-3.05 (m, 4H), 3.30 (s, 2H), 5.24 (s, 2H), 5.6-5.7 (m, 2H), 6.8-7.0 (m, 6H), 7.31 (s, 1H), 7.40 (s, 1H), 8.17 (s, 1H), 8.27 (s, 1H).

Compound 66:

{2-(5,6-Dimethylbenzimidazol-1-ylmethyl)-8-(pyrrolidin-1-ylmethyl)-10,11-dihydro-5H-dibenz[b,f]azepine}

$^1$H NMR (DMSO-$d_6$) δ(ppm): 1.5-1.8 (m, 4H), 2.27 (s, 3H), 2.28 (s, 3H), 2.3-2.4(m, 4H), 2.8-3.0 (m, 4H), 3.39 (s, 2H), 5.24 (s, 2H), 6.8-7.0 (m, 6H), 7.30 (s, 1H), 7.40 (s, 1H), 8.16 (s, 1H), 8.24 (s, 1H).

Compound 72:

{2-(5,6-Dimethylbenzimidazol-1-ylmethyl)-8-morpholinomethyl-10,11-dihydro-5H-dibenz[b,f]azepine}

$^1$H NMR (DMSO-$d_6$) δ(ppm): 2.2-2.4 (m, 10H), 2.8-3.0 (m, 4H), 3.28 (s, 2H), 3.5-3.6 (m, 4H), 5.24 (s, 2H), 6.8-7.0 (m, 6H), 7.30 (s, 1H), 7.39 (s, 1H), 8.16 (s, 1H), 8.28 (s, 1H).

Compound 77:

{2-(2-Ethylbenzimidazol-1-ylmethyl)-8-(1,2,5,6-tetrahydropyridin-1-ylmethyl)-10,11-dihydro-5H-dibenz [b,f]azepine}

$^1$H NMR (DMSO-$d_6$) δ(ppm): 1.28 (t, J = 7.4 Hz, 3H), 1.95-2.05 (m, 2H), 2.43 (t, J = 5.4 Hz, 2H), 2.6-3.0 (m, 8H), 3.32 (s, 2H), 5.28 (s, 2H), 5.1-5.5 (m, 2H), 6.75-7.0 (m, 6H), 7.1-7.25 (m, 2H), 7.47 (m, 1H), 7.55 (m, 1H), 8.26 (s, 1H).

Compound 78:

{2-(2-Ethylbenzimidazol-1-ylmethyl)-8-(pyrrolidin-1-ylmethyl)-10,11-dihydro-5H-dibenz[b,f]azepine}

$^1$H NMR (DMSO-$d_6$) δ(ppm): 1.28 (t, J = 7.4 Hz, 3H), 1.6-1.8 (m, 4H), 2.3-2.4 (m, 4H), 2.8-3.0 (m, 6H), 3.32 (s, 2H), 5.28 (s, 2H), 6.7-7.0 (m, 6H), 7.0-7.2 (m, 2H), 7.46 (m, 1H), 7.54 (m, 1H), 8.23 (s, 1H).

Compound 84:

{2-(2-Ethylbenzimidazo-1-ylmethyl)-8-morpholinomethyl-10,1 1-dihydro-5H-dibenz[b,f]azepine}

$^1$H NMR (DMSO-$d_6$) δ(ppm): 1.28 (t, J = 7.4 Hz, 3H), 2.2-2.4 (m, 4H), 2.8-3.0 (m, 6H), 3.27 (s, 2H), 3.5-3.6 (m, 4H), 5.27 (s, 2H), 6.7-7.0 (m, 6H), 7.1-7.2 (m, 2H), 7.47 (m, 1H), 7.55 (m, 1H), 8.26 (s, 1H).

Structures of compounds 88 to 90 are shown in Table 7 and analytical data thereof (APCI-MS and $^1$H-NMR) are shown below.

Compound 88:

{2-(Imidazo[4,5-b]pyridin-1-ylmethyl)-8-(1,2,5,6-tetrahydropyridin-1-ylmethyl)-10,11-dihydro-5H-dibenz [b,f]azepine}

APCI-MS: m/z 422 ($[M + H]^+$)

$^1$H NMR (DMSO-$d_6$) δ(ppm): 2.0-2.1 (m, 2H), 2.44 (t, J = 5.4 Hz, 2H), 2.75-2.8 (m, 2H), 2.8-3.0 (m, 4H), 3.30 (s, 2H), 5.33 (s, 2H), 5.5-5.6 (m, 2H), 6.8-7.0 (m, 4H), 7.0-7.05 (m, 2H), 7.27 (dd, J = 4.7 Hz, 8.0 Hz, 1H), 8.06 (d, J = 8.0 Hz, 1H), 8.27 (s, 1H), 8.37 (d, J = 4.7 Hz, 1H), 8.54 (s, 1H).

Compound 89:

{2-(Imidazo[4,5-b]pyridin-3-ylmethyl)-8-(1,2,5,6-tetrahydropyridin-1-ylmethyl)-10,11-dihydro-5H-dibenz [b,f]azepine monooxalate}

APCI-MS: m/z 422 ($[M + H]^+$)
$^1H$ NMR (DMSO-$d_6$) δ(ppm): 2.2-2.3 (m, 2H), 2.9-3.0 (m, 4H), 3.4-3.5 (m, 2H), 3.60 (t, J = 6.8 Hz, 2H), 4.05 (s, 2H), 5.37 (s, 2H), 5.67 (d, J = 10.8 Hz, 1H), 5.85 (d, J = 10.8 Hz, 1H), 6.9-7.0 (m, 2H), 7.0-7.1 (m, 4H), 7.25 (dd, J = 5.4, 8.1 Hz, 1H), 8.01 (d, J = 8.1 Hz, 1H), 8.40 (d, J = 5.4 Hz, 1H), 8.55 (s, 1H), 8.62 (s, 1H).

Compound 90:

{2-(Imidazo[4,5-c]pyridin-1-ylmethyl)-8-(1,2,5,6-tetrahydropyridin-1-ylmethyl)-10,11-dihydro-5H-dibenz[b,f] azepine}

APCI-MS: m/z 422 ($[M + H]^+$)
$^1H$ NMR ($CDCl_3$) δ(ppm): 2.1-2.2 (m, 2H), 2.56 (t, J = 5.7 Hz, 2H), 2.8-2.9 (m, 2H), 3.0-3.1 (m, 4H), 3.48 (s, 2H), 5.30 (s, 2H), 5.67 (d, J = 10.5 Hz, 1H), 5.73 (d, J = 10.5 Hz, 1H), 6.08 (s, 1H), 6.65-6.75 (m, 2H), 6.95-7.0 (m, 2H), 7.0-7.05 (m, 2H), 7.71 (d, J = 5.4 Hz, 1H), 8.02 (s, 1H), 8.45 (d, J = 5.4 Hz, 1H), 8.78 (s, 1H).

Example 22: Synthesis of compound 125 {[8-(2-Ethyl-5,7-dimethyl-3H-imidazo[4,5-b]pyridin-3-ylmethyl)-5-methyl-10,11-dihydro-5H-dibenz[b,f]azepin-2-yl]acetic acid}

The operation similar to that in step 1 of Example 52 was conducted using compound 105 prepared in Example 36 to prepare [8-(2-ethyl-5,7-dimethyl-3H-imidazo[4,5-b]pyridin-3-ylmethyl)-5-methyl-10,11-dihydro-5H-dibenz[b,f]azepin-2-yl]acetonitrile in a yield of 94%.
The operation similar to that in Example 38 was conducted using the above compound to give compound 125 in a yield of 86%.
APCI-MS: m/z 455 ($[M + H]^+$)
$^1H$ NMR (DMSO-$d_6$) δ(ppm): 1.22 (t, J = 7.3 Hz, 3H), 2.49 (s, 3H), 2.50 (s, 3H), 2.75 (q, J = 7.3 Hz, 2H), 2.9-3.1 (m, 4H), 3.19 (s, 3H), 3.42 (s, 2H), 5.32 (s, 2H), 6.81 (d, J = 8.1 Hz, 1H), 6.9-7.05 (m, 6H).

Example 23: Synthesis of compound 92 {[8-(2-Ethyl-5,7-dimethyl-3H-imidazo[4,5-b]pyridin-3-ylmethyl)-10,11-dihydro-5H-dibenz[b,f]azepin-2-ylmethyl] acetate}

Compound 13 (7.98 g, 13.1 mmol) prepared in Example 6 was dissolved in dimethyl sulfoxide (87 mL), lithium acetate (4.33 g, 65.7 mL) was added thereto and the mixture was stirred at 70°C for 2 days. The reaction solution was diluted with ethyl acetate, washed with water (for three times) and a saturated saline solution successively, dried over anhydrous magnesium sulfate and concentrated. The residue was purified using a silica gel chromatography (eluting solvent: ethyl acetate) to concentrate a fraction containing the objective product, ethanol was added to the residue and the resulting suspension was stirred at room temperature for 0.5 hour. The crystals separated out therefrom were filtered to give compound 92 (2.87 g, 6.31 mmol, yield: 48%).
APCI-MS: m/z 455 ($[M + H]^+$)
$^1H$ NMR ($CDCl_3$) δ(ppm): 1.30 (t, J = 7.5 Hz, 3H), 2.06 (s, 3H), 2.60 (s, 3H), 2.62 (s, 3H), 2. 79 (q, J = 7.5 Hz, 2H), 2.98 (m, 4H), 4.98 (s, 2H), 5.34 (s, 2H), 6.13 (s, 1H), 6.58-6.83 (m, 4H), 6.88 (s, 1H), 7.01-7.07 (m, 2H).

Example 24: Synthesis of compound 93 {[8-(2-Ethyl-5,7-dimethyl-3H-imidazo[4,5-b]pyridin-3-ylmethyl)-10,11-dihydro-5H-dibenz[b,f]azepin-2-yl] methanol}

Compound 92 (2.79 g, 6.14 mmol) prepared in Example 23 was suspended in tetrahydrofuran (61 mL), then a methanolic solution of sodium methoxide (28%, 6.2 mL, 31 mmol) was added and the mixture was stirred at room temperature for 3.5 hours. After confirming the progress of the reaction by a thin-layer chromatography, water was added to the reaction solution and the mixture was stirred at room temperature for 0.5 hour. The crystals precipitated therefrom were filtered, dried *in vacuo* and suspended in ethanol and the suspension was stirred for 1 hour under reflux and then stirred for another 1 hour at room temperature. The crystals separated out therefrom were filtered and dried *in vacuo* to give compound 93 (2.04 g, 4.95 mmol, yield: 81%).
APCI-MS: m/z 413 ($[M + H]^+$)
$^1H$ NMR ($CDCl_3$) δ(ppm): 1.30 (t, J = 7.6 Hz, 3H), 1.56 (t, J = 5.6 Hz, 1H), 2.60 (s, 3H), 2.63 (s, 3H), 2.79 (q, J = 7.6 Hz, 2H), 2.98 (m, 4H), 4.55 (d, J = 5.6 Hz, 2H), 5.34 (s, 2H), 6.03 (s, 1H), 6.59-6.85 (m, 3H), 6.88 (s, 1H), 7.03

(m, 2H).

Example 25: Synthesis of compound 94 {2-(2-Ethyl-5,7-dimethyl-3H-imidazo[4,5-b]pyridin-3-ylmethyl)-8-methoxymethyl-10,11-dihydro-5H-dibenz[b,f] azepine}

Methanol (20 µL, 0.50 mmol) was added to a suspension of sodium hydride (55%, 11 mg, 0.25 mmol) in tetrahydrofuran (0.40 mL) and the mixture was stirred at room temperature for 20 minutes. After that, the reaction solution was added to a suspension of compound 13 (30 mg, 0.050 mmol) prepared in Example 6 in tetrahydrofuran (0.20 mL) and the reaction was conducted at 60°C for 3.5 hours. After concentrating the reaction solution, the residue was dissolved in chloroform, the resulting solution was washed with water and a saturated saline solution successively, dried over anhydrous magnesium sulfate and concentrated. The residue was purified by a silica gel chromatography (eluting solvent: ethyl acetate/hexane/triethylamine = 45/50/5) to give compound 94 (6.5 mg, 15 mmol, yield: 30%).

APCI-MS: m/z 427 ($[M + H]^+$)

$^1H$ NMR ($CDCl_3$) δ(ppm): 1.30 (t, J = 7.6 Hz, 3H), 2.60 (s, 3H), 2.63 (s, 3H), 2.79 (q, J = 7.6 Hz, 2H), 2.98 (m, 4H), 3.36 (s, 3H), 4.32 (s, 2H), 5.34 (s, 2H), 6.09 (s, 1H), 6.58-6.82 (m, 4H), 6.88 (s, 1H), 7.01 (m, 2H).

Example 26: Synthesis of compound 95 {2-Allyloxymethyl-8-(2-ethyl-5,7-dimethyl-3H-imidazo [4,5-b]pyridin-3-ylmethyl)-10,11-dihydro-5H-dibenz[b,f] azepine}

The operation smilar to that in Example 25 was conducted using allyl alcohol instead of methanol to give compound 95 in a yield of 34%.

APCI-MS: m/z 453 ($[M + H]^+$)

$^1H$ NMR ($CDCl_3$)δ(ppm): 1.30 (t, J = 7.6 Hz, 3H), 2.60 (s, 3H), 2.63 (s, 3H), 2.80 (q, J = 7.6 Hz, 2H), 2.98 (m, 4H), 4.00 (dt, J = 5.6, 1.5 Hz, 2H), 4.39 (s, 2H), 5.19 (dq, J = 10.2, 1.5 Hz, 1H), 5.29 (dq, J = 17.0, 1.5 Hz, 1H), 5.34 (s, 2H), 5.95 (m, 1H), 6.10 (s, 1H), 6.58-6.83 (m, 4H), 6.88 (s, 1H), 7.03 (m, 2H).

Example 27: Synthesis of compound 96 {2-(2-Ethyl-5,7-dimethyl-3H-imidazo[4,5-b]pyridin-3-ylmethyl)-8-(2-methoxyethoxymethyl)-10,11-dihydro-5H-dibenz[b,f]azepine}

The operation similar to that in Example 25 was conducted using 2-methoxyethanol instead of methanol to give compound 96 in a yield of 9.3%.

APCI-MS: m/z 495 ($[M + H]^+$)

$^1H$ NMR ($CDCl_3$) δ(ppm): 1.30 (t, J = 7.5 Hz, 3H), 2.60 (s, 3H), 2.63 (s, 3H), 2.80 (q, J = 7.5 Hz, 2H), 2.98 (m, 4H), 3.38 (s, 3H), 3.57 (m, 4H), 4.44 (s, 2H), 5.34 (s, 2H), 6.01 (s, 1H), 6.62 (d, J = 8.6 Hz, 1H), 6.67 (d, J = 8.1 Hz, 1H), 6.82 (m, 2H), 6.88 (s, 1H), 7.00-7.06 (m, 2H).

Example 28: Synthesis of compound 97 {2-(2-Ethyl-5,7-dimethyl-3H-imidazo[4,5-b]pyridin-3-ylmethyl)-8-(2,2,2-trifluoroethoxymethyl)-10,11-dihydro-5H -dibenz[b,f]azepine}

The operation similar to that in Example 25 was conducted using 2,2,2-trifluoroethanol instead of methanol to give compound 97 in a yield of 64%.

APCI-MS: m/z 495 ($[M + H]^+$)

$^1H$ NMR ($CDCl_3$) δ(ppm): 1.30 (t, J = 7.6 Hz, 3H), 2.60 (s, 3H), 2.63 (s, 3H), 2.79 (q, J = 7.6 Hz, 2H), 2.98 (m, 4H), 3.78 (q, J = 8.7 Hz, 2H), 4.54 (s, 2H), 5.34 (s, 2H), 6.24 (s, 1H), 6.60 (d, J = 7.8 Hz, 1H), 6.71 (d, J = 8.1 Hz, 1H), 6.76-6.82 (m, 2H), 6.89 (s, 1H), 6.98-7.04 (m, 2H)

Example 29: Synthesis of compound 98 {2-(2-Ethyl-5,7-dimethyl-3H-imidazo[4,5-b]pyridin-3-ylmethyl)-8-(2-methylpropoxymethyl)-10,11-dihydro-5H-dibenz[b,f]azepine}

The operation similar to that in Example 25 was conducted using 2-methyl-1-propanol instead of methanol to give compound 98 in a yield of 11%.

APCI-MS: m/z 469 ($[M + H]^+$)

$^1H$ NMR ($CDCl_3$) δ(ppm): 0.91 (d, J = 6.7 Hz, 6H), 1.30 (t, J = 7.4 Hz, 3H), 1.89 (m, 1H), 2.60 (s, 3H), 2.63 (s, 3H), 2.79 (q, J = 7.4 Hz, 2H), 2.99 (m, 4H), 3.20 (d, J = 6.5 Hz, 2H), 4.37 (s, 2H), 5.34 (s, 2H), 6.01 (s, 1H), 6.60 (d, J = 8.9 Hz, 1H), 6.67 (d, J = 7.8 Hz, 1H), 6.81 (m, 2H), 6.88 (s, 1H), 6.98-7.05 (m, 2H).

Example 30: Synthesis of compound 99 {2-Benzyloxymethyl-8-(2-ethyl-5,7-dimethyl-3H-imidazo[4,5-b]pyridin-3-ylmethyl)-10,11-dihydro-5H-dibenz[b,f] azepine}

The operation similar to that in Example 25 was conducted using benzyl alcohol instead of methanol to give compound 99 in a yield of 78%.
APCI-MS: m/z 503 ($[M + H]^+$)
$^1H$ NMR ($CDCl_3$) δ(ppm): 1.30 (t, J = 7.5 Hz, 3H), 2.60 (s, 3H), 2.62 (s, 2H), 2.79 (q, J = 7.5 Hz, 2H), 2.97 (m, 4H), 4.42 (s, 2H), 4.53 (s, 2H), 5.33 (s, 2H), 6.20 (s, 1H), 6.59 (d, J = 7.9 Hz, 1H), 6.69 (d, J = 7.9 Hz, 1H), 6.78 (m, 2H), 6.88 (s, 1H), 7.02 (m, 2H), 7.26-7.36 (m, 5H).

Example 31: Synthesis of compound 100 {2-(2-Ethyl-5,7-dimethyl-3H-imidazo[4,5-b]pyridin-3-ylmethyl)-8-(2-phenylethoxymethyl)-10,11-dihydro-5H-dibenz[b,f]azepine}

The operation similar to that in Example 25 was conducted using 2-phenylethanol instead of methanol to give compound 100 in a yield of 38%.
APCI-MS: m/z 517 ($[M + H]^+$)
$^1H$ NMR ($CDCl_3$) δ(ppm): 1.30 (t, J = 7.5 Hz, 3H), 2.60 (s, 3H), 2.63 (s, 3H), 2.80 (q, J = 7.5 Hz, 2H), 2.91 (t, J = 7.2 Hz, 2H), 2.97 (m, 4H), 3.66 (t, J = 7.2 Hz, 2H), 4.39 (s, 2H), 5.34 (s, 2H), 6.08 (s, 1H), 6.60 (d, J = 8.7 Hz, 1H), 6.66 (d, J = 8.1 Hz, 1H), 6.80 (m, 2H), 6.88 (s, 1H), 6.94-7.01 (m, 2H), 7.19-7.30 (m, 5H).

Example 32: Synthesis of compound 101 {2-(2-Ethyl-5,7-dimethyl-3H-imidazo[4,5-b]pyridin-3-ylmethyl)-8-(pyridin-2-ylmethoxymethyl)-10,11-dihydro-5H-dibenz[b,f]azepine}

The operation similar to that in Example 25 was conducted using pyridin-2-ylmethanol instead of methanol to give compound 101 in a yield of 65%.
APCI-MS: m/z 504 ($[M + H]^+$)
$^1H$ NMR ($CDCl_3$) δ(ppm): 1.30 (t, J = 7.5 Hz, 3H), 2.60 (s, 3H), 2.63 (s, 3H), 2.80 (q, J = 7.5 Hz, 2H), 2.98 (m, 4H), 4.52 (s, 2H), 4.66 (s, 2H), 5.34 (s, 2H), 6.25 (s, 1H), 6.60 (d, J = 7.9 Hz, 1H), 6.70 (d, J = 7.9 Hz, 1H), 6.76-6.81 (m, 2H), 6.88 (s, 1H), 7.03-7.08 (m, 2H), 7.18 (br dd, J = 7.6, 4.8 Hz, 1H), 7.47 (d, J = 7.9 Hz, 1H), 7.68 (td, J = 7.7, 1.8 Hz, 1H), 8.54 (br d, J = 4.8 Hz, 1H).

Example 33: Synthesis of compound 102 {2-(2-Ethyl-5,7-dimethyl-3H-imidazo[4,5-b]pyridin-3-ylmethyl)-8-(furan-2-ylmethoxymethyl)-10,11-dihydro-5H-dibenz[b,f]azepine}

The operation similar to that in Example 25 was conducted using furan-2-ylmethanol instead of methanol to give compound 102 in a yield of 77%.
APCI-MS: m/z 493 ($[M + H]^+$)
$^1H$ NMR ($CDCl_3$) δ(ppm): 1.30 (t, J = 7.5 Hz, 3H), 2.60 (s, 3H), 2.62 (s, 3H), 2.79 (q, J = 7.5 Hz, 2H), 2.97 (m, 4H), 4.41 (s, 2H), 4.45 (s, 2H), 5.33 (s, 2H), 6.21 (br s, 1H), 6.31 (dd, J = 3.1, 0.8 Hz, 1H), 6.33 (dd, J = 3.1, 1.8 Hz, 1H), 6.58 (d, J = 8.1 Hz, 1H), 6.69 (d, J = 7.9 Hz, 1H), 6.75-6.80 (m, 2H), 6.88 (s, 1H), 7.00-7.04 (m, 2H), 7.40 (dd, J = 1.8, 0.8 Hz, 1H).

Example 34: Synthesis of compound 103 {8-(2-Ethyl-5,7-dimethyl-3H-imidazo[4,5-b]pyridin-3-ylmethyl)-10,11-dihydro-5H-dibenz[b,f]azepine-2-carbonitrile}

8-(2-Ethyl-5,7-dimethyl-3H-imidazo[4,5-b]pyridin-3-ylmethyl)-10,11-dihydro-5H-dibenz[b,f]azepin-2-carboaldehyde (650 mg, 1.58 mmol) prepared in step 1 of Example 15 was suspended in acetonitrile (16 mL), then hydroxylamine hydrochloride (153 mg, 2.38 mmol), triethylamine (0.331 mL, 2.38 mmol) and phthalic anhydride (328 mg, 2.21 mmol) were added thereto and the mixture was stirred at 80°C for one night. The reaction solution was concentrated, the residue was dissolved in chloroform, the resulting solution was washed with an aqueous ammonia solution (3%) and a saturated saline solution successively, dried over anhydrous magnesium sulfate and concentrated. The residue was purified by a silica gel chromatography (eluting solvent: methanol/chloroform = 1/99), the fraction containing the objective product was concentrated, ethanol was added to the residue and the resulting suspension was stirred at 60°C for 0.5 hour and stirred at room temperature for 1 hour. The crystals precipitated therefrom were filtered and dried *in vacuo* to give compound 103 (440 mg, 1.08 mmol, yield: 68%).
APCI-MS: m/z 408 ($[M + H]^+$)
$^1H$ NMR ($CDCl_3$)δ (ppm): 1.31 (t, J = 7.6 Hz, 3H), 2.60 (s, 3H), 2.63 (s, 3H), 2.79 (q, J = 7.6 Hz, 2H), 2.98 (m, 4H), 5.36 (s, 2H), 6.48 (s, 1H), 6.63-6.90 (m, 5H), 7.28-7.33 (m, 2H).

Example 35: Synthesis of compound 104 {2-(2-Ethyl-5,7-dimethyl-3H-imidazo[4,5-b]pyridin-3-ylmethyl)-8-(2H-tetrazol-5-yl)-10,11-dihydro-5H-dibenz [b,f]azepine}

Compound 103 prepared in Example 34 was used and, by the similar manner as in the latter part of Example 20, compound 104 was obtained in a yield of 72%
APCI-MS: m/z 451 ([M + H]$^+$)
APCI-MS: m/z 451 ([M + H]$^+$)
$^1$H NMR (DMSO-$d_6$) δ(ppm): 1.24 (t, J = 7.4 Hz, 3H), 2.48-2.53 (s x 2, 6H, overlapped with DMSO), 2.80 (q, J = 7.4 Hz, 2H), 2.86-3.02 (m, 4H), 5.32 (s, 2H), 6.83 (dd, J = 8.1, 2.1 Hz, 1H), 6.91-6.98 (m, 3H), 7.10 (d, J = 9.0 Hz, 1H), 7.65-7.70 (m, 2H), 8.20 (s, 1H).

Example 36: Synthesis of compound 105 {[8-(2-Ethyl-5,7-dimethyl-3H-imidazo[4,5-b]pyridin-3-yl methyl)-10,11-dihydro-5H-dibenz[b,f]azepin-2-yl] acetonitrile}

Compound 13 (2.04 g, 3.36 mmol) prepared in Example 6 was dissolved in dimethylformamide (17 mL), sodium cyanide (361 mg, 7.37 mmol) was added thereto and the mixture was stirred at 50°C for 10 hours. The reaction solution was cooled to room temperature, diluted with ethyl acetate, washed with a 2 mol/L aqueous solution of sodium hydroxide, water (two times) and a saturated saline solution successively, dried over anhydrous magnesium sulfate and concentrated. The residue was recrystallized from ethanol to give the objective compound 105 (751 mg, 1.78 mmol, yield: 53%).
APCI-MS: m/z 422 ([M + H]$^+$)
$^1$H NMR ($CDCl_3$) δ(ppm): 1.31 (t, J = 7. 5Hz, 3H), 2.60 (s, 3H), 2.63 (s, 3H), 2.79 (q, J = 7.5 Hz, 2H), 2.99 (m, 4H), 3.62 (s, 2H), 5.34 (s, 2H), 6.01 (s, 1H), 6.59-6.71 (m, 2H), 6.80-6.84 (m, 2H), 6.88 (s, 1H), 6.95-7.01 (m, 2H).

Example 37: Synthesis of compound 106 {2-(2-Ethyl-5,7-dimethyl-3H-imidazo[4,5-b]pyridin-3-ylmethyl)-8-(2H-tetrazol-5-ylmethyl)-10,11-dihydro-5H-dibenz[b,f]azepine}

Compound 105 prepared in Example 36 was used and, by the similar manner as in the latter part of Example 20, compound 106 was obtained in a yield of 76%
APCI-MS: m/z 465 ([M + H]$^+$)
$^1$H NMR (DMSO-$d_6$) δ(ppm): 1.22 (t, J = 7.6 Hz, 3H), 2.48-2.52 (s x 2, 6H, overlapped with DMSO), 2.78 (q, J = 7.6 Hz, 2H), 2.86 (m, 4H), 4.11 (s, 2H), 5.28 (s, 2H), 6.75-6.94 (m, 7H), 8.32 (br s, 1H).

Example 38: Synthesis of compound 107 {[8-(2-Ethyl-5,7-dimethyl-3H-imidazo[4,5-b]pyridin-3-ylmethyl)-10,11-dihydro-5H-dibenz[b,f]azepin-2-yl]acetic acid}

Compound 105 (247 mg, 0.586 mmol) prepared in Example 36 was suspended in ethanol (12 mL), sodium hydroxide (938 mg, 23.5 mmol) was added thereto and the mixture was stirred for 3 hours under a condition of heating to reflux. After confirming the progress of the reaction by a thin-layer chromatography, the reaction solution was cooled to room temperature and pH was adjusted to 5 with 1 mol/L hydrochloric acid. The crystals separated out therefrom were filtered, dried *in vacuo*, suspended in ethanol, stirred at 60°C for 0.5 hour and stirred at room temperature for 1 hour. The crystals separated out were filtered and dried in *vacuo* to give compound 107 (122 mg, 0.243 mmol, yield: 41%).
APCI-MS: m/z 441 ([M + H]$^+$)
$^1$H NMR (DMSO-$d_6$) δ(ppm): 1.23 (t, J = 7.5 Hz, 3H), 2.48-2.53 (s x 2, 6H, overlapped with DMSO), 2.78 (q, J = 7.5 Hz, 2H), 2.87 (br s, 4H), 3.38 (s, 2H), 5.38 (s, 2H), 6.74-6.94 (m, 7H), 8.27 (s, 1H), 12.15 (br s, 1H).

Example 39: Synthesis of compound 108 {Methyl [8-(2-ethyl-5,7-dimethyl-3H-imidazo[4,5-b]pyridin-3 -ylmethyl)-10,11-dihydro-5H-dibenz[b,f]azepin-2-ylmethylsulfanyl]acetate}

Compound 13 (1.04 g, 1.71 mmol) prepared in Example 6 was dissolved in chloroform (17 mL), then methyl mercaptoacetate (0.199 mL, 2.23 mmol) and 1,8-diazabicyclo[5.4.0]undec-7-ene (0.384 mL, 2.57 mmol) were added thereto and the mixture was stirred at 40°C for 7 hours. The reaction solution was concentrated, the residue was purified by a silica gel chromatography (eluting solvent: methanol/chloroform = 1/99) and a fraction containing the compound was concentrated. Ethanol was added to the residue and the resulting suspension was stirred at 60°C for 0.5 hour and then at room temperature for 1 hour. The crystals separated out were filtered to give compound 108 (628 mg, 1.25 mmol, yield: 73%).
APCI-MS: m/z 501 ([M + H]$^+$)

$^{1}$H NMR ($CDCl_3$)δ(ppm): 1.30 (t, J = 7.5 Hz, 3H), 2.60 (s, 3H), 2.63 (s, 3H), 2.80 (q, J = 7.5 Hz, 2H), 2.98 (m, 4H), 3.09 (s, 2H), 3.72 (s, 3H), 3.73 (s, 2H), 5.34 (s, 2H), 6.01 (s, 1H), 6.59-6.67 (m, 2H), 6.82 (m, 2H), 6.88 (s, 1H), 6.96-7.03 (m, 2H)

Example 40: Synthesis of compound 109 {[8-(2-Ethyl-5,7-dimethyl-3H-imidazo[4,5-b]pyridin-3-ylmethyl)-10,11-dihydro-5H-dibenz[b,f]azepin-2-ylmethylsulfanyl]acetic acid}

Compound 108 (350 mg, 0.699 mmol) prepared in Example 39 was used and, by the similar manner as in Example 12, compound 109 was obtained in a yield of 38%
APCI-MS: m/z 487 ($[M + H]^+$)
$^{1}$H NMR (DMSO-$d_6$) δ(ppm): 1.16 (t, J = 7.4 Hz, 3H), 2.42-2.50 (s x 2, 6H, overlapped with DMSO), 2.81 (q, J = 7.4 Hz, 2H), 2.88 (m, 6H), 3.49 (s, 2H), 5.22 (s, 2H), 6.67-6.89 (m, 7H), 8.18 (s, 1H).

Example 41: Synthesis of compound 110 {Ethyl 8-(2-ethyl-5,7-dimethyl-3H-imidazo[4,5-b]pyridin-3-yl-methyl)-10,11-dihydro-5H-dibenz[b,f]azepin-2-carboxylate}

Step 1:

1-(10,11-Dihydro-5H-dibenz[b,f]azepin-2-ylmethyl)-1-methylpiperidinium iodide (6.68 g, 15.4 mmol) was dissolved in dimethyl sulfoxide (110 mL), lithium acetate (5.07 g, 76.9 mmol) was added thereto and the mixture was stirred at 70°C for 2 days. The reaction solution was diluted with ethyl acetate, washed with water (for three times) and a saturated saline solution successively, dried over anhydrous magnesium sulfate and concentrated. The residue was purified by a silica gel chromatography (eluting solvent: ethyl acetate/hexane = 30/70) to give (10,11-dihydro-5H-dibenz[b,f]azepin-2-ylmethyl) acetate (2.85 g, 10.7 mmol, yielde: 69%).
APCI-MS: m/z 268 ($[M + H]^+$)
$^{1}$H NMR ($CDCl_3$) δ(ppm): 2.07 (s, 3H), 3.07 (br s, 4H), 4.99 (s, 2H), 6.05 (br s, 1H), 6.66-6.85 (m, 3H), 7.02-7.11 (m, 4H).

Step 2:

(10,11-Dihydro-5H-dibenz[b,f]azepin-2-ylmethyl) acetate (2.85 g, 10.7 mmol) prepared in the step 1 was suspended in methanol (110 mL), then a methanolic solution of sodium methoxide (38%, 1.14 mL, 5.36 mmol) was added thereto and the mixture was stirred at room temperature for 1 hour. The reaction solution was concentrated, a saturated saline solution and chloroform were added to the residue and extraction was conducted with chloroform for three times. The organic layers were combined, dried over anhydrous magnesium sulfate and concentrated. The residue was recrystallized from diisopropyl ether to give 10,11-dihydro-5H-dibenz[b,f]azepin-2-ylmethanol (1.73 g, 7.68 mmol, yield: 72%).
APCI-MS: m/z 226 ($[M + H]^+$)
$^{1}$H NMR ($CDCl_3$)δ(ppm): 1.49 (t, J = 5.8 Hz, 1H), 3.08 (br s, 4H), 4.57 (d, J = 5.8 Hz, 2H), 6.02 (br s, 1H), 6.66-6.87 (m, 3H), 7.02-7.11 (m, 4H).

Step 3:

10,11-Dihydro-5H-dibenz[b,f]azepin-2-ylmethanol (6.1 g, 70 mmol) prepared in the step 2 was dissolved in chloroform (77 mL), then manganese dioxide (4.55 g, 46.1 mmol) was added thereto and the mixture was stirred at room temperature for 8 hours. The reaction solution was filtered through Celite and the filtrate was concentrated. The residue was purifed by a silica gel chromatography (eluting solvent: ethyl acetate/hexane = 20/80) to give 10,11-dihydro-5H-dibenz[b,f]azepin-2-carboaldehyde (1.15 g, 5.15 mmol, yield: 67%).
APCI-MS: m/z 224 ($[M + H]^+$)
$^{1}$H NMR ($CDCl_3$) δ(ppm): 3.11 (m, 4H), 6.49 (br s, 1H), 6.87-6.91 (m, 3H), 7.07-7.17 (m, 2H), 7.55-7.62 (m, 2H), 9.88 (s, 1H).

Step 4:

10,11-Dihydro-5H-dibenz[b,f]azepin-2-carboaldehyde (665 mg, 2.98 mmol) prepared in the step 3 was dissolved in a mixed solvent of acetonitrile (18 mL) and water (18 mL), then dimethyl sulfoxide (2.1 mL, 30 mmol), sodium dihydrogen phosphate (1.43 g, 11. 9 mmol) and sodium chlorite (404 mg, 4.47 mmol) were added thereto and the mixture was stirred at 50°C for 4 hours. Ethyl acetate and water were added to the reaction solution and extraction

was conducted with ethyl acetate for two times. The organic layers were combined, washed with water, dried over anhydrous magnesium sulfate and concentrated. A mixed solvent (3:1) of ethyl acetate and hexane was added to the residue and the resulting suspension was stirred at 60°C for 0.5 hour and stirred at room temperature for 1 hour. The crystals separated out were filtered to give 10,11-dihydro-5H-dibenz[b,f]azepin-2-carboxylic acid (598 mg, 2.50 mmol, yield: 84%).

APCI-MS: m/z 240 ($[M + H]^+$)

$^1H$ NMR ($CDCl_3$) δ(ppm): 3.11 (m, 4H), 6.39 (br s, 1H), 6.77-6.80 (m, 3H), 7.06-7.16 (m, 2H), 7.79-7.84 (m, 2H).

Step 5:

10,11-Dihydro-5H-dibenz[b,f]azepin-2-carboxylic acid (426 mg, 1.78 mmol) prepared in the step 4 was dissolved in ethanol (8.9 mL), thionyl chloride (0.26 mL, 3.6 mmol) was added thereto and the mixture was stirred for 5 hours with heating to reflux. The reaction solution was concentrated, chloroform and a saturated aqueous solution of sodium bicarbonate were added thereto and extraction with chloroform was conducted for three times. The organic layers were combined, washed with a saturated saline solution, dried over anhydrous magnesium sulfate and concentrated. The residue was purified by a silica gel chromatography (eluting solvent: ethyl acetate/hexane = 10/90) to give ethyl 10,11-dihydro-5H-dibenz[b,f]azepin-2-carboxylate (383 mg, 1.43 mmol, yield: 81%).

APCI-MS: m/z 268 ($[M + H]^+$)

$^1H$ NMR ($CDCl_3$) δ(ppm): 1.37 (t, J = 7.0 Hz, 3H), 3.09 (m, 4H), 4.33 (q, J = 7.0 Hz, 2H), 6.34 (br s, 1H), 6.69-6.86 (m, 3H), 7.04-7.14 (m, 2H), 6.72-6.78 (m, 2H).

Step 6:

Ethyl

10,11-dihydro-5H-dibenz[b,f]azepin-2-carboxylate (443 mg, 1.66 mmol) prepared in the step 5 was dissolved in a mixed solvent of chloroform (8.3 mL) and acetic acid (8.3 mL), then piperidine (0.573 mL, 5.80 mmol) and paraformaldehyde (149 mg, 4.97 mmol) were added thereto and the mixture was heated at 60°C and stirred for 1.5 days. The reaction solution was concentrated, ethyl acetate and a saturated aqueous saline solution were added thereto and extraction with ethyl acetate was conducted. The organic layers were combined, washed with a saturated saline solution, dried over anhydrous sodium sulfate and concentrated. The residue was purified by a silica gel chromatography (eluting solvent: ethyl acetate/hexane/triethylamine = 70/25/5) and a fraction containing the objective substance was concentrated. The residue was subjected to trituration with diethyl ether to give ethyl 8-piperidinomethyl-10,11-dihydro-5H-dibenz[b,f]azepin-2-carboxylate (249 mg, 0.683 mmol, yield: 41%).

APCI-MS: m/z 365 ($[M + H]^+$)

$^1H$ NMR ($CDCl_3$) δ(ppm): 1.34-1.46 (m, 5H), 1.67 (m, 4H), 2.36 (br s, 4H), 3.08 (m, 4H), 3.38 (s, 2H), 4.33 (q, J = 7.1 Hz, 2H), 6.32 (s, 1H), 6.67-6.74 (m, 2H), 6.99-7.06 (m, 2H), 7.72-7.76 (m, 2H).

Step 7

Ethyl

8-piperidinomethyl-10,11-dihydro-5H-dibenz[b,f]azepine-2-carboxylate (231 mg, 0.634 mmol) prepared in the step 6 was dissolved in dichloromethane (3.2 mL), methyl iodide (59.2 μL, 0.951 mmol) was added thereto and the mixture was stirred at room temperature for one night. The reaction solution was concentrated in *vacuo* to give 1-(8-ethoxycarbonyl-10,11-dihydro-5H-dibenz[b,f]azepin-2-ylmethyl)-1-methylpiperidinium iodide (321 mg, 0.634 mmol, yield: 100%).

$^1H$ NMR ($CDCl_3$) δ(ppm): 1. 37 (t, J = 7.1 Hz, 3H), 1.75-1.95 (m, 6H), 2.96 (br s, 4H), 3.11 (s, 3H), 3.50 (m, 2H), 3.70 (m, 2H), 4.32 (q, J = 7.1 Hz, 2H), 4.90 (s, 2H), 7.14-7.35 (m, 4H), 7.49 (s, 1H), 7.71 (m, 2H).

Step 8:

2-Ethyl-5,7-dimethyl-3H-imidazo[4,5-b]pyridine (180 mg, 1.03 mmol) was dissolved in dimethylformamide (0.60 mL), sodium hydride (55%, 33.6 mg, 0.770 mmol) was added thereto with stirring by dividing into several times and the mixture was stirred at 50°C for 0.5 hour. The reaction solution was cooled down to room temperature, then a solution of 1-(8-ethoxycarbonyl-10,11-dihydro-5H-dibenz[b,f]azepin-2-ylmethyl)-1-methylpiperidinium iodide (130 mg, 0.256 mmol) prepared in the step 7 dissolved in dimethylformamide (1.2 mL) was added thereto and the mixture was stirred at room temperature for 1 hour. The reaction solution was diluted with ethyl acetate, washed with water, water

and a saturated aqueous saline solution successively, dried over anhydrous magnesium sulfate and concentrated. The residue was purified by a silica gel chromatography (eluting solvent: methanol/chloroform = 1/99) and a fraction containing the objective substance was concentrated. Diethyl ether was added to the residue and the mixture was stirred for 0.5 hours with heating to reflux and, after that, stirred at room temperature for 1 hour. The crystals separated out therefrom were filtered to give compound 110 (76.7 mg, 0.169 mmol, yield: 66%).

APCI-MS: m/z 455 ($[M + H]^+$)

$^1H$ NMR ($CDCl_3$) δ(ppm): 1.31 (t, J = 7.6 Hz, 3H), 1.36 (t, J = 7.1 Hz, 3H), 2.60 (s, 3H), 2.63 (s, 3H), 2.80 (q, J = 7.6 Hz, 2H), 2.97 (m, 2H), 3.40 (m, 2H), 4.32 (q, J = 7.1 Hz, 2H), 5.36 (s, 2H), 6.35 (s, 1H), 6.64-6.71 (m, 2H), 6.82-6.90 (m, 3H), 7.70-7.74 (m, 2H).

Example 42: Synthesis of compound 111 {8-(2-Ethyl-5,7-dimethyl-3H-imidazo[4,5-b]pyridin-3-ylmethyl)-10,11-dihydro-5H-dibenz[b,f]azepine-2-carboxylic acid}

The operation similar to that in Example 12 was conducted using compound 110 (900 mg, 1.98 mmol) prepared in Example 41 to give compound 111 in a yield of 97%.

APCI-MS: m/z 427 ($[M + H]^+$)

$^1H$ NMR (DMSO-$d_6$) δ(ppm): 1.24 (t, J = 7.5 Hz, 3H), 2.51-2.54 (s x 2, 6H, overlapped with DMSO), 2.82-2.99 (m, 6H), 5.37 (s, 2H), 6.84-7.03 (m, 5H), 7.58 (m, 2H), 8.87 (br s, 1H), 12.25 (br s, 1H).

Example 43: Synthesis of compound 112 {[8-(2-Ethyl-5,7-dimethyl-3H-imidazo[4,5-b]pyridin-3-ylmethyl)-10,11-dihydro-5H-dibenz[b,f]azepin-2-yl] (4-methylpiperazin-1-yl)methanone}

Compound 111 (100 mg, 0.234 mmol) prepared in Example 42 was dissolved in a mixed solvent of dimethylformamide (2.3 mL) and tetrahydrofuran (4.6 mL), then 4-methylpiperazine (39 μL, 0.352 mmol), 1-ethyl-3-(3-dimethylaminopropyl)carbodiimide monohydrochloride (89.7 mg, 0.468 mmol) and 1-hydroxybenzotriazole (35.8 mg, 0.234 mmol) were added thereto and the mixture was stirred at room temperature for 8 hours. After confirming the progress of the reaction by a thin-layer chromatography, the reaction solution was concentrated. The residue was dissolved in chloroform and the resulting solution was washed with water (for two times), a saturated sodium bicarbonate solution and a saturated saline solution successively, dried over anhydrous magnesium sulfate and concentrated. Diethyl ether was added to the residue, the resulting suspension was stirred at room temperature for 1 hour and a solid was filtered to give compound 112 (47.7 mg, 0.0938 mmol, yield: 40%).

APCI-MS: m/z 509 ($[M + H]^+$)

$^1H$ NMR ($CDCl_3$)δ(ppm): 1.31 (t, J = 7.5 Hz, 3H), 2.33 (s, 3H), 2.43 (br s, 4H), 2.60 (s, 3H), 2.63 (s, 3H), 2.80 (q, J = 7.5 Hz, 2H), 2.99 (m, 4H), 3.66 (br s, 4H), 5.35 (s, 2H), 6.18 (s, 1H), 6.62-6.69 (m, 2H), 6.83 (m, 2H), 6.85 (s, 1H), 7.10-7.15 (m, 2H).

Example 44: Synthesis of compound 113 {[8-(2-Ethyl-5,7-dimethyl-3H-imidazo[4,5-b]pyridin-3-ylmethyl)-10,11-dihydro-5H-dibenz[b,f]azepin-2-yl] (pyrrolidine-1-yl)methanone}

The operation similar to that in Example 43 was conducted using pyrrolidine instead of 4-methylpiperazine to give compound 113 in a yield of 90%.

APCI-MS: m/z 480 ($[M + H]^+$)

$^1H$ NMR ($CDCl_3$) δ(ppm): 1.31 (t, J = 7.5 Hz, 3H), 1.88 (br s, 4H), 2.60 (s, 3H), 2.63 (s, 3H), 2.80 (q, J = 7.5 Hz, 2H), 2.99 (m, 4H), 3.56 (m, 4H), 5.35 (s, 2H), 6.19 (s, 1H), 6.62-6.69 (m, 2H), 6.81-6.86 (m, 2H), 6.89 (s, 1H), 7.24-7.29 (m, 2H).

Example 45: Synthesis of compound 114 {[8-(2-Ethyl-5,7-dimethyl-3H-imidazo[4,5-b]pyridin-3-ylmethyl)-10,11-dihydro-5H-dibenz[b,f]azepin-2-yl] (4-hydroxypiperidino)methanone}

The operation similar to that in Example 43 was conducted using 4-piperidinol instead of 4-methylpiperazine to give compound 114 in a yield of 62%.

APCI-MS: m/z 510 ($[M + H]^+$)

$^1H$ NMR ($CDCl_3$)δ(ppm): 1.31 (t, J = 7.0 Hz, 3H), 1.48-1.58 (m, 2H), 1.86-1.97 (m, 2H), 2.60 (s, 3H), 2.63 (s, 2H), 2.80 (q, J = 7.0 Hz, 2H), 2.99 (m, 4H), 3.22-3.33 (m, 2H), 3.91-4.00 (m, 3H), 5.36 (s, 2H), 6.21 (s, 1H), 6.62-6.70 (m, 2H), 6.81-6.85 (m, 2H), 6.89 (s, 1H), 7.08-7.14 (m, 2H).

Example 46: Synthesis of compound 115 {[8-(2-Ethyl-5,7-dimethyl-3H-imidazo[4,5-b]pyridin-3-ylmethyl)-10,11-dihydro-5H-dibenz[b,f]azepin-2-carboxylic acid (2-hydroxyethyl)amide}

The operation similar to that in Example 43 was conducted using ethanolamine instead of 4-methylpiperazine to give compound 115 in a yield of 82%.

APCI-MS: m/z 470 ($[M + H]^+$)

$^1H$ NMR ($CDCl_3$) δ(ppm): 1.31 (t, J = 7.5 Hz, 3H), 1.71 (br s, 1H), 2.60 (s, 3H), 2.63 (s, 3H), 2.79 (q, J = 7.5 Hz, 2H), 2.97 (.m, 4H), 3.59 (m, 2H), 3.81 (t, J = 9.6 Hz, 2H), 5.35 (s, 2H), 6.41 (s, 1H), 6.54 (t, J = 5.6 Hz, 1H), 6.63-6.71 (m, 2H), 6.80-6.84 (m, 2H), 6.99 (s, 1H), 7.44-7.48 (m, 2H).

Example 47: Synthesis of compound 116 {[8-(2-Ethyl-5,7-dimethyl-3H-imidazo[4,5-b]pyridin-3-ylmethyl)-10,11-dihydro-5H-dibenz[b,f]azepine-2-carboxylic acid[2-(pyrolidin-1-yl)ethyl]amide}

The operation similar to that in Example 43 was conducted using 2-(pyrrolidine-1-yl)ethylamine instead of 4-methylpiperazine to give compound 116 in a yield of 92%.

APCI-MS: m/z 523 ($[M + H]^+$)

$^1H$ NMR ($CDCl_3$) δ(ppm): 1.31 (t, J = 7.6 Hz, 3H), 1.78 (m, 4H), 1.57 (m, 4H), 2.60 (s, 3H), 2.63 (s, 3H), 2.70 (t, J = 5.9 Hz, 2H), 2.79 (q, J = 7.6 Hz, 2H), 2.97 (m, 2H), 3.04 (m, 2H), 3.53 (q, J = 5.7 Hz, 2H), 5.35 (s, 2H), 6.30 (s, 1H), 6.63-6.72 (m, 3H), 6.83 (m, 2H), 6.89 (s, 1H), 7.45-7.52 (m, 2H).

Example 48: Synthesis of compound 117 {[8-(2-Ethyl-5,7-dimethyl-3H-imidazo[4,5-b]pyridin-3-ylmethyl)-10,11-dihydro-5H-dibenz[b,f]azepin-2-yl] (morpholino)methanone}

The operation similar to that in Example 43 was conducted using morpholine instead of 4-methylpiperazine to give compound 117 in a yield of 98%.

APCI-MS: m/z 496 ($[M + H]^+$)

$^1H$ NMR ($CDCl_3$) δ(ppm): 1.31 (t, J = 7.5 Hz, 3H), 2.60 (s, 3H), 2.63 (s, 3H), 2.80 (q J = 7.5 Hz, 2H), 2.99 (m, 4H), 3.66 (m, 8H), .5.35 (s, 2H), 6.22 (s, 1H), 6.62-6.71 (m, 2H), 6.81-6.86 (m, 2H), 6.89 (s, 1H), 7.10-7.16 (m, 2H).

Example 49: Synthesis of compound 118 {8-(2-Ethyl-5,7-dimethyl-3H-imidazo[4,5-b]pyridin-3-ylmethyl)-10,11-dihydro-5H-dibenz[b,f]azepine-2-carboxylic acid bis(2-hydroxyethyl)amide}

The operation similar to that in Example 43 was conducted using 2-(2-hydroxyethylamino)ethanol instead of 4-methylpiperazine to give compound 118 in a yield of 38%.

APCI-MS: m/z 514 ($[M + H]^+$)

$^1H$ NMR ($CDCl_3$) δ(ppm): 1.21 (t, J = 7.5 Hz, 3H), 2.60 (s, 3H), 2.63 (s, 3H), 2.80 (q, J = 7.5 Hz, 2H), 2.98 (m, 4H), 3.23 (br s, 2H), 3.63 (br s, 4H), 3.87 (br s, 4H), 5.35 (s, 2H), 6.20 (s, 1H), 6.62-6.69 (m, 2H), 6.83 (m, 2H), 6.89 (s, 1H), 7.24-7.29 (m, 2H).

Example 50: Synthesis of compound 119 {8-(2-Ethyl-5,7-dimethyl-3H-imidazo[4,5-b]pyridin-3-ylmethyl)-10,11-dihydro-5H-dibenz[b,f]azepine-2-carboxylic acid amide}

The operation similar to that in Example 43 was conducted using ammonia instead of 4-methylpiperazine to give compound 119 in a yield of 57%.

APCI-MS: m/z 426 ($[M + H]^+$)

$^1H$ NMR ($CDCl_3$) δ(ppm): 1. 23 (t, J = 7. 4 Hz, 3H), 2.48-2.52 (s x 2, 6H, overlapped with DMSO), 2.78 (q, J = 7.4 Hz, 2H), 2.92 (br q, J = 7.3 Hz, 4H), 5.31 (s, 2H), 6.78-7.00 (m, 6H), 7.52-7.65 (m, 3H), 8.68 (s, 1H).

Example 51: Synthesis of compound 120 {2-(2-Ethyl-5,7-dimethyl-3H-imidazo[4,5-b]pyridin-3-ylmethyl)-5-methyl-8-(pyrrolidin-1-ylmethyl)-10,11-dihydro -5H-dibenz[b,f]azepine}

Compound 3 prepared in Example 3 (400 mg, 0.876 mmol) was dissolved in acetic acid (8.8 mL), then paraformaldehyde (0.47 g, 16 mmol) and sodium cyanoborohydride (2.2 g, 10 mmol) were added thereto and the mixture was stirred at room temperature for 5 hours. Chloroform and a saturated sodium bicarbonate solution were added to the reaction solution and the aqueous layer was extracted with chloroform twice. The organic layer was washed with a saturated saline solution, dried over anhydrous magnesium sulfate and concentrated. The residue was purified by an NH-silica gel chromatography (eluting solvent: chloroform/hexane = 50/50) to give compound 120 (342 mg, 0.713 mmol, yield: 81%).

APCI-MS: m/z 480 ($[M + H]^+$)

$^1H$ NMR ($CDCl_3$) δ(ppm): 1.31 (t, J = 7.5 Hz, 3H), 1.76 (m, 4H), 2.47 (m, 4H), 2.58 (s, 3H), 2.62 (s, 3H), 2.78 (q, J = 7.5 Hz, 2H), 3.06 (m, 4H), 3.29 (s, 3H), 3.50 (s, 2H), 5.35 (s, 2H), 6.83-6.98 (m, 5H), 7.02-7.08 (m, 2H).

Example 52: Synthesis of compound 121 {1-[8-(2-Ethyl-5,7-dimethyl-3H-imidazo[4,5-b]pyridin-3-ylmethyl)-5-methyl-10,11-dihydro-5H-dibenz[b,f]azepin-2-ylmethyl]piperidine-4-carboxylic acid}

Step 1:

Compound 16 (1.20 g, 2.18 mmol) prepared in Example 9 was dissolved in acetic acid (10 mL), then paraformaldehyde (0.73 g, 21.8 mmol) and sodium cyanoborohydride (0.58 g, 8.70 mmol) were added thereto and the mixture was stirred at room temperature for 15 hours. Ethyl acetate and a 1 mol/L aqueous solution of sodium hydroxide were added to the reaction solution and the aqueous layer was extracted with ethyl acetate. The organic layer was dried over anhydrous magnesium sulfate and the solvent was evaporated *in vacuo*. The residue was purified by an NH-silica gel chromatography (eluting solvent: a mixed solvent of hexane-ethyl acetate) to give ethyl 1-[8-(2-ethyl-5,7-dimethyl-3H-imidazo[4,5-b]pyridin-3-ylmethyl)-5-methyl-10,11-dihydro-5H-dibenz[b,f]azepin-2-ylmethyl]piperidine-4-carboxylate (1.25 g, 2.18 mmol, yield: 100%).

APCI-MS: m/z 566 ($[M + H]^+$)

$^1H$ NMR ($CDCl_3$) δ(ppm): 1.23 (t, J = 7.4 Hz, 3H), 1.31 (t, J = 7.6 Hz, 3H), 1.6-2.0 (m, 6H), 2.23 (m, 1H), 2.58 (s, 3H), 2.62 (s, 3H), 2.73 (q, J = 7.4Hz, 2H), 2.75-2.9 (m, 2H), 3.0-3.15 (m, 4H), 3.28 (s, 3H), 3.36 (s, 2H), 4.10 (q, J = 7.6 Hz, 2H), 5.34 (s, 2H), 6.8-7.1 (m, 7H).

Step 2:

The operation similar to that in Example 12 was conducted using ethyl 1-[8-(2-ethyl-5,7-dimethyl-3H-imidazo [4,5-b]pyridin-3-ylmethyl)-5-methyl-10,11-dihydro-5H-dibenz[b,f]azepin-2-ylmethyl]piperidine-4-carboxylate prepared in the step 1 to give compound 121 in a yield of 42%.

APCI-MS: m/z 538 ($[M + H]^+$)

$^1H$ NMR (DMSO-$d_6$) δ(ppm): 1.23 (t, J = 7.4 Hz, 3H), 1.5-1.8 (m, 2H), 1.8-2.0 (m, 2H), 2.2-2.4 (m, 2H), 2.49 (s, 3H), 2.50 (s, 3H), 2.78 (q, J = 7.4 Hz, 2H), 2.8-3.05 (m, 8H), 3.22 (s, 2H), 3.5-3.9 (m, 2H), 5.34 (s, 2H), 6.85 (dd, J = 2.0, 8.4 Hz, 1H), 6.93 (s, 1H), 6.94 (d, J = 2.0 Hz, 1H), 7.0-7.2 (m, 4H).

Example 53: Synthesis of compound 122 {2-(2-Ethyl-5,7-dimethyl-3H-imidazo[4,5-b]pyridin-3-ylmethyl)-5-methyl-8-[4-(2H-tetrazol-5-yl) piperidinomethyl]-10,11-dihydro-5H-dibenz[b,f]azepine monohydrochloride}

The operation similar to that in the step 1 of Example 52 was conducted using 1-[8-(2-ethyl-5,7-dimethyl-3H-imidazo[4,5-b]pyridin-3-yl methyl)-10,11-dihydro-5H-dibenz[b,f]azepin-2-ylmethyl]-piperidine-4-carbonitrile prepared in Example 20 to prepare 1-[8-(2-ethyl-5,7-dimethyl-3H-imidazo[4,5-b]pyridin-3-ylmethyl)-5-methyl-10,11-dihydro-5H-dibenz[b,f]azepin-2-ylmethyl]piperidine-4-carbonitrile in a yield of 92%. The operation similar to that in the latter part of Example 20 was conducted using the above compound to prepare 2-(2-ethyl-5,7-dimethyl-3H-imidazo[4,5-b]pyridin-3-ylmethyl)-5-methyl-8-[4-(2H-tetrazol-5-yl)piperidinomethyl]-10,11-dihydro-5H-dibenz[b,f]azepine in a yield of 10%. This was dissolved in chloroform, a solution of 4 mol/L of hydrogen chloride in ethyl acetate was added thereto and the solid separated out therefrom were filtered to prepare compound 122.

APCI-MS: m/z 562 ($[M + H]^+$)

$^1H$ NMR (DMSO-$d_6$) δ(ppm): 1.28 (t, J = 7.6 Hz, 3H), 2.0-2.5 (m, 4H), 2.58 (s, 3H), 2.63 (s, 3H), 2.9-3.2 (m, 8H), 3.2-3.3 (m, 4H), 3.4-3.6 (m, 2H), 4.17 (s, 2H), 5.56 (s, 2H), 7.0-7.2 (m, 4H), 7.2-7.4 (m, 3H), 10.79 (s, 1H).

Example 54: Synthesis of compound 123 {1-[8-(2-Ethyl-5,7-dimethyl-3H-imidazo[4,5-b]pyridin-3-ylmethyl)-5-methyl-10,11-dihydro-5H-dibenz[b,f]azepin-2-ylmethyl]piperidin-4-ylmethanol}

Ethyl

1-[8-(2-ethyl-5,7-dimethyl-3H-imidazo[4,5-b]pyridin-3-ylmethyl)-5-methyl-10,11-dihydro-5H-dibenz[b,f] azepin-2-ylmethyl]piperidine-4-carboxylate (0.61 g, 1.08 mmol) prepared in the step 1 of Example 52 was dissolved in dichloromethane (10 mL), cooled at -78°C and stirred. A 1 mol/L solution of diisopropylaluminum hydride in toluene (3.20 mL, 3.20 mmol) was added to the reaction solution at the same temperature and the mixture was stirred for 3 hours at the same temperature and, after that, at room temperature for 10 minutes. A saturated aqueous solution of Rochelle salt and ethyl acetate were added to the reaction solution and the mixture was stirred for 30 minutes. The

aqueous layer was extracted with ethyl acetate, the organic layer was dried over anhydrous magnesium sulfate and the solvent was evaporated *in vacuo*. The residue was subjected to recrystallization from ethyl acetate to prepare compound 123 (0.26 g, 0.50 mmol, yield: 46%).

APCI-MS: m/z 524 ($[M + H]^+$)

$^1H$ NMR (DMSO-$d_6$) δ(ppm): 1.0-1.15 (m, 2H), 1.23 (t, J = 7.5 Hz, 3H), 1.25-1.3 (m, 1H), 1.5-1.65 (m, 2H), 1.7-1.9 (m, 2H), 2.49 (s, 3H), 2.50 (s, 3H), 2.75 (q, J = 7.5 Hz, 2H), 2.95-3.05 (m, 4H), 3.15-3.25 (m, 5H), 3.25-3.50 (m, 4H), 5.32 (s, 2H), 6.81 (dd, J = 2.0, 8.5 Hz, 1H), 6.90-7.05 (m, 6H).

Example 55: Synthesis of compound 124 [2-(2-Ethyl-5,7-dimethyl-3H-imidazo[4,5-b]pyridin-3-ylmethyl)-5-methyl-8-(2H-tetrazol-5-yl)-10,11-dihydro-5H-dibenz[b,f]azepine]

The operation similar to that in the step 1 of Example 52 was conducted using compound 103 prepared in Example 34 to prepare 8-(2-ethyl-5,7-dimethyl-3H-imidazo[4,5-b]pyridin-3-ylmethyl)-5-methyl-10,11-dihydro-5H-dibenz[b,f]azepine-2-carbonitrile in a yield of 83%.

The operation similar to that in the latter part of Example 20 was conducted using the above compound to prepare compound 124 in a yield of 20%.

APCI-MS: m/z 465 ($[M + H]^+$)

$^1H$ NMR (DMSO-$d_6$) δ(ppm): 1.24 (t, J = 7.7 Hz, 3H), 2.50 (s, 3H), 2.51 (s, 3H), 2.80 (q, J = 7.7 Hz, 2H), 3.0-3.1 (m, 2H), 3.3-3.35 (m, 2H), 3.40 (s, 3H), 5.38 (s, 2H), 6.90 (dd, J = 2.2, 8.4 Hz, 1H), 6.95 (s, 1H), 7.02 (d, J = 2.2 Hz, 1H), 7.10 (d, J = 8.4 Hz, 1H), 7.24 (d, J = 8.4 Hz, 2H), 7.75 (d, J = 2.2 Hz, 1H), 7.79 (dd, J = 2.2, 8.4 Hz, 1H).

Referential Example 1: Synthesis of compound 91 {1,4-Bis[4-(3-chlorobenzylamino)-6-cyclopropylcarbonyl-7,8 -dihydro-5H-pyrido[4,3-d]pyrimidin-2-yl]piperazine}

Compound 91 was synthesized according to the following step 1 to step 8.

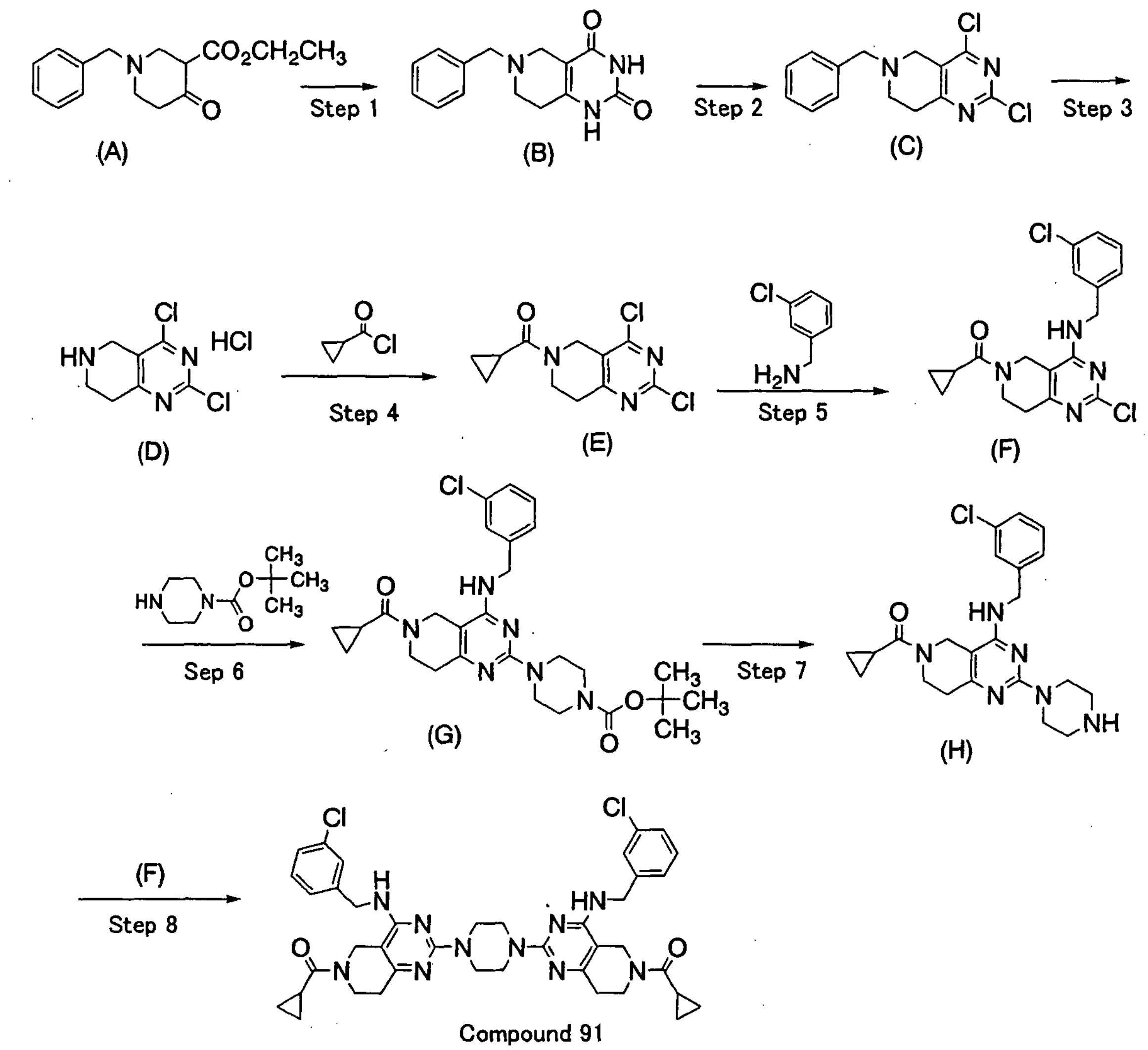

(A) (B) (C) (D) (E) (F) (G) (H)

Compound 91

Step 1:

A commercially available compound (A) (100 g, 0.335 mol) was dissolved in ethanol (1,500 mL), then urea (100 g, 1.67 mol) and sodium methoxide (227 g, 1.18 mol) were added thereto and the mixture was made to react for 24 hours under the condition of heating to reflux. Progress of the reaction was confirmed by a thin-layer chromatography and, after cooled, crystals separated out therefrom were filtered. The crystals were suspended in water, hydrochloric acid (6 mol/L) was added thereto and pH was adjusted to 6.0. Stirring was further conducted at room temperature for 1 hour and the crystals separated out therefrom were filtered and dried *in vacuo* to prepare compound (B) (60 g, yield: 70%).

Step 2:

Phosphorus oxychloride (300 mL) was added to compound (B) (30.0 g, 0.116 mol) prepared in the step 1 and the mixture was stirred under a heating condition for 5 hours. After confirming the progress of the reaction by a thin-layer chromatography, an excessive phosphorus oxychloride was evaporated *in vacuo*. After that, 2-propanol (300 mL) was added to the residue and a suspension containing the crystals separated out therefrom was stirred under the condition of heating to reflux for 1 hour and further stirred at room temperature for 1 hour. The crystals separated out were filtered and dried *in vacuo* to prepare compound (C) (33 g, yield: 85%).

Step 3:

Compound (C) (35.0 g, 0.106 mol) prepared in the step 2 was dissolved in 1,2-dichloroethane (850 mL), then triethylamine (14.9 mL, 0.107 mol) and 1-chloroethyl chloroformate (34.1 mL, 0.316 mol) were added thereto and the mixture was stirred under a condition of heating to reflux for 5 hours. After confirming the progress of the reaction by a thin-layer chromatography, the reaction mixture was cooled, washed with water and a saturated aqueous saline solution successively and dried over anhydrous magnesium sulfate. The resulting solution was concentrated and the residue was purified by a column chromatography (silica gel, n-hexane:ethyl acetate = 3:1). The product was dissolved in methanol (850 mL) and stirred under a condition of heating to reflux for 1 hour. After confirming the progress of the reaction by a thin-layer chromatography, it was concentrated to dryness to prepare compound (D) (23.5 g, yield: 95%).

Step 4:

Compound (D) (11.8 g, 49.1 mmol) prepared in Step 3 was dissolved in dichloromethane (300 mL), then cyclopropanecarbonyl chloride (5.4 mL, 1.2 equivalents) and triethylamine (20.4 mL, 3.0 equivalents) were added thereto and the mixture was stirred at room temperature for 1 hour. The resulting reaction solution was washed with water and a saturated sodium bicarbonate solution and dried over magnesium sulfate. After evaporating the solvent, diisopropyl ether was added to the residue and the suspension was stirred for not shorter than 1 hour. After that, the crystals separated out therefrom were filtered and dried *in vacuo* to prepare compound (E) (12.5 g, yield: 94%).

Step 5:

Compound (E) (12.5 g, 45.9 mmol) prepared in the step 4 was dissolved in tetrahydrofuran (400 mL), then triethylamine (19.2 ml, 3 equivalents) and 3-chlorobenzylamine (11.2 mL, 2 equivalents) were added and the mixture was stirred at 40°C for 20 hours. The salt separated out therefrom was removed by filtration and the solvent was evaporated. The residue was purified by a chromatography (chloroform:methanol = 100:1 → 40:1) and a mixed solvent of hexane/ethyl acetate (3:1) was added to a concentrated residue of a fraction containing the objective substance so that the crystals were separated out. The suspension containing the crystals was stirred for 1 hour and the crystals separated out therefrom were filtered and dried *in vacuo* to prepare compound (F) (11.9 g, yield: 69%).

Step 6:

Compound (F) (5.0 g, 13.3 mmol) prepared in the step 5 was dissolved in dioxane (100 mL), then tert-butyl 1-piperazinecarboxylate (4.9 g, 2 equivalents) and sodium carbonate (14.0 g, 10 equivalents) were added thereto and the mixture was stirred at 90°C for 3 days. The resulting reaction solution was filtered to remove sodium carbonate, extraction was conducted by addition of water and chloroform to the filtrate and the organic layer was dried over magnesium sulfate. After evaporation of the solvent, a mixed solvent of hexane/ethyl acetate (3:1) was added and the suspension was stirred for 1 hour. After that, the crystals separated out therefrom were filtered and dried in *vacuo* to prepare compound (G) (6.4 g, yield: 92%).

Step 7

A 20% solution of trifluoroacetic acid in dichloromethane (50 mL) was added to compound (G) (6.3 g, 12.0 mmol) prepared in the step 6 and the mixture was stirred at room temperature for 1 hour. After evaporating the solvent from the reaction solution, diisopropyl ether was added to the residue and the resulting suspension was stirred for 1 hour. After that, the crystals separated out therefrom were filtered and dried *in vacuo* to prepare compound (H) (4.9 g, yield: 97%).

Step 8:

Compound (H) (3.8 g, 8.90 mmol) prepared in the step 7 and compound (F) (4.5 g, 1.05 equivalent) prepared in the step 5 were dissolved in dioxane (100 mL), sodium carbonate (10.6 g, 10 equivalents) was added and the mixture was stirred at 90°C for 1 week. The resulting reaction solution was filtered to remove sodium carbonate, water was added to the filtrate and extraction was conducted with chloroform. The organic layer was dried over magnesium sulfate, the solvent was evaporated and the residue was purified by a column chromatography (ethyl acetate: triethylamine = 10:1). A mixed solvent of hexane/ethyl acetate (3:1) was added to a concentrated residue of a fraction containing the obejective substance and the resulting suspension was stirred for 1 hour. After that, the crystals separated out therefrom were filtered and dried *in vacuo* to prepare compound 91 (1.0 g, yield: 23%).

APCI-MS: m/z 767 ([M + H]$^+$)

$^1$H NMR ($CDCl_3$) δ(ppm): 0.7-0.9 (m, 4H), 1.0-1.1 (m, 4H), 1.7-1.9 (m, 2H), 2.6-2.8 (m, 4H), 3.75 (s, 8H), 3.8-4.0 (m, 4H), 4.3-4. 4 (m, 4H), 4.6-4.7 (m, 4H), 4.8-4.9 (m, 2H), 7.1-7.3 (m, 8H).

Referential Example 1a: Synthesis of N-methyl-N-(2-trityl-2H-tetrazol-5-ylmethyl)amine

2-Trityl-2H-tetrazol-5-ylmethanol (2.00 g, 5.84 mmol), N-methyl-2-nitrobenzenesulfonamide (1.64 g, 7.59 mmol) and triphenylphosphine (1.53 g, 5.84 mmol) were dissolved in a mixed solvent of tetrahydrofuran (30 mL) and toluene (20 mL), then a solution of diethyl azodicarboxylate in toluene (40%, 2.65 mL, 5.84 mmol) was added thereto and the mixture was stirred at room temperature for throughout a night. After the mixture was passed through a silica gel column (eluting solvent: ethyl acetate/hexane = 40/60) to remove original point components, acetone (5 mL) and acetonitrile (25 mL) were added to the residue prepared by concentrating *in vacuo*.

Mercaptoacetic acid (0.73 mL, 11 mmol) and 1,8-diazabicyclo[5,4,0]undec-7-ene (3.1 mL, 21 mmol) were added to the resulting suspension and the mixture was stirred at 60°C for 7 hours. The reaction solution was concentrated and the residue was dissolved in ethyl acetate. The resulting solution was washed with a saturated aqueous solution of sodium bicarbonate and a saturated saline solution successively, dried over anhydrous magnesium sulfate and concentrated. The residue was purified by a silica gel chromatography (eluting solvent: triethylamine/ethyl acetate = 1/99) to prepare N-methyl-N-(2-trityl-2H-tetrazol-5-yl)methylamine (396 mg, 1.11 mmol, yield: 19.0%).

$^1$H NMR ($CDCl_3$) δ(ppm): 2.45 (s, 3H), 4.07 (s, 2H), 7.07-7.36 (m, 15H).

Referential Example 2: Construction of a host-vector system

(1) Construction of Gal4 -ER expression plasmid pGERbsrR2

pSV2bsr (manufactured by Kaken Seiyaku) was cleaved with PvuII and EcoRI and subjected to a Klenow treatment to prepare a PvuII (blunt end)-EcoRI (blunt end) fragment of 2.6 kb.

ERαAF2 in pM containing the Gal4-ER chimeric gene [*Cell,* 54, 199 (1988); *Proc. Natl. Acad. Sci., USA,* 90, 1657 (1993)] (apportioned from Dr. Shigeaki Kato, University of Tokyo) was cleaved with AatII and NdeI and subjected to a Klenow treatment to prepare an AatII (blunt end)-NdeI (blunt end) fragment.

The above mentioned PvuII (blunt end)-EcoRI (blunt end) fragment derived from pSV2bsr and the AatII (blunt end)-NdeI (blunt end) fragment derived from ERαAF2 in pM were ligated to construct a plasmid pGERbsrR2. pGERbsrR2 is able to express a chimeric protein (Gal4-ER) consisting of a DNA binding domain of transcription factor Gal4p derived from a yeast (*Saccharomyces cerevisiae*) and a ligand binding domain of estrogen receptor.

(2) Construction of an inducible expression plasmid of firefly luciferase

pcDNA3 (Invitrogen) was cleaved with XhoI and subjected to a Klenow treatment to prepare a XhoI (blunt end) fragment. The fragment was ligated to construct pcDNA3 where cleaved sites by XhoI disappeared. pcDNA3 where the cleaved site with XhoI disappeared was cleaved with KpnI and subjected to a Klenow treatment to prepare a KpnI (blunt end) fragment. The fragment was ligated to construct pcDNA3 where cleaved sites with XhoI and KpnI disappeared. The plasmid was cleaved with BglII and subjected to a Klenow treatment to a prepare BglII (blunt end) fragment.

pAMoERC3Sc (Japanese Published Unexamined Patent Application No. 336,963/1993) was cleaved with XhoI and NsiI and subjected to a Klenow treatment to obtain a XhoI (blunt end)-NsiI (blunt end) fragment of 2.2 kb having an oriP sequence.

The above-mentioned BglII (blunt end) fragment derived from pcDNA3 where XhoI-cleaved site and KpnI-cleaved site disappeared and the XhoI (blunt end)-NsiI (blunt end) fragment derived from pAMoERC3Sc were ligated to construct a plasmid pcDNA3-oriP. pcDNA3-oriP was cleaved with XhoI and HindIII to obtain a XhoI-HindIII fragment.

pSE0luc2 (WO 98/14474) was cleaved with XhoI and NcoI and subjected to a Klenow treatment to obtain a XhoI (blunt end)-NcoI (blunt end) fragment comprising the ampicillin-resistant gene. The fragments were ligated to construct a plasmid pASd1-luc1. After pASd1-luc1 was cleaved with XhoI and HindIII, a XhoI-HindIII fragment of 0.11 kb were obtained.

The above-mentioned XhoI-HindIII fragment derived from pcDNA3-oriP and the XhoI-HindIII fragment derived from pASd1-luc1 were ligated to construct a plasmid pcDNA3-oriP-Sd1. pcDNA3-oriP-Sd1 was cleaved with XhoI and KpnI to obtain a XhoI-KpnI fragment.

Four kinds of DNAs having nucleotide sequences represented by SEQ ID NOS: 1, 2, 3 and 4, respectively, were synthesized by DNA synthetizer. When the synthetic DNAs were mixed and annealed, a double-stranded DNA having polyadenylation signal was constructed. Each of the synthetic DNAs was phosphorylated using T4 polynucleotide kinase, mixed and annealed to give a double-stranded DNA.

When the double-stranded DNA was ligated to the XhoI-KpnI fragment derived from pcDNA3-oriP-Sd1, a plasmid pcDNA3-oriP-Sd1-pA was constructed. pcDNA3-oriP-Sd1-pA was cleaved with XhoI and subjected to a Klenow treatment to obtain a XhoI (blunt end) fragment.

pFR-luc (manufactured by Stratagene) was cleaved with HindIII and BamHI and subjected to a Klenow treatment to obtain a HindIII (blunt end)-BamHI (blunt end) fragment of 0.14 kb.

The above-mentioned XhoI (blunt end) fragment derived from pcDNA3-oriP-Sd1-pA and the HindIII-BamHI fragment derived from pFR-luc were ligated to obtain a plasmid pAGalSd1. The pAGalSd1 comprises a promoter having a sequence where Gal4p-responsive elements (UASG) are repeated for 5 times. pAGalSd1 was cleaved with EcoRI and subjected to a Klenow treatment to obtain a EcoRI (blunt end) fragment.

pSE0luc2 (WO 98/14474) was cleaved with HindIII and SacI and subjected to a Klenow treatment to prepare a HindIII (blunt end)-SacI (blunt end) fragment of 1.7 kb comprising the firefly luciferase gene.

The above-mentioned HindIII (blunt end)-SacI (blunt end) fragments derived from pSE0luc2 and the EcoRI (blunt end) derived from pAGalSd1 were ligated to construct a plasmid pAGalSd1-luc.

Among the two HindIII sites existing in pAGalSd1-luc, only a HindIII site far from the firefly luciferase gene was made disappeared by a Klenow treatment to construct pAGalSd4-luc.

pAGalSd4-luc was cleaved with Asp718 and subjected to a partial digestion with StuI to obtain an Asp718-StuI fragment of 9.5 kb derived from pAGalSd4-luc. The DNA fragments were subjected to a Klenow treatment and self-ligated to construct a plasmid pAGal9-luc.

(3) Construction of inducible expression vectors pAGal9-d and pAGal9-nd

Expression plasmid pAGal9-luc having oriP of Epstein-Barr virus was cleaved with HindIII and SacI to prepare a HindIII-SacI fragment of 6.9 kb containing oriP.

pAMo-d (Japanese Published Unexamined Patent Application No. 211,885/2001) was cleaved with HindIII and SacI to prepare a HindIII-SacI fragment comprising the tetracycline-resistant gene ($Tc^R$).

The above-mentioned HindIII-SacI fragment derived from pAGal9-luc and the HindIII-SacI fragment derived from pAMo-d were ligated to construct a plasmid pAGal9-d where the firefly luciferase gene in pAGal9-luc was substituted with a stuffer sequence of pAMo-d. pAGal9-luc was cleaved with HindIII and SacI to prepare a HindIII-SacI fragment of 6.9 kb.

pAMo-nd (Japanese Published Unexamined Patent Application No. 211,885/2001) was cleaved with HindIII and SacI to prepare a HindIII-SacI fragment comprising the tetracycline-resistant gene.

The above-mentioned HindIII-SacI fragment derived from pAGal9-luc and the HindIII-SacI fragment derived from pAMo-nd were ligated to construct a plasmid pAGal9-nd where the firefly luciferase gene in pAGal9-luc was substituted with a stuffer sequence of pAMo-nd.

(4) Preparation of a cell line KJMGER8 where Gal4-ER expression plasmid pGERbsrR2 was integrated in chromosomal DNA of Namalwa KJM-1 cells

Gal4-ER chimeric transcription factor expression plasmid pGERbsrR2 was dissolved in a TE buffer [10 mmol/L Tris-HCl (pH 8.0) and 1 mmol/L of ethylenediamine tetraacetate] so as to make 1 μg/μL and, after that, the plasmid, 4 μg for $6 \times 10^6$ cells, was transfected to Namalwa KJM-1 cells [*Cytotechnology,* 1, 151 (1988)] by an electroporation method [*Cytotechnology,* 3, 133 (1990)] to prepare transformed cells. Namalwa KJM-1 cell is a B-cell line adapted for serum-free culture, and capable of expressing the EBNA-1 gene.

The transformant was suspended in 8 ml of an RPMI 1640-ITPSG medium [a medium where a 1/40 amount of 7.5% $NaHCO_3$, 3% 200 mmol/L of L-glutamine solution (manufactured by Invitrogen), 0.5% penicillin-streptomycin solution (manufactured by Invitrogen comprising 5,000 units/ml of penicillin and 5,000 μg/ml of streptomycin), 10 mmol/L of N-2-hydroxyethylpiperazine-N'-2-hydroxypropane-3-sulfonic acid(HEPES), 3 μg/ml insulin, 5 μg/ml transferrin, 5 mmol/L sodium pyruvate, 125 nmol/L sodium selenite and 1 mg/ml galactose were added to an RPMI 1640 medium (manufactured by Nissui Seiyaku)] and cultured at 37°C in a $CO_2$ incubator for 24 hours.

After the cultivation, blasticidin S (KK-400: manufactured by Kaken Seiyaku) was added so as to make 2. 0 μg/ml, dispensed in a 96-well plate (500 to 2,000 cells/well) and cultivation was carried out to obtain many stable transformants (single clones) where pGERbsrR2 was integrated in chromosomal DNA. Each transformant was subcultured in RPMI 1640-ITPSG medium containing 2.0 μg/ml of blasticidin S.

By the method as mentioned below, an excellent stable transformant KJMGER8 cell having high induction ratio and low background upon non-inducing stage was selected from the above-mentioned stable transformants. An inducible expression plasmid pAGalSd1-luc of firefly luciferase was transfected to each transformant by an electroporation method and cultured for 2 days.

After the cultivation, 17β-estradiol (E8875: manufactured by Sigma) (final concentration 10 nmol/L) was added and, after the cultivation for 24 hours more, the firefly luciferase activity was measured. For the measurement of the activity, a luminometer-LB 953 (manufactured by Berthold) was used, 100 μl of a buffer for dissolving the cells [1% Triton X-100, 100 mmol/L $KH_2PO_4$ (pH 7.8) and 1 mmol/L dithiothreitol] was automatically injected into the above culture solution, then 300 μl of a substrate solution [25 mmol/L glycylglycine (pH 7.8), 15 mmol/L $MgSO_4$, 5 mmol/L ATP and 0.33 mmol/L luciferin] was automatically injected and the amounts of emission of light during 10 seconds was measured for adopting as a luciferase activity. For comparison, luciferase activity under the condition where no 17β-estradiol was added was also measured.

Luciferase activity under the condition where 17β-estradiol was added and luciferase activity under the condition where no 17β-estradiol was added were compared, induction ratio for gene expression was calculated, and KJMGER8 cell was selected as a clone with high induction ratio and low luciferase activity in the condition without addition of 17β-estradiol.

Referential Example 3: Construction of a reporter plasmid pACREpluc where firefly luciferase is a reporter

pACREpluc which is a reporter plasmid capable of expressing a firefly luciferase gene under the control of cAMP-responding element (CRE) was constructed by the following method. pACREpluc has oriP of Epstein Barr virus and the hygromycin-resistant gene.

pAMo [*J. Biol. Chem.,* 268, 22782 (1993); another name: pAMoPRC3Sc (Japanese Published Unexamined Patent Application No. 336,963/1993)] was partially digested with ClaI to obtain a DNA fragment where one site was cleaved. The DNA fragment was partially digested by MluI to obtain a ClaI-MluI fragment of 9.5 kb. pAGE248 [*J. Biol. Chem.,* 269, 14730 (1994)] was cleaved with ClaI and MluI to obtain a ClaI-MluI fragment of 1.5 kb comprising the hygromycin-resistant gene. The ClaI-MluI fragment derived from pAMo and the ClaI-MluI fragment derived from pAGE248 were ligated to construct a plasmid pAMoh.

pAMoh was cleaved with XhoI and HindIII to obtain a XhoI-HindIII fragment comprising the hygromycin-resistant gene. pAGal9-luc was cleaved with SalI and HindIII to obtain a SalI-HindIII fragment comprising oriP and Gal4UAS. The SalI-HindIII fragment derived from pAGal9-luc and the above-mentioned XhoI-HindIII fragment derived from pAMoh were ligated to construct a plasmid pAGal9h.

pBluescriptII KS+ (manufactured by Toyoboseki) was cleaved with SalI and XhoI and subjected to a dephosphorylation using phosphatase (Alkaline Phosphatase E. coli C75; manufactured by Takara Shuzo) to obtain a SalI-XhoI fragment comprising the ampicillin-resistant gene. As a result of annealing of synthetic oligonucleotides having nucleotide sequences of SEQ ID NOS: 5 and 6, respectively, a double-stranded DNA containing two CRE sequences was prepared. The double-stranded DNA was ligated to the above SalI-XhoI fragment derived from pBluescriptII KS+ to construct a plasmid pBS-CREI comprising two CRE sequences. The pBS-CREI is a plasmid where the double-stranded DNA is inserted in such a direction that cleaved site with SalI and cleaved site with XhoI are regenerated and has each one of the above cleaved sites.

pBS-CREI was cleaved with ScaI and XhoI to prepare a ScaI-XhoI fragment comprising ori of a phage f1. pBS-CREI was cleaved with ScaI and SalI to prepare a ScaI-SalI fragment comprising ColE1 ori. The ScaI-XhoI fragment and the ScaI-SalI fragment derived from pBS-CREI were ligated to construct pBS-CREII comprising 4 CRE sequences.

pBS-CREII was cleaved with ScaI and XhoI to obtain a ScaI-XhoI fragment comprising ori of a phage f1. pBS-CREII was cleaved with ScaI and SalI to obtain a ScaI-SalI fragment comprising ColE1 ori. The ScaI-XhoI fragment and the ScaI-SalI fragment derived from pBS-CREII were ligated to construct pBS-CREIV comprising 8 CRE sequences.

pBS-CREIV was cleaved with ScaI and XhoI to obtain a ScaI-XhoI fragment comprising ori of a phage f1. pBS-CREIV was cleaved with ScaI and SalI to obtain a ScaI-SalI fragment comprising Co1E1 ori. The ScaI-XhoI fragment and the ScaI-SalI fragment derived from pBS-CREIV were ligated to construct pBS-CREVIII comprising 16 CRE sequences.

pBS-CREVIII was cleaved with XhoI, subjected to a Klenow treatment, and further cleaved with HindIII to obtain a HindIII-XhoI (blunt end) fragment comprising 16 CREs. pAGalSd1 was cleaved with MluI and HindIII to obtain a MluI-HindIII fragment of 1.4 kb. pAGal19h was cleaved with XbaI, subjected to a Klenow treatment, and further cleaved with MluI to give a XbaI (blunt end)-MluI fragment. The HindIII-XhoI (blunt end) fragment derived from pBS-CREVIII, the MluI-HindIII fragment derived from pAGalSd1 and the XbaI (blunt end)-MluI fragment derived from pAGal19h were ligated to prepare a plasmid pACREh.

pAGal9-luc was cleaved with XhoI and NotI to obtain a XhoI-NotI fragment comprising the firefly luciferase gene. pACREh was cleaved with XhoI and NotI to obtain a XhoI-NotI fragment comprising CRE sequences. The XhoI-NotI fragment derived from pAGal9-luc and the XhoI-NotI fragment derived from pACREh were ligated to construct a plasmid pACREluc.

pACREluc was cleaved with HindIII, subjected to a Klenow treatment, and further cleaved with XhoI to obtain a HindIII (blunt end)-XhoI fragment comprising CRE and a HindIII (blunt end)-XhoI fragment comprising the firefly luciferase gene, respectively. The above-mentioned two HindIII (blunt end)-XhoI fragments derived from pACREluc were ligated to construct a plasmid pACREllucH in which HindIII site in upstream of CRE sequence in pACREluc disappeared.

pGL3-Enhancer vector (manufactured by Promega) was cleaved with HindIII and HpaI to obtain a HindIII-HpaI fragment comprising the luc+ gene (an improved firefly luciferase gene). pACREllucH was cleaved with NotI, subjected to a Klenow treatment, and further cleaved with HindIII to obtain a HindIII-NotI (blunt end) fragment containing CRE. The HindIII-HpaI fragment derived from pGL3-Enhancer vector and the HindIII-NotI (blunt end) fragment derived from pACREllucH were ligated to construct a plasmid pACREpluc.

Referential Example 4: Construction of a plasmid with inducible expression of GPR4

1 μg of mRNA derived from human lung (manufactured by Clontech) was used and a single-stranded cDNA was synthesized using a SUPERSCRIPT First-Stranded Synthesis System for RT-PCR (manufactured by Gibco). GPR4 cDNA was prepared by PCR using a solution (5 μl) where said single-stranded cDNA was diluted with water to an extent of 250-fold as a template and synthetic DNA having nucleotide sequences represented by SEQ ID NO: 7 and NO: 8 as a GPR4 gene-specific primer. Sequence of the GPR4 gene-specific primer was designed on the basis of sequence information of GPR4 gene (GenBank Accession No. U21051). With regard to an enzyme, PfuTurbo DNA Polymerase (manufactured by Stratagene) was used. With regard to a buffer for conducting the PCR, a buffer of 10-fold concentration attached to the enzyme was used. The PCR was conducted by treating at 95°C for 5 minutes and conducting 30 cycles of reaction each comprising at 94°C for 1 minute, at 60°C for 1 minute and at 72°C for 1 minute using a thermal cycler DNA engine (manufactured by MJ Research).

The amplified GPR4 cDNA fragment was cleaved with HindIII and NotI which cleave a sequence designed on a primer. Fragment containing GPR4 cDNA was recovered by an agarose gel electrophoretic method.

Said cleaved fragment was integrated between HindIII and NotI of plasmid pAGal9-nd whereupon a plasmid pAGal9-GPR4 with inducible expression of GPR4 was constructed.

Nucleotide sequences of 5'-end and 3'-end of said cDNA were determined using a primer (synthetic DNA having sequences represented by SEQ ID NO: 9 and NO: 10) which is specific to nucleotide sequence in pAGal9-nd. A synthetic DNA which is specific to the determined nucleotide sequence was prepared and, when it was used as a primer, a nucleotide sequence farther ahead was determined. As a result of repeating said method, total nucleotide sequence of said cDNA was determined and it was confirmed to code for GPR4. For the determination of the nucleotide sequence, a DNA sequencer 377 of Perkin Elmer and a reaction kit (ABI Prism™ BigDye™ Terminator Cycle Sequencing Ready Reaction kit: Applied Biosystems) were used.

A nucleotide sequence of DNA fragment integrated into a plasmid was determined and it was confirmed to code for GPR4.

Referential Example 5: Construction of assay cells of GPR4

2 μg of plasmid pAGal9-GPR4 with inducible expression of GPR4 and 2 μg of reporter plasmid pACREpluc were co-transferred into $6 \times 10^6$ cell of KJMGER8 by the above-mentioned electroporation. The transformants were suspended in 8 ml of an RPMI 1640-ITPSG medium and cultured in a $CO_2$ incubator at 37°C for 24 hours. After the cultivation, blasticidin S (2.0 μg/ml), hygromycin B (300 μg/ml) and geneticin (500 μg/ml) were added and cultivation was carried out for 14 days more, and stable transformants (called GPR4 assay cells) were obtained. The transformants were subcultured in an RPMI 1640-ITPSG medium containing blasticidin S (2.0 μg/ml), hygromycin B (300 μg/ml) and geneticin (500 μg/ml).

Similarly, control plasmid pAGal9-nd (2μg) and reporter plasmid pAGREpluc (2 μg) were co-transferred into KJMGER8 and a stable transformant (called control cell) was obtaiend.

Referential Example 6: Cloning of DNA coding for human GPR4 homolog derived from mice

Based upon the nucleotide sequence information of human GPR4 gene [Accession (AC) No. U21051], search was conducted using a database of NCBI as an object. As a result, mouse genome sequence (AC 073784) and plural expression sequence tag (EST) sequences (BF 178464, AA 968193, AA 798732, AI 840893 and AI 851037) were selected as sequences having high homology. A nucleotide sequence of a gene constructed from said mouse genome sequence and EST is shown in SEQ ID NO: 14 and an amino acid sequence of a polypeptide encoded by said gene is shown in SEQ ID NO: 13. When said amino acid was compared with the amino acid sequence of human GPR4 using an analysis program [GENETYX WIN ver. 5.0 (manufactured by Software)], it is confirmed that an identity score

is 92.7%.

Therefore, it is presumed that a polypeptide having the amino acid sequence represented by SEQ ID NO: 13 is a human GPR4 homolog of mouse (mouse GPR4).

Accordingly, DNA coding for mouse GPR4 can be prepared by a PCR where mouse cDNA library which is commercially available or can be prepared by a known method is used as a template and an oligonucleotide which is able to be designed and synthesized depending upon the nucleotide sequence represented by SEQ ID NO: 14 as a primer set.

Referential Example 7: Cloning of DNA coding for a human GPR4 homolog derived from rats

**[0487]** - Based upon the nucleotide sequence information of human GPR4 gene (AC No. U21051), search was conducted using a database of NCBI as an object. As a result, two rat genome sequences (AC 119447.2 and AC 096180.2) and plural rat EST sequences (BF 544182, AI 170948, AI 008858, AI 235374, AI 502871 and BQ 194515) were selected as sequences having high homology. Based upon those sequences and the nucleotide sequence information of mice shown by SEQ ID NO: 14, oligonucleotides having the nucleotide sequences represented by SEQ ID NO: 15 and SEQ ID NO: 16 were prepared.

Each 1.0 μmol/L of said oligonucleotides was used as a primer set and 2 μL of cDNA prepared from mRNA derived from rat lung was used as a template whereby, in order to make the concentration of each component which is mentioned below 200 μmol/L, 40 μL of a reaction solution containing 2.5 units of dNTP (dATP, dGTP, dCTP, dTTP), Tag Gold (manufactured by Perkin Elmer) and 1 × Taq Gold (Mg plus) buffer (Perkin Elmer) was prepared and then a PCR was conducted under the following condition.

Thus, a thermal cycler PTC-200 (manufactured by MJ Research) was used and heating was conducted at 95°C for 10 minutes, then 30 cycles of reaction each comprises 94°C for 1 minute, 55°C for 1 minute and 72°C for 1 minute were conducted and, further, heating at 72°C for 5 minutes was conducted.

From the resulting reaction solution of the PCR, 5 μL was collected and, after confirming by agarose gel electrophoresis that about 1.1 kb of DNA fragments presumed to be DNA coding for GPR4 were amplified, DNA fragments were eluted and recovered using QIAEX II Gel Extraction Kit (manufactured by Qiagen) according to the manual attached to the kit.

The above-recovered DNA fragments (50 ng) and 50 ng of pT7Blue T-Vector (manufactured by Novagen) were ligated using DNA Ligation Kit ver. 2 (manufactured by Takara Shuzo) according to the manual attached to the kit to prepare a recombinant plasmid DNA. A plasmid pT7RG was prepared by a conventional method from a transformant obtained by a transformation of *Escherichia coli* JM109 strain using the resulting recombinant plasmid DNA. As a result of determination of the total nucleotide sequence of the plasmid pT7RG, the pT7RG contains about 1.1 kb of cDNA having the nucleotide sequence represented by SEQ ID NO: 18. An amino acid sequence of a polypeptide correspongding to DNA comprising the nucleotide sequence represented by SEQ ID NO: 18 is shown in SEQ ID NO: 17. When the amino acid sequence was compared with amino acid sequences of human and mouse GPR4 using an analysis program [GENETYX WIN ver. 5.0 (manufactured by Software)], it is confirmed that identity scores are 93.0% and 99.2%, respectively.

Consequently, it has been clarified that a polypeptide having the amino acids represented by SEQ ID NO: 17 is a human GPR4 homolog of rat (rat GRP4).

Preparation Example 1: Tablet

A tablet comprising the following composition is prepared by a conventional method.

Formulation:

| | |
|---|---|
| Compound 1 | 20 mg |
| Lactose | 143.4 mg |
| Starch | 30 mg |
| Hydroxypropyl cellulose Magnesium stearate | 6 mg 0.6 mg |
| | 200 mg |

Preparation Example 2: Injection solution

Injection solution comprising the following composition is prepared by a conventional method.

Formulation:

| | |
|---|---|
| Compound 5 | 2 mg |
| Pure soybean oil | 200 mg |
| Pure egg yolk lecithin | 24 mg |
| Glycerol for injection | 50 mg |
| Distilled water for injection | 1.72 ml |
| | 2.00 ml |

Industrial Applicability

The present invention provides an agent for prevention and/or treatment of itching comprising a substance capable of suppressing the function involved in signal transduction of GPR4 as an active ingredient; a nitrogen-containing tricyclic compound or a quaternary ammonium salt thereof, or a pharmaceutically acceptable salt thereof, which has an action of prevention and/or treatment of itching; a nitrogen-containing tricyclic compound or a quaternary ammonium salt thereof, or a pharmaceutically acceptable salt thereof, which has a suppressive action for the function involved in signal transduction of GPR4; an agent for prevention and/or treatment of itching comprising a nitrogen-containing tricyclic compound or a quaternary ammonium salt thereof, or a pharmaceutically acceptable salt thereof as an active ingredient; a suppressor of the function involved in signal transduction of GPR4 comprising a nitrogen-containing tricyclic compound or a quaternary ammonium salt thereof, or a pharmaceutically acceptable salt thereof as an active ingredient; and a method for screening of a therapeutic agent for itching wherein reduction in the number of scratching behavior induced by SPC is used as an index.

Free Text of Sequence Listing

SEQ ID NO: 1 - Illustration of artificial sequence: Synthetic DNA

SEQ ID NO: 2 - Illustration of artificial sequence: Synthetic DNA

SEQ ID NO: 3 - Illustration of artificial sequence: Synthetic DNA

SEQ ID NO: 4 - Illustration of artificial sequence: Synthetic DNA

SEQ ID NO: 5 - Illustration of artificial sequence: Synthetic DNA

SEQ ID NO: 6 - Illustration of artificial sequence: Synthetic DNA

SEQ ID NO: 7 - Illustration of artificial sequence:

Synthetic DNA

SEQ ID NO: 8 - Illustration of artificial sequence: Synthetic DNA

SEQ ID NO: 9 - Illustration of artificial sequence: Synthetic DNA

SEQ ID NO: 10 - Illustration of artificial sequence: Synthetic DNA

SEQ ID NO: 15 - Illustration of artificial sequence: Synthetic DNA

SEQ ID NO: 16 - Illustration of artificial sequence: Synthetic DNA

SEQ ID NO: 19 - Illustration of artificial sequence: Synthetic DNA

SEQ ID NO: 20 - Illustration of artificial sequence: Synthetic DNA

## Claims

1. An agent for prevention and/or treatment of itching, which comprises, as an active ingredient, a substance capable of suppressing the function involved in signal transduction of a protein having the amino acid sequence represented by SEQ ID NO: 11.

2. An agent for prevention and/or treatment of itching, which comprises one of the following 1) to 4) as an active ingredient:

 1) an oligonucleotide having a sequence complementary to that of oligonucleotide comprising continuous 5 to 60 nucleotides selected from the nucleotide sequence represented by SEQ ID NO: 12 or a derivative of said oligonucleotide,
 2) an oligonucleotide having a sequence complementary to that of oligonucleotide comprising continuous 5 to 60 nucleotides selected from the nucleotide sequence represented by SEQ ID NO: 14 or a derivative of said oligonucleotide,
 3) an oligonucleotide having a sequence complementary to that of oligonucleotide comprising continuous 5 to 60 nucleotides selected from the nucleotide sequence represented by SEQ ID NO: 18 or a derivative of said oligonucleotide, and
 4) an oligonucleotide comprising 5 to 60 nucleotides which hybridizes under stringent conditions with DNA having the nucleotide sequence represented by one member selected from SEQ ID NOs: 12, 14 and 18 and which is capable of suppressing the function involved in signal transduction of a protein having the amino acid sequence represented by SEQ ID NO: 11 or a derivative of said oligonucleotide.

3. An agent for prevention and/or treatment of itching, which comprises one of the following 1) to 4) as an active ingredient:

 1) an antibody which recognizes a protein having the amino acid sequence represented by SEQ ID NO: 11,
 2) an antibody which recognizes a protein having the amino acid sequence represented by SEQ ID NO: 13,
 3) an antibody which recognizes a protein having the amino acid sequence represented by SEQ ID NO: 17, and
 4) an antibody, which recognizes a protein having the amino acid sequence in which one or more amino acid (s) is/are deleted, substituted or added in the amino acid sequence represented by one member selected from SEQ ID NOs:11, 13 and 17 and which has the function involved in signal tranduction of a protein having the amino acid sequence represented by SEQ ID NO:11.

4. A nitrogen-containing tricyclic compound represented by the formula (I) or a quaternary ammonium salt thereof, or a pharmaceutically acceptable salt thereof;

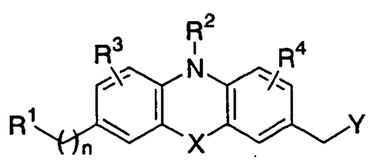

(I)

[wherein $R^1$ represents a substituted or unsubstituted heterocyclic group, $-NR^5R^6$ (wherein $R^5$ and $R^6$ are the same or different and each represents hydrogen, substituted or unsubstituted lower alkyl, substituted or unsubstituted cycloalkyl, substituted or unsubstituted lower alkenyl, substituted or unsubstituted lower alkynyl, substituted or unsubstituted aralkyl or substituted or unsubstituted heterocyclic alkyl, or $R^5$ and $R^6$ are combined together with the adjacent nitrogen atom to form a substituted or unsubstituted heterocyclic group), $-OR^7$ (wherein $R^7$ represents hydrogen, substituted or unsubstituted lower alkyl, substituted or unsubstituted lower alkanoyl, substituted or unsubstituted cycloalkyl, substituted or unsubstituted lower alkenyl, substituted or unsubstituted lower alkynyl, substituted or unsubstituted aryl, substituted or unsubstituted aralkyl or substituted or unsubstituted heterocyclic alkyl), $-SR^{7a}$ (wherein $R^{7a}$ has the same meaning as the above $R^7$), $-CONR^{5a}R^{6a}$ (wherein $R^{5a}$ and $R^{6a}$ have the same meanings as the above $R^5$ and $R^6$, respectively), $-CO_2R^{7b}$ (wherein $R^{7b}$ has the same meaning as the above $R^7$), $-N^+R^{5b}R^{6b}R^8$ (wherein $R^{5b}$ and $R^{6b}$ have the same meanings as the above $R^5$ and $R^6$, respectively, and $R^8$ represents lower alkyl, lower alkenyl or aralkyl), formyl, carboxy or cyano;

$R^2$ represents hydrogen, substituted or unsubstituted lower alkyl, substituted or unsubstituted cycloalkyl,

substituted or unsubstituted lower alkenyl, substituted or unsubstituted lower alkynyl, substituted or unsubstituted aralkyl or substituted or unsubstituted heterocyclic alkyl;

$R^3$ and $R^4$ are the same or different and each represents hydrogen, lower alkyl or halogen;
n represents 0 or 1;
X represents $-(CH_2)_2-$ or -CH=CH-; and
Y represents the formula (II);

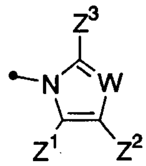

(II)

(wherein W represents CH or a nitrogen atom;
$Z^1$ and $Z^2$ are the same or different and each represents hydrogen, substituted or unsubstituted lower alkyl, substituted or unsubstituted cycloalkyl, substituted or unsubstituted lower alkenyl, substituted or unsubstituted lower alkynyl, substituted or unsubstituted aryl, substituted or unsubstituted aralkyl or substituted or unsubstituted heterocyclic alkyl, or $Z^1$ and $Z^2$ are combined together with two carbon atoms being adjacent to each of them to form a substituted or unsubstituted aromatic ring or substituted or unsubstituted heterocycle; and
$Z^3$ represents hydrogen, substituted or unsubstituted lower alkyl, substituted or unsubstituted cycloalkyl, substituted or unsubstituted lower alkenyl, substituted or unsubstituted lower alkynyl, substituted or unsubstituted aryl, a substituted or unsubstituted heterocyclic group, substituted or unsubstituted aralkyl or substituted or unsubstituted heterocyclic alkyl)].

**5.** The nitrogen-containing tricyclic compound or the quaternary ammonium salt thereof, or the pharmaceutically acceptable salt thereof according to claim 4, wherein $R^1$ is $-NR^5R^6$ and $R^5$ and $R^6$ are combined together with the adjacent nitrogen atom to form a substituted or unsubstituted heterocyclic group.

**6.** The nitrogen-containing tricyclic compound or the quaternary ammonium salt thereof, or the pharmaceutically acceptable salt thereof according to claim 4 or 5, wherein $R^2$ is hydrogen.

**7.** The nitrogen-containing tricyclic compound or the quaternary ammonium salt thereof, or the pharmaceutically acceptable salt thereof according to any one of claims 4 to 6, wherein $R^3$ and $R^4$ are hydrogen.

**8.** The nitrogen-containing tricyclic compound or the quaternary ammonium salt thereof, or the pharmaceutically acceptable salt thereof according to any one of claims 4 to 7, wherein $Z^1$ and $Z^2$ are combined together with two carbon atoms being adjacent to each of them to form substituted or unsubstituted heterocycle.

**9.** A pharmaceutical compositon comprising the nitrogen-containing tricyclic compound or the quaternary ammonium salt thereof, or the pharmaceutically acceptable salt thereof according to any one of claims 4 to 8 as an active ingredient.

**10.** An agent for prevention and/or treatment of itching comprising the nitrogen-containing tricyclic compound or the quaternary ammonium salt thereof, or the pharmaceutically acceptable salt thereof according to any one of claims 4 to 8 as an active ingredient.

**11.** A suppressor of the function involved in signal transduction of a protein having the amino acid sequence represented by SEQ ID NO: 11, comprising the nitrogen-containing tricyclic compound or the quaternary ammonium salt thereof, or the pharmaceutically acceptable salt thereof according to any one of claims 4 to 8 as an active ingredient.

**12.** A method for screening a therapeutic agent for itching, which comprises the following steps 1) to 4):

1) the step where sphingosyl phosphorylcholine (SPC) is subcutaneously and intracutaneously administered to a mammal except a human being so that scratching behavior in the mammal except a human being is induced,

2) the step where numbers of scratching behavior in the mammal except a human being induced by SPC in the presence or absence of the test compound are measured,
3) the step where numbers of scratching behavior in the mammal except a human being induced by SPC in the presence of the test compound and in the absence of the test compound are compared, and
4) the step where a substance which decreases the number of scratching behavior induced by SPC is selected from test compounds.

**13.** A method for prevention and/or treatment of itching, which comprises administering an effective amount of the nitrogen-containing tricyclic compound or the quaternary ammonium salt thereof, or the pharmaceutically acceptable salt thereof described in any one of claims 4 to 8.

**14.** Use of the nitrogen-containing tricyclic compound or the quaternary ammonium salt thereof, or the pharmaceutically acceptable salt thereof described in any one of claims 4 to 8 for the manufacture of an agent for prevention and/or treatment of itching.

**15.** The agent for prevention and/or treatment of itching according to any one of claims 1 to 3 and 10, wherein the itching is that which is accompanied by a disease selected from a skin disease, a liver/biliary tract disease, a renal disease, an endocrinal/metabolic disease, a blood disease, a malignant tumor, a nerve disease and AIDS.

**16.** The agent for prevention and/or treatment of itching according to claim 15, wherein the itching is that accompanied by skin disease and said skin disease is that selected from atopic dermatitis, eczema/dermatitis, urticaria, pruritus, xeroderma, bite, scabies, fungal infection, skin itching, hypertrophic cicatrix, psoriasis, blister and drug eruption.

**17.** A method for prevention and/or treatment of itching, which comprises administering a therapeutically effective amount of a substance capable of suppressing the function involved in signal transduction of a protein having the amino acid sequence mentioned in SEQ ID NO: 11.

**18.** A method for prevention and/or treatment of itching, which comprises administering a therapeutically effective amount of an oligonucleotide or a derivative of said oligonucleotide which is any one of 1) to 4) described in claim 2.

**19.** A method for prevention and/or treatment of itching, which comprises administering a therapeutically effective amount of an antibody which is any one of 1) to 4) described in claim 3.

**20.** Use of a substance capable of suppressing the function involved in signal transduction of a protein having the amino acid sequence represented by SEQ ID NO: 11 for the manufacture of an agent for prevention and/or treatment of itching.

**21.** Use of an oligonucleotide or a derivative of said oligonucleotide which is any one of 1) to 4) described in claim 2 for the manufacture of an agent for prevention and/or treatment of itching.

**22.** Use of an antibody which is any one of 1) to 4) described in claim 3 for the manufacture of an agent for prevention and/or treatment of itching.

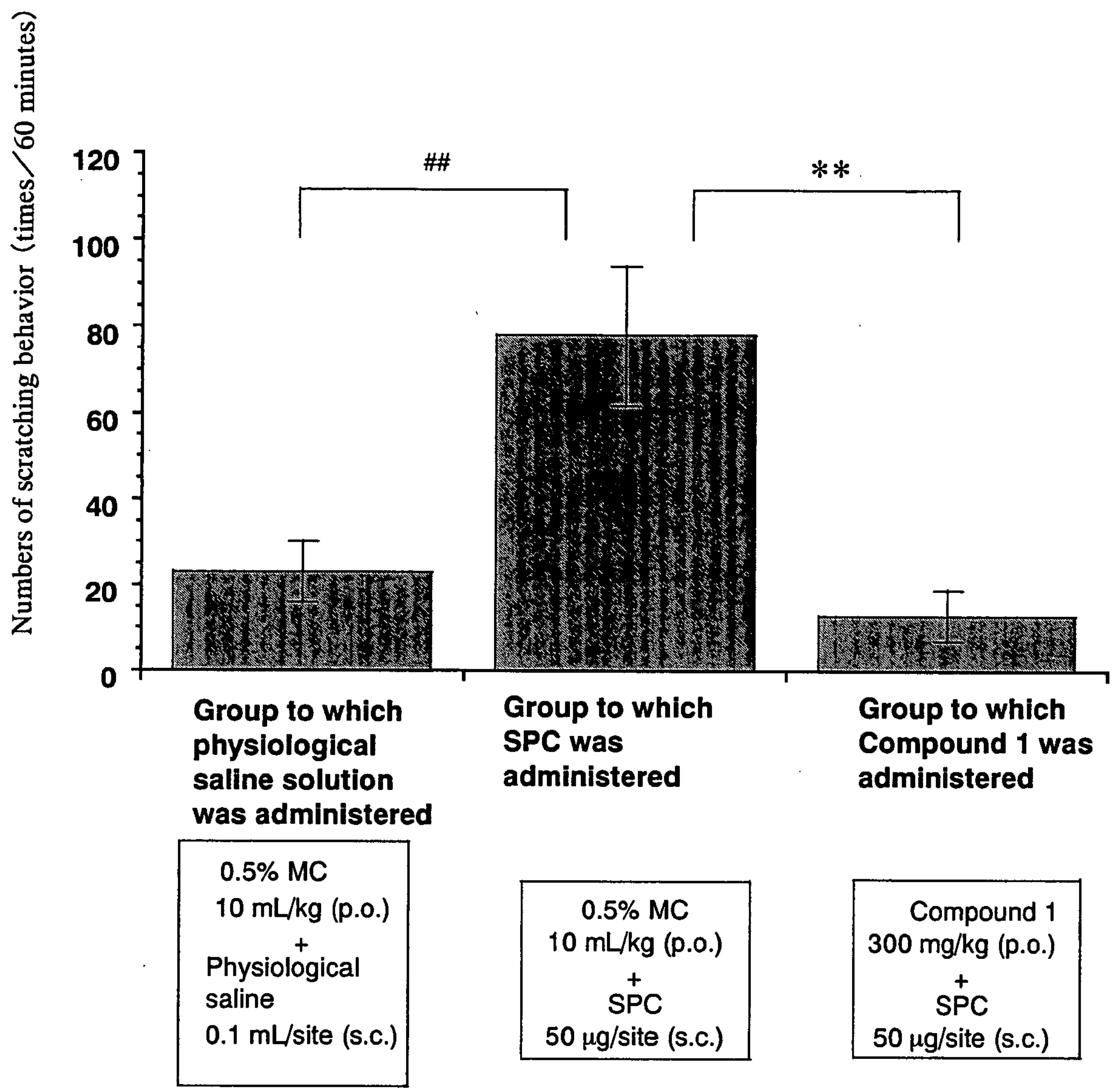

Numbers of scratching behavior (times/60 minutes)
120
100
80
60
40
20
0
##
**
Group to which physiological saline solution was administered
Group to which SPC was administered
Group to which Compound 1 was administered
0.5% MC 10 mL/kg (p.o.) + Physiological saline 0.1 mL/site (s.c.)
0.5% MC 10 mL/kg (p.o.) + SPC 50 μg/site (s.c.)
Compound 1 300 mg/kg (p.o.) + SPC 50 μg/site (s.c.)


Fig.1

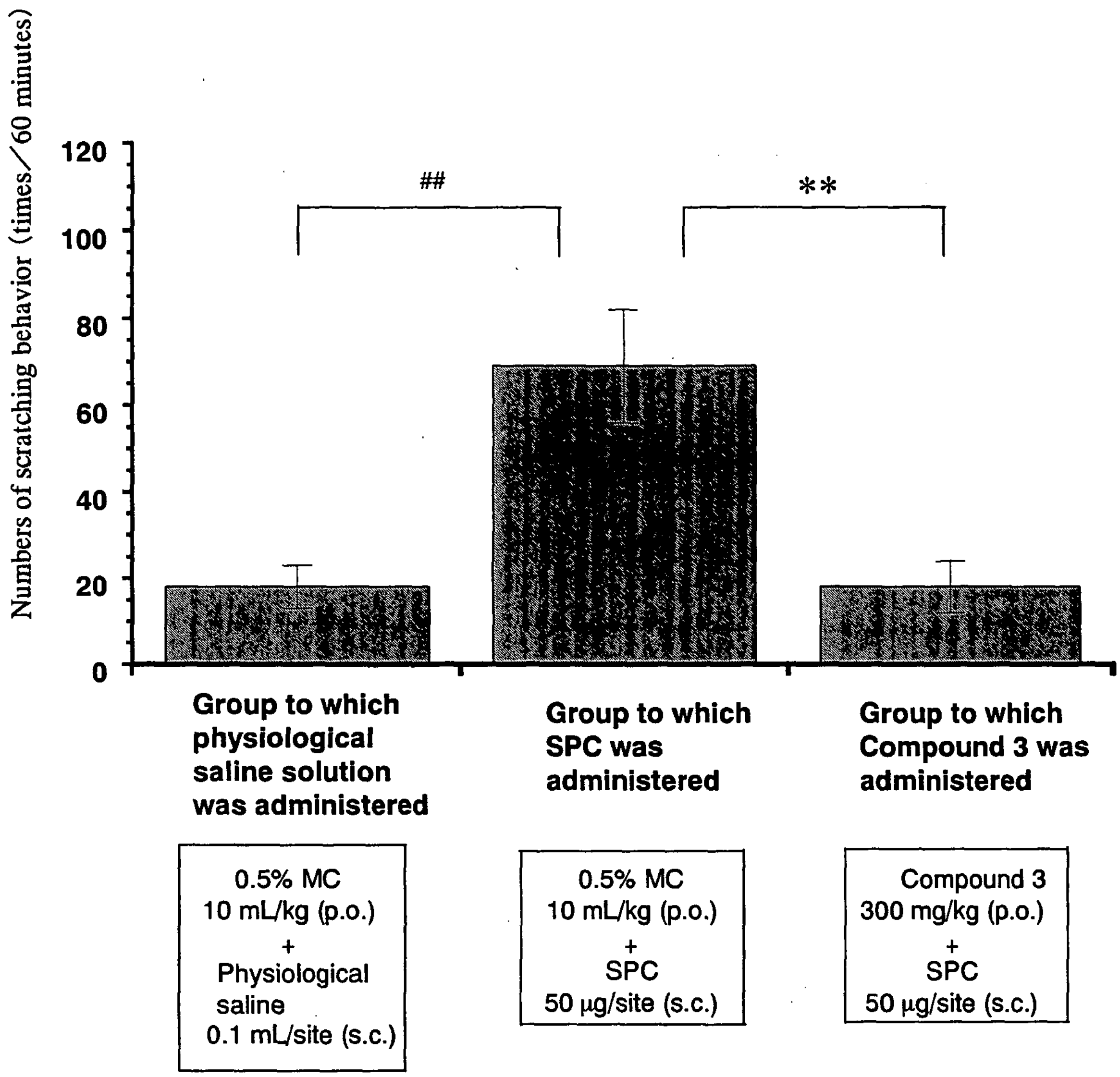
Numbers of scratching behavior (times／60 minutes)
120
100
80
60
40
20
0
##
**
Group to which physiological saline solution was administered
Group to which SPC was administered
Group to which Compound 3 was administered
0.5% MC 10 mL/kg (p.o.) + Physiological saline 0.1 mL/site (s.c.)
0.5% MC 10 mL/kg (p.o.) + SPC 50 μg/site (s.c.)
Compound 3 300 mg/kg (p.o.) + SPC 50 μg/site (s.c.)

Fig .2

## INTERNATIONAL SEARCH REPORT

International application No.
PCT/IB03/03475

A. CLASSIFICATION OF SUBJECT MATTER
Int.Cl[7] A61K45/00, 31/519, 31/55, 31/7088, 39/395, 48/00, A61P17/04, 43/00, C07D401/14, 403/06, 417/14, 471/14, 519/00

According to International Patent Classification (IPC) or to both national classification and IPC

B. FIELDS SEARCHED

Minimum documentation searched (classification system followed by classification symbols)
Int.Cl[7] A61K45/00-45/08, 31/00-31/80, 48/00, A61P1/00-43/00, C07D401/00-401/14, 403/00-403/06, 417/00-417/14, 471/00-471/14, 519/00

Documentation searched other than minimum documentation to the extent that such documents are included in the fields searched

Electronic data base consulted during the international search (name of data base and, where practicable, search terms used)
CAPLUS(STN), REGISTRY(STN), WPI(DIALOG), MEDLINE(STN), EMBASE(STN), BIOSIS(STN), BIOTECHABS(STN), JSTPLUS(JOIS), JMEDPLUS(JOIS), DDBJ/GanBank/EMBL, SwissProt/PIR/PDB, GeneSeq

C. DOCUMENTS CONSIDERED TO BE RELEVANT

| Category* | Citation of document, with indication, where appropriate, of the relevant passages | Relevant to claim No. |
|---|---|---|
| Y | WO 02/24222 A2 (THE CLEVELAND CLINIC FOUNDATION), 28 March, 2002 (28.03.02), Claims; examples & AU 200192867 A & US 2002/0107197 A1 | 1-3,12,15, 16,20-22 |
| Y | BEERS, M.H. et al., (ed.) THE MERCK MANUAL OF DIAGNOSIS AND THERAPY., 17th Edition., 1999, ISBN 0911910-10-7, ISSN 0076-6526, pages 786 to 793; particularly, page ATOPIC DERMATITIS | 1-3,12,15, 16,20-22 |
| Y | WO 02/061087 A2 (LIFESPAN BIOSCIENCES, INC.), 08 August, 2002 (08.08.02), Claims; [265] to [266]; sequence Nos. 272, 273 (Family: none) | 1-3,12,15, 16,20-22 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 11 December, 2003 (11.12.03) | 24 December, 2003 (24.12.03) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/IB03/03475

C (Continuation). DOCUMENTS CONSIDERED TO BE RELEVANT

| Category* | Citation of document, with indication, where appropriate, of the relevant passages | Relevant to claim No. |
|---|---|---|
| Y | HEIBER, M. et al., Isolation of Three Novel Human Encoding G Protein-Coupled Receptors., DNA Cell Biol., 1995, 14(1), pages 25 to 35 | 1-3,12,15, 16,20-22 |
| Y | MAHADEVAN, M.S. et al., Isolation of a Novel G Protein-Coupled Receptor(GPR4) Localized to Chromosome 19q13.3. Genomics, 1995, 30, pages 84 to 88 | 1-3,12,15, 16,20-22 |
| A | EP 549352 A2 (KYOWA HAKKO KOGYO CO., LTD.), 30 June, 1993 (30.06.93), & JP 6-228065 A & US 5378701 A & US 5478840 A & US 5607955 A | 4-11,14-16 |
| A | EP 325755 A1 (KYOWA HAKKO KOGYO CO., LTD.), 02 August, 1989 (02.08.89), & JP 1-308274 A & AU 8826711 A & US 4994463 A & US 5143922 A & US 5302602 A | 4-11,14-16 |
| A | JP 9-40662 A (KYOWA HAKKO KOGYO CO., LTD.), 10 February, 1997 (10.02.97), (Family: none) | 4-11,14-16 |

Form PCT/ISA/210 (continuation of second sheet) (July 1998)

**INTERNATIONAL SEARCH REPORT**

International application No.
PCT/JP03/03475

**Box I Observations where certain claims were found unsearchable (Continuation of item 2 of first sheet)**

This international search report has not been established in respect of certain claims under Article 17(2)(a) for the following reasons:

1. [X] Claims Nos.: 13, 17-19
because they relate to subject matter not required to be searched by this Authority, namely:
Claims 13 and 17 to 19 pertain to methods for treatment of the human body by therapy (PCT Article 17(2)(a)(i), PCT Rule 39.1(iv)).

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:
The technical matter common to claims 1-3 and 12, parts of claims 15 and 16 referring to claims 1-3, and claims 20-22 is prevention and/or treatment of itch by using the active ingredient a substance capable of suppressing the signal-transducing functions of a protein having an amino acid sequence set forth in sequence number 11, while that common to claims 4-11 and 14, and parts of claims 15 and 16 referring to claim 10 is compounds represented by the general formula (I) in themselves.
Therefore, there is no technical matter common to both groups which is considered as a special technical feature, and the two groups of inventions

(continued to extra sheet)

1. [X] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** [ ] The additional search fees were accompanied by the applicant's protest.
[X] No protest accompanied the payment of additional search fees.

Form PCT/ISA/210 (continuation of first sheet (1)) (July 1998)

**INTERNATIONAL SEARCH REPORT**

International application No.
PCT/IB03/03475

Continuation of Box No. II of continuation of first sheet(1)

are not considered as being so linked as to form a single general inventive concept.

Form PCT/ISA/210 (extra sheet) (July 1998)

SEQUENCE LISTING

<110> KYOWA HAKKO KOGYO CO., LTD

<120> An agent for prevention and/or treatment of itching

<130> 11503WO1

<140>
<141>

<150> JP 2002/241522
<151> 2002-08-22

<160> 20

<170> PatentIn Ver. 2.1

<210> 1
<211> 54
<212> DNA
<213> Artificial Sequence

<220>
<223> Description of Artificial Sequence: synthetic DNA

<400> 1
tcgacaaata aagcaatagc atcacaaatt tcacaaataa agcatttttt tcaa 54

<210> 2
<211> 54

<212> DNA
<213> Artificial Sequence

<220>
<223> Description of Artificial Sequence: synthetic DNA

<400> 2
tgcattgaaa aaaatgcttt atttgtgaaa tttgtgatgc tattgcttta tttg 54

<210> 3
<211> 39
<212> DNA
<213> Artificial Sequence

<220>
<223> Description of Artificial Sequence: synthetic DNA

<400> 3
tgcattctag ttgtggtttg tccaaactcg agcccgggg 39

<210> 4
<211> 39
<212> DNA
<213> Artificial Sequence

<220>
<223> Description of Artificial Sequence: synthetic DNA

<400> 4
gtacccccgg gctcgagttt ggacaaacca caactagaa 39

<210> 5
<211> 40
<212> DNA

<213> Artificial Sequence

<220>
<223> Description of Artificial Sequence: synthetic DNA

<400> 5
tcgacggtat cgattcgact gacgtcatac ttgacgtcac 40

<210> 6
<211> 40
<212> DNA
<213> Artificial Sequence

<220>
<223> Description of Artificial Sequence: synthetic DNA

<400> 6
tcgagtgacg tcaagtatga cgtcagtcga atcgataccg 40

<210> 7
<211> 29
<212> DNA
<213> Artificial Sequence

<220>
<223> Description of Artificial Sequence: synthetic DNA

<400> 7
gccccagaag cttaagtgcc caccatggg 29

<210> 8
<211> 33
<212> DNA

<213> Artificial Sequence

<220>
<223> Description of Artificial Sequence: synthetic DNA

<400> 8
gttcattgtg gcggccgcag catcttcagc tgc 33

<210> 9
<211> 25
<212> DNA
<213> Artificial Sequence

<220>
<223> Description of Artificial Sequence: synthetic DNA

<400> 9
cggagactct agagggtata taatg 25

<210> 10
<211> 21
<212> DNA
<213> Artificial Sequence

<220>
<223> Description of Artificial Sequence: synthetic DNA

<400> 10
ctaatacgac tcactatagg g 21

<210> 11
<211> 362

<212> PRT
<213> Homo sapiens

<400> 11
Met Gly Asn His Thr Trp Glu Gly Cys His Val Asp Ser Arg Val Asp
1               5                   10                  15

His Leu Phe Pro Pro Ser Leu Tyr Ile Phe Val Ile Gly Val Gly Leu
            20                  25                  30

Pro Thr Asn Cys Leu Ala Leu Trp Ala Ala Tyr Arg Gln Val Gln Gln
        35                  40                  45

Arg Asn Glu Leu Gly Val Tyr Leu Met Asn Leu Ser Ile Ala Asp Leu
    50                  55                  60

Leu Tyr Ile Cys Thr Leu Pro Leu Trp Val Asp Tyr Phe Leu His His
65                  70                  75                  80

Asp Asn Trp Ile His Gly Pro Gly Ser Cys Lys Leu Phe Gly Phe Ile
                85                  90                  95

Phe Tyr Thr Asn Ile Tyr Ile Ser Ile Ala Phe Leu Cys Cys Ile Ser
            100                 105                 110

Val Asp Arg Tyr Leu Ala Val Ala His Pro Leu Arg Phe Ala Arg Leu
        115                 120                 125

Arg Arg Val Lys Thr Ala Val Ala Val Ser Ser Val Val Trp Ala Thr
    130                 135                 140

Glu Leu Gly Ala Asn Ser Ala Pro Leu Phe His Asp Glu Leu Phe Arg
145                 150                 155                 160

Asp Arg Tyr Asn His Thr Phe Cys Phe Glu Lys Phe Pro Met Glu Gly
                165                 170                 175

Trp Val Ala Trp Met Asn Leu Tyr Arg Val Phe Val Gly Phe Leu Phe
180 185 190

Pro Trp Ala Leu Met Leu Leu Ser Tyr Arg Gly Ile Leu Arg Ala Val
195 200 205

Arg Gly Ser Val Ser Thr Glu Arg Gln Glu Lys Ala Lys Ile Lys Arg
210 215 220

Leu Ala Leu Ser Leu Ile Ala Ile Val Leu Val Cys Phe Ala Pro Tyr
225 230 235 240

His Val Leu Leu Leu Ser Arg Ser Ala Ile Tyr Leu Gly Arg Pro Trp
245 250 255

Asp Cys Gly Phe Glu Glu Arg Val Phe Ser Ala Tyr His Ser Ser Leu
260 265 270

Ala Phe Thr Ser Leu Asn Cys Val Ala Asp Pro Ile Leu Tyr Cys Leu
275 280 285

Val Asn Glu Gly Ala Arg Ser Asp Val Ala Lys Ala Leu His Asn Leu
290 295 300

Leu Arg Phe Leu Ala Ser Asp Lys Pro Gln Glu Met Ala Asn Ala Ser
305 310 315 320

Leu Thr Leu Glu Thr Pro Leu Thr Ser Lys Arg Asn Ser Thr Ala Lys
325 330 335

Ala Met Thr Gly Ser Trp Ala Ala Thr Pro Pro Ser Gln Gly Asp Gln
340 345 350

Val Gln Leu Lys Met Leu Pro Pro Ala Gln
355 360

<210> 12
<211> 2932
<212> DNA
<213> Homo sapiens

<400> 12
ctgcagtcag gcggtgaact gacttcatcc caatccctca gcccccacca ggaccagtct 60
ggagtccctc ccctgccccc attgaaattt cccttccgtc cccaaactta cctctgatct 120
agaccttact cacctccttc ctgtttccta agactccttc ctgccgtcca cagaccgagc 180
cttttatctt tgtccaccct gtgccagaca cctccttttc cagaaccttc tccttactgg 240
tgaccttact tatctctgtt gctttctggg gtcctaggaa atgccagcac tcccacccac 300
attgcctgaa ctttccaaca ctccctagct gcgctgtgtc ctatctcaac acttcctcat 360
gtatttcttg tgtcttctag aacattcccc cgccattatt acttcaatat ggctacacat 420
acttcctaat tgccctgcaa accatctcct tctcaccatt gcccagcgat gctttcgtct 480
cctccataaa cactcccgga gaccaatttt tgtgtcaccc ccatactccc tcgttgacac 540
actgactcca tacataacct ccttgaaaaa cctctttatt aatctcacca tcctccagac 600
ttccctcctg tcataattcc atccctcctc caacttttcc ctctcaagct ctgcccttcc 660
cagcccagcc cagcctaccc aacctcatct cttccctgta gaccacatcc caccatgttc 720
ccctgagcct ccaaggaagg ggctcagggg gccccatggc ctcccgctcc ctgtggcccc 780
acagcccccg tgggccaggg gaagcgcccc agaagccgaa gtgcccacc atg ggc aac 838
Met Gly Asn
1

cac acg tgg gag ggc tgc cac gtg gac tcg cgc gtg gac cac ctc ttt 886
His Thr Trp Glu Gly Cys His Val Asp Ser Arg Val Asp His Leu Phe
5 10 15

ccg cca tcc ctc tac atc ttt gtc atc ggc gtg ggg ctg ccc acc aac 934
Pro Pro Ser Leu Tyr Ile Phe Val Ile Gly Val Gly Leu Pro Thr Asn
20 25 30 35

tgc ctg gct ctg tgg gcg gcc tac cgc cag gtg caa cag cgc aac gag 982
Cys Leu Ala Leu Trp Ala Ala Tyr Arg Gln Val Gln Gln Arg Asn Glu
40 45 50

ctg ggc gtc tac ctg atg aac ctc agc atc gcc gac ctg ctg tac atc 1030
Leu Gly Val Tyr Leu Met Asn Leu Ser Ile Ala Asp Leu Leu Tyr Ile
55 60 65

tgc acg ctg ccg ctg tgg gtg gac tac ttc ctg cac cac gac aac tgg 1078
Cys Thr Leu Pro Leu Trp Val Asp Tyr Phe Leu His His Asp Asn Trp
70 75 80

atc cac ggc ccc ggg tcc tgc aag ctc ttt ggg ttc atc ttc tac acc 1126
Ile His Gly Pro Gly Ser Cys Lys Leu Phe Gly Phe Ile Phe Tyr Thr
85 90 95

aat atc tac atc agc atc gcc ttc ctg tgc tgc atc tcg gtg gac cgc 1174
Asn Ile Tyr Ile Ser Ile Ala Phe Leu Cys Cys Ile Ser Val Asp Arg
100 105 110 115

tac ctg gct gtg gcc cac cca ctc cgc ttc gcc cgc ctg cgc cgc gtc 1222
Tyr Leu Ala Val Ala His Pro Leu Arg Phe Ala Arg Leu Arg Arg Val
120 125 130

aag acc gcc gtg gcc gtg agc tcc gtg gtc tgg gcc acg gag ctg ggc 1270
Lys Thr Ala Val Ala Val Ser Ser Val Val Trp Ala Thr Glu Leu Gly
135 140 145

gcc aac tcg gcg ccc ctg ttc cat gac gag ctc ttc cga gac cgc tac 1318
Ala Asn Ser Ala Pro Leu Phe His Asp Glu Leu Phe Arg Asp Arg Tyr
150 155 160

aac cac acc ttc tgc ttt gag aag ttc ccc atg gaa ggc tgg gtg gcc 1366
Asn His Thr Phe Cys Phe Glu Lys Phe Pro Met Glu Gly Trp Val Ala
165 170 175

tgg atg aac ctc tat cgg gtg ttc gtg ggc ttc ctc ttc ccg tgg gcg 1414
Trp Met Asn Leu Tyr Arg Val Phe Val Gly Phe Leu Phe Pro Trp Ala
180 185 190 195